(19) Europäisches Patentamt
European Patent Office
Office européen des brevets

(11) EP 3 350 323 B1

(12) **EUROPEAN PATENT SPECIFICATION**

(45) Date of publication and mention of the grant of the patent:
**14.04.2021 Bulletin 2021/15**

(21) Application number: **16769936.2**

(22) Date of filing: **15.09.2016**

(51) Int Cl.:
*C12N 9/42* (2006.01) *C11D 3/386* (2006.01)

(86) International application number:
**PCT/EP2016/071854**

(87) International publication number:
**WO 2017/046260 (23.03.2017 Gazette 2017/12)**

(54) **POLYPEPTIDES HAVING XANTHAN DEGRADING ACTIVITY AND POLYNUCLEOTIDES ENCODING SAME**

POLYPEPTIDE MIT XANTHANABBAUENDER AKTIVITÄT UND POLYNUKLEOTIDE ZUR CODIERUNG DAVON

POLYPEPTIDES PRÉSENTANT UNE ACTIVITÉ DE DÉGRADATION DE XYLANASE ET POLYNUCLÉOTIDES CODANT POUR CEUX-CI

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.09.2015 EP 15185641**

(43) Date of publication of application:
**25.07.2018 Bulletin 2018/30**

(73) Proprietor: **Novozymes A/S**
**2880 Bagsvaerd (DK)**

(72) Inventors:
- **SEGURA, Dorotea, Raventos**
  **2880 Bagsvaerd (DK)**
- **ANDERSON, Lars**
  **2880 Bagsvaerd (DK)**
- **PALMÉN, Lorena, G,**
  **2880 Bagsvaerd (DK)**
- **CHRISTIANSEN, Liv, S,**
  **2880 Bagsvaerd (DK)**
- **HALLIN, Peter, F,**
  **2880 Bagsvaerd (DK)**
- **MURPHY, Leigh**
  **2880 Bagsvaerd (DK)**
- **OVERGAARD, Mette, L,D,**
  **2880 Bagsvaerd (DK)**
- **MONRAD, Rune, N,**
  **2880 Bagsvaerd (DK)**
- **O'CONNELL, Timothy**
  **86899 Landsberg am Lech (DE)**
- **TONDERA, Susanne**
  **40597 Düsseldorf (DE)**
- **MUSSMANN, Nina**
  **47877 Willich (DE)**
- **HERBST, Daniela**
  **40237 Düsseldorf (DE)**

(74) Representative: **NZ EPO Representatives**
**Krogshoejvej 36**
**2880 Bagsvaerd (DK)**

(56) References cited:
**WO-A1-2013/167581**

- **DATABASE UniProt [Online] 1 May 2013 (2013-05-01), "SubName: Full=Uncharacterized protein {ECO:0000313|EMBL:EMD99756.1};", XP002763582, retrieved from EBI accession no. UNIPROT:M2V1S3 Database accession no. M2V1S3**
- **DATABASE UniProt [Online] 6 March 2013 (2013-03-06), "SubName: Full=Endoglucanase {ECO:0000313|EMBL:AGA76691.1};", XP002763583, retrieved from EBI accession no. UNIPROT:L0FVA9 Database accession no. L0FVA9**
- **DATABASE UniProt [Online] 28 November 2012 (2012-11-28), "SubName: Full=Uncharacterized protein {ECO:0000313|EMBL:EJZ63897.1};", XP002763584, retrieved from EBI accession no. UNIPROT:K0WXE1 Database accession no. K0WXE1**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 350 323 B1

## Description

### Reference to a Sequence Listing

This application contains a Sequence Listing in computer readable form.

### Background of the invention

### Field of the invention

The present invention relates to polypeptides having xanthan degrading activity. In particular the invention relates to such polypeptides within the glycosyl hydrolase family 5 (GH5) having xanthan degrading activity, and to polynucleotides encoding the polypeptides, as set forth in the appended set of claims. The invention also relates to nucleic acid constructs, vectors, and host cells comprising the polynucleotides as well as methods of producing and using the polypeptides.

### Description of the related art

Xanthan gum is a polysaccharide secreted by the bacterium *Xanthomonas campestris.* It is produced by the fermentation of glucose, sucrose, or lactose in an aqueous growth medium by X. *campestris.* After a fermentation period, the polysaccharide is precipitated from the growth medium with isopropyl alcohol, dried, and ground into a fine powder. Later, the powder is added to a liquid medium to form the gum.

Xanthan is composed of pentasaccharide subunits, forming a cellulose backbone with trisaccharide side chains composed of mannose-(beta1,4)-glucuronic-acid-(beta1,2)-mannose attached to alternate glucose residues in the backbone by alpha1,3 linkages. This biopolymer is of great commercial significance because of its superior pseudoplasticity, thixotropy, and viscosity.

In recent years xanthan gum has been widely used as an ingredient in many consumer products including foods (e.g., as thickening agent in salad dressings and dairy products) and cosmetics (e.g., as stabilizer and thickener in toothpaste and make-up to prevent ingredients from separating) and cosmetics (e.g., sun creams).

In addition, xanthan gum has found use in the oil industry where xanthan gum is used in large quantities to thicken drilling mud. These fluids serve to carry the solids cut by the drilling bit back to the surface. When the circulation stops, the solids still remain suspended in the drilling fluid. The widespread use of horizontal drilling has led to its expanded use. Xanthan gum is also added to self-consolidating concrete, including concrete poured underwater, to increase its viscosity.

The widespread use of xanthan gum has led to a desire to be able to degrade solutions or gels of xanthan gum. Complete enzymatic degradation of xanthan gum has till now required several enzymatic activities including xanthan lyase activity and endo-beta-1,4-glucanase activity. Xanthan lyases are enzymes that cleave the beta-D-mannosylalpha-beta-D-1,4-glucuronosyl bond of xanthan and have been described in the literature. Xanthan degrading enzymes are known in the art e.g., two xanthan lyases isolated from *Paenibacillus alginolyticus* XL-1 (e.g., Ruijssenaars et al. (1999) 'A pyruvated mannose-specific xanthan lyase involved in xanthan degradation by Paenibacillus alginolyticus XL-1', Appl. Environ. Microbiol. 65(6): 2446-2452, and Ruijssenaars et al. (2000), 'A novel gene encoding xanthan lyase of Paenibacillus alginolyticus strain XL-1', Appl. Environ. Microbiol. 66(9): 3945-3950).

Glycosyl hydrolases are enzymes that catalyze the hydrolysis of the glycosyl bond to release smaller sugars. There are over 100 classes of Glycosyl hydrolases which have been classified, see Henrissat et al. (1991) 'A classification of glycosyl hydrolases based on amino-acid sequence similarities', J. Biochem. 280: 309-316 and the Uniprot website at www.cazy.org. The glycosyl hydrolase family 5 (GH5) includes endo-glucanases (EC 3.2.1.4), endo-beta-1,4-xylanase (EC 3.2.1.8); beta-glucosidase (EC 3.2.1.21); beta-mannosidase (EC 3.2.1.25). However, until now identification of xanthan degrading enzymes have not been reported in glycosyl hydrolase family 5.

The mature peptide in SEQ ID NO: 2 is 45 % identical and the mature peptide in SEQ ID NO: 4 is 57 % identical to a predicted endoglucanase from the genome of *Echinicola vietnamensis* (UNIPROT: L0FVA9).

The mature peptide in SEQ ID NO: 6 is 47 % identical to an uncharacterized protein from the genome of *Barnesiella intestinihominis* (UNIPROT: K0WXE1).

The mature peptide in SEQ ID NO: 8 is 100 % identical to an uncharacterized protein from the genome of *Pseudomonas stutzeri* (UNIPROT: M2V1S3).

WO 2013/167581 discloses GH9 endoglucanase polypeptides having xanthan degrading activity and polynucleotides encoding the same.

## Summary of the invention

The invention provides new and improved enzymes for the degradation of xanthan gum and the use of such enzymes, such as in the drilling and oil industries, as set forth in the appended set of claims.

The present inventors have surprisingly discovered a new group of enzymes that have xanthan degrading activity - and which do not belong to any glycosyl hydrolase family previously known to comprise this enzymatic activity. The enzymes have no significant sequence similarity to any known enzyme having xanthan degrading activity.

The present invention provides polypeptides having xanthan degrading activity, i.e., having activity on xanthan gum and/or having activity on xanthan gum pretreated with xanthan lyase. The present invention further provides polynucleotides encoding the polypeptides.

Accordingly, the present invention provides a polypeptide of glycosyl hydrolase family 5 having xanthan degrading activity. More particularly, the present invention provides a polypeptide of glycosyl hydrolase family 5 having xanthan degrading activity, selected from the group consisting of:

(a) a polypeptide having at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or 100% sequence identity to the mature polypeptide of any of SEQ ID NO: 2, SEQ ID NO: 4, or SEQ ID NO: 6;
(b) a fragment of the polypeptide of (a) that has xanthan degrading activity; and
(c) a polypeptide comprising the polypeptide of (a) or (b) and an N-terminal and/or C-terminal His-tag.

The present invention also relates to polynucleotides encoding the polypeptides of the present invention; nucleic acid constructs; recombinant expression vectors; recombinant host cells comprising the polynucleotides; and methods of producing the polypeptides.

The present invention also relates to methods of degrading xanthan gum using the polypeptides, such as in methods for extraction of oil and natural gas, e.g., for controlling viscosity of a drilling fluid or a borehole filtercake.

## Overview of Sequence Listing

SEQ ID NO: 1 is the DNA sequence of the EXa gene as isolated from an *Opitutaceae* sp.

SEQ ID NO: 2 is the amino acid sequence of the EXa GH5 polypeptide as deduced from SEQ ID NO: 1.

SEQ ID NO: 3 is the DNA sequence of the EXb gene as isolated from an environmental sample

SEQ ID NO: 4 is the amino acid sequence of the EXb GH5 polypeptide as deduced from SEQ ID NO: 3.

SEQ ID NO: 5 is the DNA sequence of the EXc gene as isolated from an environmental sample

SEQ ID NO: 6 is the amino acid sequence of the EXc GH5 polypeptide as deduced from SEQ ID NO: 5.

SEQ ID NO: 7 is the DNA sequence of the EXd gene as obtained from a public database (UNIPROT M2V1S3, originating from a strain of *Pseudomonas stutzeri* collected from a Galapagos Rift hydrothermal vent, Ecuador).

SEQ ID NO: 8 is the amino acid sequence of the EXd GH5 polypeptide as deduced from SEQ ID NO: 7.

SEQ ID NO:9 is synth codon optimized DNA encoding the EXa GH5 polypeptide.

SEQ ID NO:10 is synth codon optimized DNA encoding the EXb GH5 polypeptide.

SEQ ID NO:11 is synth codon optimized DNA encoding the EXc GH5 polypeptide.

SEQ ID NO:12 is synth codon optimized DNA encoding the EXd GH5 polypeptide.

SEQ ID NO:13 is the EXa GH5 polypeptide + His affinity tag expressed in *E.coli.*

SEQ ID NO:14 is the EXb GH5 polypeptide + His affinity tag expressed in *E.coli.*

SEQ ID NO:15 the EXc GH5 polypeptide + His affinity tag expressed in *E.coli.*

SEQ ID NO:16 is the EXb GH5 polypeptide + His affinity tag expressed in *B.subtilis.*

SEQ ID NO:17 is the EXc GH5 polypeptide + His affinity tag expressed in *B.subtilis.*

SEQ ID NO:18 is the EXd GH5 polypeptide + His affinity tag expressed in *B.subtilis.*

SEQ ID NO:19 is the His affinity tag sequence.

SEQ ID NO:20 is the amino acid sequence of the *Bacillus clausii* secretion signal.

SEQ ID NO:21 is the amino acid sequence of a xanthan lyase XLa from a *Paenibacillus* sp (SEQ ID NO: 8 from WO2013167581).

SEQ ID NO:22 is the amino acid sequence of a xanthan lyase XLb from a *Paenibacillus* sp (SEQ ID NO: 66 from WO2013167581).

SEQ ID NO:23 is the amino acid sequence of a xanthan lyase XLc from a *Paenibacillus* sp (SEQ ID NO: 68 from WO2013167581).

SEQ ID NO:24 is the amino acid sequence of a xanthan lyase XLd from a *Paenibacillus* sp (SEQ ID NO: 120 from WO2013167581).

| Identity Matrix for mature peptides | | | | |
|---|---|---|---|---|
| | SEQ ID NO:2 EXa | SEQ ID NO:4 EXb | SEQ ID NO:6 EXc | SEQ ID NO:8 EXd |
| SEQ ID NO:2 EXa | | 50 | 71 | 27 |
| SEQ ID NO:4 EXb | | | 47 | 31 |
| SEQ ID NO:6 EXc | | | | 27 |
| SEQ ID NO:8 EXd | | | | |

## Detailed Description of the Invention

The present invention provides GH5 polypeptides having xanthan degrading activity and polynucleotides encoding the polypeptides. The polypeptides do not belong to a GH family known to comprise enzymes which degrade xanthan. In addition, the combination of xanthan lyase and an enzyme of the invention having xanthan degrading activity shows a synergistic improved wash performance over using either a xanthan lyase or a GH5 polypeptide having xanthan degrading activity.

## Definitions

**Allelic variant:** The term "allelic variant" means any of two or more alternative forms of a gene occupying the same chromosomal locus. Allelic variation arises naturally through mutation, and may result in polymorphism within populations. Gene mutations can be silent (no change in the encoded polypeptide) or may encode polypeptides having altered amino acid sequences. An allelic variant of a polypeptide is a polypeptide encoded by an allelic variant of a gene.

**Catalytic domain:** The term "catalytic domain" means the region of an enzyme containing the catalytic machinery of the enzyme.

**cDNA:** The term "cDNA" means a DNA molecule that can be prepared by reverse transcription from a mature, spliced, mRNA molecule obtained from a eukaryotic or prokaryotic cell. cDNA lacks intron sequences that may be

present in the corresponding genomic DNA. The initial, primary RNA transcript is a precursor to mRNA that is processed through a series of steps, including splicing, before appearing as mature spliced mRNA.

**Coding sequence:** The term "coding sequence" means a polynucleotide, which directly specifies the amino acid sequence of a polypeptide. The boundaries of the coding sequence are generally determined by an open reading frame, which begins with a start codon such as ATG, GTG, or TTG and ends with a stop codon such as TAA, TAG, or TGA. The coding sequence may be a genomic DNA, cDNA, synthetic DNA, or a combination thereof.

**Colour clarification:** During washing and wearing loose or broken fibers can accumulate on the surface of the fabrics. One consequence can be that the colours of the fabric appear less bright or less intense because of the surface contaminations. Removal of the loose or broken fibers from the textile will partly restore the original colours and looks of the textile. By the term "colour clarification", as used herein, is meant the partial restoration of the initial colours of textile.

**Control sequences:** The term "control sequences" means nucleic acid sequences necessary for expression of a polynucleotide encoding a mature polypeptide of the present invention. Each control sequence may be native (*i.e.,* from the same gene) or foreign (*i.e.,* from a different gene) to the polynucleotide encoding the polypeptide or native or foreign to each other. Such control sequences include, but are not limited to, a leader, polyadenylation sequence, propeptide sequence, promoter, signal peptide sequence, and transcription terminator. At a minimum, the control sequences include a promoter, and transcriptional and translational stop signals. The control sequences may be provided with linkers for the purpose of introducing specific restriction sites facilitating ligation of the control sequences with the coding region of the polynucleotide encoding a polypeptide.

**Detergent Composition:** the term "detergent composition" refers to compositions that find use in the removal of undesired compounds from items to be cleaned, such as textiles, dishes, and hard surfaces. The terms encompass any materials/compounds selected for the particular type of cleaning composition desired and the form of the product (e.g., liquid, gel, powder, granulate, paste, or spray compositions) and includes, but is not limited to, detergent compositions (e.g., liquid and/or solid laundry detergents and fine fabric detergents; hard surface cleaning formulations, such as for glass, wood, ceramic and metal counter tops and windows; carpet cleaners; oven cleaners; fabric fresheners; fabric softeners; and textile and laundry pre-spotters, as well as dish wash detergents). In addition to containing an enzyme of the invention, the detergent formulation may contain one or more additional enzymes, and/or components such as surfactants, builders, chelators or chelating agents, bleach system or bleach components, polymers, fabric conditioners, foam boosters, suds suppressors, dyes, perfume, tannish inhibitors, optical brighteners, bactericides, fungicides, soil suspending agents, anti-corrosion agents, enzyme inhibitors or stabilizers, enzyme activators, transferase(s), hydrolytic enzymes, oxido reductases, bluing agents and fluorescent dyes, antioxidants, and solubilizers.

**Dish wash:** The term "dish wash" refers to all forms of washing dishes, e.g., by hand or automatic dish wash. Washing dishes includes, but is not limited to, the cleaning of all forms of crockery such as plates, cups, glasses, bowls, all forms of cutlery such as spoons, knives, forks and serving utensils as well as ceramics, plastics, metals, china, glass and acrylics.

**Dish washing composition:** The term "dish washing composition" refers to all forms of compositions for cleaning hard surfaces. The present invention is not restricted to any particular type of dish wash composition or any particular detergent.

**Enzyme Detergency benefit:** The term "enzyme detergency benefit" is defined herein as the advantageous effect an enzyme may add to a detergent compared to the same detergent without the enzyme. Important detergency benefits which can be provided by enzymes are stain removal with no or very little visible soils after washing and or cleaning, prevention or reduction of redeposition of soils released in the washing process an effect that also is termed anti-redeposition, restoring fully or partly the whiteness of textiles, which originally were white but after repeated use and wash have obtained a greyish or yellowish appearance an effect that also is termed whitening. Textile care benefits, which are not directly related to catalytic stain removal or prevention of redeposition of soils are also important for enzyme detergency benefits. Examples of such textile care benefits are prevention or reduction of dye transfer from one fabric to another fabric or another part of the same fabric an effect that is also termed dye transfer inhibition or anti-backstaining, removal of protruding or broken fibers from a fabric surface to decrease pilling tendencies or remove already existing pills or fuzz an effect that also is termed anti-pilling, improvement of the fabric-softness, colour clarification of the fabric and removal of particulate soils which are trapped in the fibers of the fabric or garment. Enzymatic bleaching is a further enzyme detergency benefit where the catalytic activity generally is used to catalyze the formation of bleaching component such as hydrogen peroxide or other peroxides.

**Expression:** The term "expression" includes any step involved in the production of a polypeptide including, but not limited to, transcription, post-transcriptional modification, translation, post-translational modification, and secretion.

**Expression vector:** The term "expression vector" means a linear or circular DNA molecule that comprises a polynucleotide encoding a polypeptide and is operably linked to control sequences that provide for its expression.

**Fragment:** The term "fragment" means a polypeptide having one or more (e.g., several) amino acids absent from

the amino and/or carboxyl terminus of a mature polypeptide or domain; wherein the fragment has xanthan degrading activity.

**Hard surface cleaning:** The term "Hard surface cleaning" is defined herein as cleaning of hard surfaces wherein hard surfaces may include floors, tables, walls, roofs etc. as well as surfaces of hard objects such as cars (car wash) and dishes (dish wash). Dish washing includes but are not limited to cleaning of plates, cups, glasses, bowls, and cutlery such as spoons, knives, forks, serving utensils, ceramics, plastics, metals, china, glass and acrylics.

**Host cell:** The term "host cell" means any cell type that is susceptible to transformation, transfection, transduction, or the like with a nucleic acid construct or expression vector comprising a GH5 polynucleotide of the present invention. The term "host cell" encompasses any progeny of a parent cell that is not identical to the parent cell due to mutations that occur during replication.

**Improved wash performance:** The term "improved wash performance" is defined herein as a (variant) enzyme (also a blend of enzymes, not necessarily only variants but also backbones, and in combination with certain cleaning composition etc.) displaying an alteration of the wash performance of a protease variant relative to the wash performance of the parent protease variant e.g. by increased stain removal. The term "wash performance" includes wash performance in laundry but also e.g. in dish wash.

**Isolated:** The term "isolated" means a substance in a form or environment that does not occur in nature. Non-limiting examples of isolated substances include (1) any non-naturally occurring substance, (2) any substance including, but not limited to, any enzyme, variant, nucleic acid, protein, peptide or cofactor, that is at least partially removed from one or more or all of the naturally occurring constituents with which it is associated in nature; (3) any substance modified by the hand of man relative to that substance found in nature; or (4) any substance modified by increasing the amount of the substance relative to other components with which it is naturally associated (*e.g.*, recombinant production in a host cell; multiple copies of a gene encoding the substance; and use of a stronger promoter than the promoter naturally associated with the gene encoding the substance). An isolated substance may be present in a fermentation broth sample; e.g. a host cell may be genetically modified to express the polypeptide of the invention. The fermentation broth from that host cell will comprise the isolated polypeptide.

**Mature polypeptide:** The term "mature polypeptide" means a polypeptide in its final form following translation and any post-translational modifications, such as N-terminal processing, C-terminal truncation, glycosylation, phosphorylation, etc. In one aspect, the mature polypeptide is amino acids 1 to 802 of SEQ ID NO: 2. In a second aspect, the mature polypeptide is amino acids 1 to 808 of SEQ ID NO: 4. In a third aspect, the mature polypeptide is amino acids 1 to 800 of SEQ ID NO: 6. In a fourth aspect, the mature polypeptide is amino acids 1 to 657 of SEQ ID NO: 8. It is known in the art that a host cell may produce a mixture of two of more different mature polypeptides (*i.e.,* with a different C-terminal and/or N-terminal amino acid) expressed by the same polynucleotide. It is also known in the art that different host cells process polypeptides differently, and thus, one host cell expressing a polynucleotide may produce a different mature polypeptide (*e.g.*, having a different C-terminal and/or N-terminal amino acid) as compared to another host cell expressing the same polynucleotide.

**Mature polypeptide coding sequence:** The term "mature polypeptide coding sequence" means a polynucleotide that encodes a mature polypeptide having xanthan degrading activity. In one aspect, the mature polypeptide coding sequence is nucleotides 109 to 2514 of SEQ ID NO: 1. Nucleotides 1 to 108 of SEQ ID NO: 1 encode a signal peptide. In one aspect, the mature polypeptide coding sequence is nucleotides 112 to 2493 of SEQ ID NO: 3. Nucleotides 1 to 111 of SEQ ID NO: 3 encode a signal peptide. In one aspect, the mature polypeptide coding sequence is nucleotides 106 to 2505 of SEQ ID NO: 5. Nucleotides 1 to 105 of SEQ ID NO: 5 encode a signal peptide. In one aspect, the mature polypeptide coding sequence is nucleotides 109 to 2079 of SEQ ID NO: 7. Nucleotides 1 to 108 of SEQ ID NO: 7 encode a signal peptide.

**Nucleic acid construct:** The term "nucleic acid construct" means a nucleic acid molecule, either single- or double-stranded, which is isolated from a naturally occurring gene or is modified to contain segments of nucleic acids in a manner that would not otherwise exist in nature or which is synthetic, which comprises one or more control sequences.

**Operably linked:** The term "operably linked" means a configuration in which a control sequence is placed at an appropriate position relative to the coding sequence of a polynucleotide such that the control sequence directs expression of the coding sequence.

**Sequence identity:** The relatedness between two amino acid sequences or between two nucleotide sequences is described by the parameter "sequence identity".

For purposes of the present invention, the sequence identity between two amino acid sequences is determined using the Needleman-Wunsch algorithm (Needleman and Wunsch, 1970, J. Mol. Biol. 48: 443-453) as implemented in the Needle program of the EMBOSS package (EMBOSS: The European Molecular Biology Open Software Suite, Rice et al., 2000, Trends Genet. 16: 276-277), preferably version 5.0.0 or later. The parameters used are gap open penalty of 10, gap extension penalty of 0.5, and the EBLOSUM62 (EMBOSS version of BLOSUM62) substitution matrix. The output of Needle labeled "longest identity" (obtained using the -nobrief option) is used as the percent identity and is

calculated as follows:

(Identical Residues x 100)/(Length of Alignment – Total Number of Gaps in Alignment)

For purposes of the present invention, the sequence identity between two deoxyribonucleotide sequences is determined using the Needleman-Wunsch algorithm (Needleman and Wunsch, 1970, *supra*) as implemented in the Needle program of the EMBOSS package (EMBOSS: The European Molecular Biology Open Software Suite, Rice et al., 2000, supra), preferably version 5.0.0 or later. The parameters used are gap open penalty of 10, gap extension penalty of 0.5, and the EDNAFULL (EMBOSS version of NCBI NUC4.4) substitution matrix. The output of Needle labeled "longest identity" (obtained using the -nobrief option) is used as the percent identity and is calculated as follows:

(Identical Deoxyribonucleotides x 100)/(Length of Alignment – Total Number of Gaps in Alignment)

**Stringency conditions:** The term "very low stringency conditions" means for probes of at least 100 nucleotides in length, prehybridization and hybridization at 42°C in 5X SSPE, 0.3% SDS, 200 micrograms/ml sheared and denatured salmon sperm DNA, and 25% formamide, following standard Southern blotting procedures for 12 to 24 hours. The carrier material is finally washed three times each for 15 minutes using 2X SSC, 0.2% SDS at 45°C.

The term "low stringency conditions" means for probes of at least 100 nucleotides in length, prehybridization and hybridization at 42°C in 5X SSPE, 0.3% SDS, 200 micrograms/ml sheared and denatured salmon sperm DNA, and 25% formamide, following standard Southern blotting procedures for 12 to 24 hours. The carrier material is finally washed three times each for 15 minutes using 2X SSC, 0.2% SDS at 50°C.

The term "medium stringency conditions" means for probes of at least 100 nucleotides in length, prehybridization and hybridization at 42°C in 5X SSPE, 0.3% SDS, 200 micrograms/ml sheared and denatured salmon sperm DNA, and 35% formamide, following standard Southern blotting procedures for 12 to 24 hours. The carrier material is finally washed three times each for 15 minutes using 2X SSC, 0.2% SDS at 55°C.

The term "medium-high stringency conditions" means for probes of at least 100 nucleotides in length, prehybridization and hybridization at 42°C in 5X SSPE, 0.3% SDS, 200 micrograms/ml sheared and denatured salmon sperm DNA, and 35% formamide, following standard Southern blotting procedures for 12 to 24 hours. The carrier material is finally washed three times each for 15 minutes using 2X SSC, 0.2% SDS at 60°C.

The term "high stringency conditions" means for probes of at least 100 nucleotides in length, prehybridization and hybridization at 42°C in 5X SSPE, 0.3% SDS, 200 micrograms/ml sheared and denatured salmon sperm DNA, and 50% formamide, following standard Southern blotting procedures for 12 to 24 hours. The carrier material is finally washed three times each for 15 minutes using 2X SSC, 0.2% SDS at 65°C.

The term "very high stringency conditions" means for probes of at least 100 nucleotides in length, prehybridization and hybridization at 42°C in 5X SSPE, 0.3% SDS, 200 micrograms/ml sheared and denatured salmon sperm DNA, and 50% formamide, following standard Southern blotting procedures for 12 to 24 hours. The carrier material is finally washed three times each for 15 minutes using 2X SSC, 0.2% SDS at 70°C.]

**Subsequence:** The term "subsequence" means a polynucleotide having one or more (*e.g.*, several) nucleotides absent from the 5' and/or 3' end of a mature polypeptide coding sequence; wherein the subsequence encodes a fragment having xanthan degrading activity.

**Textile:** The term "textile" means any textile material including yarns, yarn intermediates, fibers, non-woven materials, natural materials, synthetic materials, and any other textile material, fabrics made of these materials and products made from fabrics (e.g., garments and other articles). The textile or fabric may be in the form of knits, wovens, denims, non-wovens, felts, yarns, and towelling. The textile may be cellulose based such as natural cellulosics, including cotton, flax/linen, jute, ramie, sisal or coir or manmade cellulosics (e.g. originating from wood pulp) including viscose/rayon, ramie, cellulose acetate fibers (tricell), lyocell or blends thereof. The textile or fabric may also be non-cellulose based such as natural polyamides including wool, camel, cashmere, mohair, rabit and silk or synthetic polymer such as nylon, aramid, polyester, acrylic, polypropylen and spandex/elastane, or blends thereof as well as blend of cellulose based and non-cellulose based fibers. Examples of blends are blends of cotton and/or rayon/viscose with one or more companion material such as wool, synthetic fibers (e.g. polyamide fibers, acrylic fibers, polyester fibers, polyvinyl alcohol fibers, polyvinyl chloride fibers, polyurethane fibers, polyurea fibers, aramid fibers), and cellulose-containing fibers (e.g. rayon/viscose, ramie, flax/linen, jute, cellulose acetate fibers, lyocell). Fabric may be conventional washable laundry, for example stained household laundry. When the term fabric or garment is used it is intended to include the broader term textiles as well.

**Textile care benefit:** "Textile care benefits", which are not directly related to catalytic stain removal or prevention

of redeposition of soils, are also important for enzyme detergency benefits. Examples of such textile care benefits are prevention or reduction of dye transfer from one textile to another textile or another part of the same textile an effect that is also termed dye transfer inhibition or anti-backstaining, removal of protruding or broken fibers from a textile surface to decrease pilling tendencies or remove already existing pills or fuzz an effect that also is termed anti-pilling, improvement of the textile-softness, colour clarification of the textile and removal of particulate soils which are trapped in the fibers of the textile. Enzymatic bleaching is a further enzyme detergency benefit where the catalytic activity generally is used to catalyze the formation of bleaching component such as hydrogen peroxide or other peroxides or other bleaching species.

**Wash performance:** The term "wash performance" is used as an enzyme's ability to remove stains present on the object to be cleaned during e.g. wash or hard surface cleaning. The improvement in the wash performance may be quantified by calculating the so-called intensity value (Int) as defined in 'Automatic Mechanical Stress Assay (AMSA) for laundry' herein. See also the wash performance test in Example 18 herein.

**Whiteness:** The term "Whiteness" is defined herein as a broad term with different meanings in different regions and for different customers. Loss of whiteness can e.g. be due to greying, yellowing, or removal of optical brighteners/hueing agents. Greying and yellowing can be due to soil redeposition, body soils, colouring from e.g. iron and copper ions or dye transfer. Whiteness might include one or several issues from the list below: colorant or dye effects; incomplete stain removal (e.g. body soils, sebum ect.); re-deposition (greying, yellowing or other discolorations of the object) (removed soils re-associates with other part of textile, soiled or unsoiled); chemical changes in textile during application; and clarification or brightening of colours.

**Xanthan Lyase:** The term "xanthan lyase" is defined herein as an enzyme that cleaves the beta-D-mannosyl-beta-D-1,4-glucuronosyl bonds in xanthan gum (EC 4.2.2.12). For purposes of the present invention, xanthan lyase activity is determined according to the procedure described in the Examples in the 'Xanthan lyase activity assay.

**Xanthan degrading activity:** The term "xanthan degrading activity" is defined herein as ability to cause viscosity reduction of a xanthan solution. Xanthan solution is highly viscous even at low polymer concentrations, and this viscosity is associated with the polymer degree of xanthan. Therefore, viscosity reduction can be used to monitor xanthan degradation. The viscosity reduction may be detected using the viscosity pressure assay described in Example 6.

Xanthan degrading activity includes activity towards intact xanthan as well as activity towards xanthan pretreated with xanthan lyase (modified xanthan gum - see Example 8).

**Activity on xanthan gum**: The term "GH5 polypeptide having activity on xanthan gum" or a "polypeptide having activity on xanthan gum and belonging to the GH5 class of glycosyl hydrolases" is defined as a polypeptide comprising a domain belonging to the GH5 class of glycosyl hydrolases, and having significant activity on xanthan gum. In one aspect of the invention a GH5 polypeptide having activity on xanthan gum may be a polypeptide having a sequence selected among SEQ ID NO: 2, SEQ ID NO: 4, SEQ ID NO: 6, and SEQ ID NO: 8.

**Activity on xanthan gum pretreated with xanthan lyase**: The term "GH5 polypeptide having activity on xanthan gum pretreated with xanthan lyase" or a "polypeptide having activity on xanthan gum pretreated with xanthan lyase and belonging to the GH5 class of glycosyl hydrolases" is defined as a polypeptide comprising a domain belonging to the GH5 class of glycosyl hydrolases, and having significant activity on xanthan gum pretreated with xanthan lyase (modified xanthan gum - see Example 8). In one aspect of the invention a GH5 polypeptide having activity on xanthan gum pretreated with xanthan lyase may be a polypeptide having a sequence selected among SEQ ID NO: 2, SEQ ID NO: 4, SEQ ID NO: 6, and SEQ ID NO: 8.

## Polypeptides having xanthan degrading activity

In an embodiment, the present invention relates to polypeptides having a sequence identity to the mature polypeptide of any of SEQ ID NO: 2, 4, and 6 of at least 80%, at least 85%, at least 90%, at least 91%, at least 92%, at least 93%, at least 94%, at least 95%, at least 96%, at least 97%, at least 98%, at least 99%, or 100%, which have xanthan degrading activity. In one aspect, the polypeptides differ by up to 10 amino acids, *e.g.*, 1, 2, 3, 4, 5, 6, 7, 8, 9, or 10, from the mature polypeptide of any of SEQ ID NO: 2, 4, and 6.

In a particular embodiment the invention relates to polypeptides having a sequence identity to the mature polypeptide of any of SEQ ID NO: 2, 4, and 6 of at least 80%, at least 85%, at least 90%, at least 91%, at least 92%, at least 93%, at least 94%, at least 95%, at least 96%, at least 97%, at least 98%, at least 99%, or 100%, and wherein the polypeptide has at least at least 70% of the xanthan degrading activity of the mature polypeptide of any of SEQ ID NO: 2, 4, and 6.

In a particular embodiment the invention relates to polypeptides having a sequence identity to the mature polypeptide of any of SEQ ID NO: 2, 4, and 6 of at least 80%, at least 85%, at least 90%, at least 91%, at least 92%, at least 93%, at least 94%, at least 95%, at least 96%, at least 97%, at least 98%, at least 99%, or 100%, and wherein the polypeptide has at least at least 75% of the xanthan degrading activity of the mature polypeptide of any of SEQ ID NO: 2, 4, and 6.

In a particular embodiment the invention relates to polypeptides having a sequence identity to the mature

polypeptide of any of SEQ ID NO: 2, 4, and 6 of at least 80%, at least 85%, at least 90%, at least 91%, at least 92%, at least 93%, at least 94%, at least 95%, at least 96%, at least 97%, at least 98%, at least 99%, or 100%, and wherein the polypeptide has at least at least 80% of the xanthan degrading activity of the mature polypeptide of any of SEQ ID NO: 2, 4, and 6.

In a particular embodiment the invention relates to polypeptides having a sequence identity to the mature polypeptide of any of SEQ ID NO: 2, 4, and 6 of at least 80%, at least 85%, at least 90%, at least 91%, at least 92%, at least 93%, at least 94%, at least 95%, at least 96%, at least 97%, at least 98%, at least 99%, or 100%, and wherein the polypeptide has at least at least 85% of the xanthan degrading activity of the mature polypeptide of any of SEQ ID NO: 2, 4, and 6.

In a particular embodiment the invention relates to polypeptides having a sequence identity to the mature polypeptide of any of SEQ ID NO: 2, 4, and 6 of at least 80%, at least 85%, at least 90%, at least 91%, at least 92%, at least 93%, at least 94%, at least 95%, at least 96%, at least 97%, at least 98%, at least 99%, or 100%, and wherein the polypeptide has at least at least 90% of the xanthan degrading activity of the mature polypeptide of any of SEQ ID NO: 2, 4, and 6.

In a particular embodiment the invention relates to polypeptides having a sequence identity to the mature polypeptide of any of SEQ ID NO: 2, 4, and 6 of at least 80%, at least 85%, at least 90%, at least 91%, at least 92%, at least 93%, at least 94%, at least 95%, at least 96%, at least 97%, at least 98%, at least 99%, or 100%, and wherein the polypeptide has at least at least 95% of the xanthan degrading activity of the mature polypeptide of any of SEQ ID NO: 2, 4, and 6.

In a particular embodiment the invention relates to polypeptides having a sequence identity to the mature polypeptide of any of SEQ ID NO: 2, 4, and 6 of at least 80%, at least 85%, at least 90%, at least 91%, at least 92%, at least 93%, at least 94%, at least 95%, at least 96%, at least 97%, at least 98%, at least 99%, or 100%, and wherein the polypeptide has at least at least 100% of the xanthan degrading activity of the mature polypeptide of any of SEQ ID NO: 2, 4, and 6.

In an embodiment, the polypeptide has been isolated. A polypeptide of the present invention preferably comprises or consists of the amino acid sequence of any of SEQ ID NO: 2, 4, and 6 or an allelic variant thereof; or is a fragment thereof having xanthan degrading activity. In another aspect, the polypeptide comprises or consists of the mature polypeptide of any of SEQ ID NO: 2, 4, and 6. In another aspect, the polypeptide comprises or consists of amino acids 1 to 802 of SEQ ID NO: 2, amino acids 1 to 808 of SEQ ID NO: 4, amino acids 1 to 800 of SEQ ID NO: 6.

In another embodiment, the present invention relates to a polypeptide having xanthan degrading activity encoded by a polynucleotide that hybridizes under very low stringency conditions, low stringency conditions, medium stringency conditions, medium-high stringency conditions, high stringency conditions, or very high stringency conditions with (i) the mature polypeptide coding sequence of SEQ ID NO: 1, (ii), or (iii) the full-length complement of (i) or (ii) (Sambrook et al., 1989, Molecular Cloning, A Laboratory Manual, 2d edition, Cold Spring Harbor, New York). In an embodiment, the polypeptide has been isolated.

The polynucleotide of any of SEQ ID NO: 1, 3, 5, or 7 or a subsequence thereof, as well as the polypeptide of any of SEQ ID NO: 2, 4, 6 and 8 or a fragment thereof may be used to design nucleic acid probes to identify and clone DNA encoding polypeptides having xanthan degrading activity from strains of different genera or species according to methods well known in the art. In particular, such probes can be used for hybridization with the genomic DNA or cDNA of a cell of interest, following standard Southern blotting procedures, in order to identify and isolate the corresponding gene therein. Such probes can be considerably shorter than the entire sequence, but should be at least 15, *e.g.*, at least 25, at least 35, or at least 70 nucleotides in length. Preferably, the nucleic acid probe is at least 100 nucleotides in length, *e.g.*, at least 200 nucleotides, at least 300 nucleotides, at least 400 nucleotides, at least 500 nucleotides, at least 600 nucleotides, at least 700 nucleotides, at least 800 nucleotides, or at least 900 nucleotides in length. Both DNA and RNA probes can be used. The probes are typically labeled for detecting the corresponding gene (for example, with $^{32}P$, $^{3}H$, $^{35}S$, biotin, or avidin). Such probes are encompassed by the present invention.

A genomic DNA or cDNA library prepared from such other strains may be screened for DNA that hybridizes with the probes described above and encodes a polypeptide having xanthan degrading activity. Genomic or other DNA from such other strains may be separated by agarose or polyacrylamide gel electrophoresis, or other separation techniques. DNA from the libraries or the separated DNA may be transferred to and immobilized on nitrocellulose or other suitable carrier material. In order to identify a clone or DNA that hybridizes with SEQ ID NO: 1 or a subsequence thereof, the carrier material is used in a Southern blot.

For purposes of the present invention, hybridization indicates that the polynucleotide hybridizes to a labeled nucleic acid probe corresponding to (i) any of SEQ ID NO: 1, 3, or 5; (ii) the mature polypeptide coding sequence of any of SEQ ID NO: 1, 3, or 5; (iii) the full-length complement thereof; or (iv) a subsequence thereof; under very low to very high stringency conditions. Molecules to which the nucleic acid probe hybridizes under these conditions can be detected using, for example, X-ray film or any other detection means known in the art.

In another embodiment, the present invention relates to a polypeptide having xanthan degrading activity encoded

by a polynucleotide having a sequence identity to the mature polypeptide coding sequence of any of SEQ ID NO: 1, 3, or 5 of at least 60%, *e.g.*, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100%. In a further embodiment, the polypeptide has been isolated.

In another embodiment, the present invention relates to variants of the mature polypeptide of any of SEQ ID NO: 2, 4, and 6 comprising a substitution, deletion, and/or insertion at one or more (*e.g.*, several) positions. In an embodiment, the number of amino acid substitutions, deletions and/or insertions introduced into the mature polypeptide of any of SEQ ID NO: 2, 4, and 6 is up to 10, *e.g.,* 1, 2, 3, 4, 5, 6, 7, 8, 9, or 10. The amino acid changes may be of a minor nature, that is conservative amino acid substitutions or insertions that do not significantly affect the folding and/or activity of the protein; small deletions, typically of 1-30 amino acids; small amino- or carboxyl-terminal extensions, such as an amino-terminal methionine residue; a small linker peptide of up to 20-25 residues; or a small extension that facilitates purification by changing net charge or another function, such as a poly-histidine tag, an antigenic epitope or a binding domain. SEQ ID NO: 13, 14 and 15 show the polypeptides of the invention (SEQ ID NO: 2, 4 and 6) with an N-terminal poly histidine tag (His-tag). SEQ ID NO: 16, 17 and 18 show the polypeptides (SEQ ID NO: 4, 6 and 8) with an N-terminal poly histidine tag.

Examples of conservative substitutions are within the groups of basic amino acids (arginine, lysine and histidine), acidic amino acids (glutamic acid and aspartic acid), polar amino acids (glutamine and asparagine), hydrophobic amino acids (leucine, isoleucine and valine), aromatic amino acids (phenylalanine, tryptophan and tyrosine), and small amino acids (glycine, alanine, serine, threonine and methionine). Amino acid substitutions that do not generally alter specific activity are known in the art and are described, for example, by H. Neurath and R.L. Hill, 1979, In, The Proteins, Academic Press, New York. Common substitutions are Ala/Ser, Val/Ile, Asp/Glu, Thr/Ser, Ala/Gly, Ala/Thr, Ser/Asn, Ala/Val, Ser/Gly, Tyr/Phe, Ala/Pro, Lys/Arg, Asp/Asn, Leu/Ile, Leu/Val, Ala/Glu, and Asp/Gly.

**Sources of polypeptides having xanthan degrading activity**

A polypeptide having xanthan degrading activity of the present invention may be obtained from microorganisms of any genus. For purposes of the present invention, the term "obtained from" as used herein in connection with a given source shall mean that the polypeptide encoded by a polynucleotide is produced by the source or by a strain in which the polynucleotide from the source has been inserted.

In an aspect, the polypeptide is a polypeptide obtained from an *Opitutaceae* species.

The polypeptide may be identified and obtained from other sources including microorganisms isolated from nature (*e.g.*, soil, composts, water, etc.) or DNA samples obtained directly from natural materials (*e.g.*, soil, composts, water, etc.) using the above-mentioned probes. Techniques for isolating microorganisms and DNA directly from natural habitats are well known in the art. A polynucleotide encoding the polypeptide may then be obtained by similarly screening a genomic DNA or cDNA library of another microorganism or mixed DNA sample. Once a polynucleotide encoding a polypeptide has been detected with the probe(s), the polynucleotide can be isolated or cloned by utilizing techniques that are known to those of ordinary skill in the art (see, *e.g.,* Sambrook *et al.,* 1989, *supra*).

**Polynucleotides**

The present invention also relates to polynucleotides encoding a polypeptide, as described herein. In an embodiment, the polynucleotide encoding the polypeptide of the present invention has been isolated.

The techniques used to isolate or clone a polynucleotide are known in the art and include isolation from genomic DNA or cDNA, or a combination thereof. The cloning of the polynucleotides from genomic DNA can be effected, *e.g.*, by using the well-known polymerase chain reaction (PCR) or antibody screening of expression libraries to detect cloned DNA fragments with shared structural features. See, *e.g.,* Innis et al., 1990, PCR: A Guide to Methods and Application, Academic Press, New York. Other nucleic acid amplification procedures such as ligase chain reaction (LCR), ligation activated transcription (LAT) and polynucleotide-based amplification (NASBA) may be used. The polynucleotides may be cloned from a strain of an *Opitutaceae* species, or a related organism and thus, for example, may be an allelic or species variant of the polypeptide encoding region of the polynucleotide.

Modification of a polynucleotide encoding a polypeptide of the present invention may be necessary for synthesizing polypeptides substantially similar to the polypeptide. The term "substantially similar" to the polypeptide refers to non-naturally occurring forms of the polypeptide.

**Nucleic Acid Constructs**

The present invention also relates to nucleic acid constructs comprising a GH5 polynucleotide of the present invention operably linked to one or more control sequences that direct the expression of the coding sequence in a suitable

host cell under conditions compatible with the control sequences.

The polynucleotide may be manipulated in a variety of ways to provide for expression of the polypeptide. Manipulation of the polynucleotide prior to its insertion into a vector may be desirable or necessary depending on the expression vector. The techniques for modifying polynucleotides utilizing recombinant DNA methods are well known in the art.

The control sequence may be a promoter, a polynucleotide that is recognized by a host cell for expression of a polynucleotide encoding a polypeptide of the present invention. The promoter contains transcriptional control sequences that mediate the expression of the polypeptide. The promoter may be any polynucleotide that shows transcriptional activity in the host cell including variant, truncated, and hybrid promoters, and may be obtained from genes encoding extracellular or intracellular polypeptides either homologous or heterologous to the host cell.

Examples of suitable promoters for directing transcription of the nucleic acid constructs of the present invention in a bacterial host cell are the promoters obtained from the *Bacillus amyloliquefaciens* alpha-amylase gene (*amyQ*), *Bacillus licheniformis* alpha-amylase gene (*amyL*), *Bacillus licheniformis* penicillinase gene (*penP*), *Bacillus stearothermophilus* maltogenic amylase gene (*amyM*), *Bacillus subtilis* levansucrase gene (*sacB*), *Bacillus subtilis xylA* and *xylB* genes, *Bacillus thuringiensis cryIIIA* gene (Agaisse and Lereclus, 1994, Molecular Microbiology 13: 97-107), *E. coli lac* operon, *E. coli trc* promoter (Egon et al., 1988, Gene 69: 301-315), *Streptomyces coelicolor* agarase gene (*dagA*), and prokaryotic beta-lactamase gene (Villa-Kamaroff et al., 1978, Proc. Natl. Acad. Sci. USA 75: 3727-3731), as well as the *tac* promoter (DeBoer et al., 1983, Proc. Natl. Acad. Sci. USA 80: 21-25). Further promoters are described in "Useful proteins from recombinant bacteria" in Gilbert et al., 1980, Scientific American 242: 74-94; and in Sambrook *et al.,* 1989, *supra.* Examples of tandem promoters are disclosed in WO 99/43835.

Examples of suitable promoters for directing transcription of the nucleic acid constructs of the present invention in a filamentous fungal host cell are promoters obtained from the genes for *Aspergillus nidulans* acetamidase, *Aspergillus niger* neutral alpha-amylase, *Aspergillus niger* acid stable alpha-amylase, *Aspergillus niger* or *Aspergillus awamori* glucoamylase (*glaA*), *Aspergillus oryzae* TAKA amylase, *Aspergillus oryzae* alkaline protease, *Aspergillus oryzae* triose phosphate isomerase, *Fusarium oxysporum* trypsin-like protease (WO 96/00787), *Fusarium venenatum* amyloglucosidase (WO 00/56900), *Fusarium venenatum* Daria (WO 00/56900), *Fusarium venenatum* Quinn (WO 00/56900), *Rhizomucor miehei* lipase, *Rhizomucor miehei* aspartic proteinase, *Trichoderma reesei* beta-glucosidase, *Trichoderma reesei* cellobiohydrolase I, *Trichoderma reesei* cellobiohydrolase II, *Trichoderma reesei* endoglucanase I, *Trichoderma reesei* endoglucanase II, *Trichoderma reesei* endoglucanase III, *Trichoderma reesei* endoglucanase V, *Trichoderma reesei* xylanase I, *Trichoderma reesei* xylanase II, *Trichoderma reesei* xylanase III, *Trichoderma reesei* beta-xylosidase, and *Trichoderma reesei* translation elongation factor, as well as the NA2-tpi promoter (a modified promoter from an *Aspergillus* neutral alpha-amylase gene in which the untranslated leader has been replaced by an untranslated leader from an *Aspergillus* triose phosphate isomerase gene; non-limiting examples include modified promoters from an *Aspergillus niger* neutral alpha-amylase gene in which the untranslated leader has been replaced by an untranslated leader from an *Aspergillus nidulans* or *Aspergillus oryzae* triose phosphate isomerase gene); and variant, truncated, and hybrid promoters thereof. Other promoters are described in U.S. Patent No. 6,011,147.

In a yeast host, useful promoters are obtained from the genes for *Saccharomyces cerevisiae* enolase (ENO-1), *Saccharomyces cerevisiae* galactokinase (GAL1), *Saccharomyces cerevisiae* alcohol dehydrogenase/glyceraldehyde-3-phosphate dehydrogenase (ADH1, ADH2/GAP), *Saccharomyces cerevisiae* triose phosphate isomerase (TPI), *Saccharomyces cerevisiae* metallothionein (CUP1), and *Saccharomyces cerevisiae* 3-phosphoglycerate kinase. Other useful promoters for yeast host cells are described by Romanos et al., 1992, Yeast 8: 423-488.

The control sequence may also be a transcription terminator, which is recognized by a host cell to terminate transcription. The terminator is operably linked to the 3'-terminus of the polynucleotide encoding the polypeptide. Any terminator that is functional in the host cell may be used in the present invention.

Preferred terminators for bacterial host cells are obtained from the genes for *Bacillus clausii* alkaline protease (*aprH*), *Bacillus licheniformis* alpha-amylase (*amyL*), and *Escherichia coli* ribosomal RNA (*rrnB*).

Preferred terminators for filamentous fungal host cells are obtained from the genes for *Aspergillus nidulans* acetamidase, *Aspergillus nidulans* anthranilate synthase, *Aspergillus niger* glucoamylase, *Aspergillus niger* alpha-glucosidase, *Aspergillus oryzae* TAKA amylase, *Fusarium oxysporum* trypsin-like protease, *Trichoderma reesei* beta-glucosidase, *Trichoderma reesei* cellobiohydrolase I, *Trichoderma reesei* cellobiohydrolase II, *Trichoderma reesei* endoglucanase I, *Trichoderma reesei* endoglucanase II, *Trichoderma reesei* endoglucanase III, *Trichoderma reesei* endoglucanase V, *Trichoderma reesei* xylanase I, *Trichoderma reesei* xylanase II, *Trichoderma reesei* xylanase III, *Trichoderma reesei* beta-xylosidase, and *Trichoderma reesei* translation elongation factor.

Preferred terminators for yeast host cells are obtained from the genes for *Saccharomyces cerevisiae* enolase, *Saccharomyces cerevisiae* cytochrome C (CYC1), and *Saccharomyces cerevisiae* glyceraldehyde-3-phosphate dehydrogenase. Other useful terminators for yeast host cells are described by Romanos *et al.,* 1992, *supra.*

The control sequence may also be an mRNA stabilizer region downstream of a promoter and upstream of the coding sequence of a gene which increases expression of the gene.

Examples of suitable mRNA stabilizer regions are obtained from a *Bacillus thuringiensis cryIIIA* gene (WO 94/25612) and a *Bacillus subtilis* SP82 gene (Hue et al., 1995, Journal of Bacteriology 177: 3465-3471).

The control sequence may also be a leader, a nontranslated region of an mRNA that is important for translation by the host cell. The leader is operably linked to the 5'-terminus of the polynucleotide encoding the polypeptide. Any leader that is functional in the host cell may be used.

Preferred leaders for filamentous fungal host cells are obtained from the genes for *Aspergillus oryzae* TAKA amylase and *Aspergillus nidulans* triose phosphate isomerase.

Suitable leaders for yeast host cells are obtained from the genes for *Saccharomyces cerevisiae* enolase (ENO-1), *Saccharomyces cerevisiae* 3-phosphoglycerate kinase, *Saccharomyces cerevisiae* alpha-factor, and *Saccharomyces cerevisiae* alcohol dehydrogenase/glyceraldehyde-3-phosphate dehydrogenase (ADH2/GAP).

The control sequence may also be a polyadenylation sequence, a sequence operably linked to the 3'-terminus of the polynucleotide and, when transcribed, is recognized by the host cell as a signal to add polyadenosine residues to transcribed mRNA. Any polyadenylation sequence that is functional in the host cell may be used.

Preferred polyadenylation sequences for filamentous fungal host cells are obtained from the genes for *Aspergillus nidulans* anthranilate synthase, *Aspergillus niger* glucoamylase, *Aspergillus niger* alpha-glucosidase *Aspergillus oryzae* TAKA amylase, and *Fusarium oxysporum* trypsin-like protease.

Useful polyadenylation sequences for yeast host cells are described by Guo and Sherman, 1995, Mol. Cellular Biol. 15: 5983-5990.

The control sequence may also be a signal peptide coding region that encodes a signal peptide linked to the N-terminus of a polypeptide and directs the polypeptide into the cell's secretory pathway. The 5'-end of the coding sequence of the polynucleotide may inherently contain a signal peptide coding sequence naturally linked in translation reading frame with the segment of the coding sequence that encodes the polypeptide. Alternatively, the 5'-end of the coding sequence may contain a signal peptide coding sequence that is foreign to the coding sequence. A foreign signal peptide coding sequence may be required where the coding sequence does not naturally contain a signal peptide coding sequence. Alternatively, a foreign signal peptide coding sequence may simply replace the natural signal peptide coding sequence in order to enhance secretion of the polypeptide. However, any signal peptide coding sequence that directs the expressed polypeptide into the secretory pathway of a host cell may be used.

Effective signal peptide coding sequences for bacterial host cells are the signal peptide coding sequences obtained from the genes for *Bacillus* NCIB 11837 maltogenic amylase, *Bacillus licheniformis* subtilisin, *Bacillus licheniformis* beta-lactamase, *Bacillus stearothermophilus* alpha-amylase, *Bacillus stearothermophilus* neutral proteases (*nprT, nprS, nprM*), and *Bacillus subtilis prsA.* Further signal peptides are described by Simonen and Palva, 1993, Microbiological Reviews 57: 109-137.

Effective signal peptide coding sequences for filamentous fungal host cells are the signal peptide coding sequences obtained from the genes for *Aspergillus niger* neutral amylase, *Aspergillus niger* glucoamylase, *Aspergillus oryzae* TAKA amylase, *Humicola insolens* cellulase, *Humicola insolens* endoglucanase V, *Humicola lanuginosa* lipase, and *Rhizomucor miehei* aspartic proteinase.

Useful signal peptides for yeast host cells are obtained from the genes for *Saccharomyces cerevisiae* alpha-factor and *Saccharomyces cerevisiae* invertase. Other useful signal peptide coding sequences are described by Romanos *et al.,* 1992, *supra.*

The control sequence may also be a propeptide coding sequence that encodes a propeptide positioned at the N-terminus of a polypeptide. The resultant polypeptide is known as a proenzyme or propolypeptide (or a zymogen in some cases). A propolypeptide is generally inactive and can be converted to an active polypeptide by catalytic or autocatalytic cleavage of the propeptide from the propolypeptide. The propeptide coding sequence may be obtained from the genes for *Bacillus subtilis* alkaline protease (*aprE*), *Bacillus subtilis* neutral protease (*nprT*), *Myceliophthora thermophila* laccase (WO 95/33836), *Rhizomucor miehei* aspartic proteinase, and *Saccharomyces cerevisiae* alpha-factor.

Where both signal peptide and propeptide sequences are present, the propeptide sequence is positioned next to the N-terminus of a polypeptide and the signal peptide sequence is positioned next to the N-terminus of the propeptide sequence.

It may also be desirable to add regulatory sequences that regulate expression of the polypeptide relative to the growth of the host cell. Examples of regulatory sequences are those that cause expression of the gene to be turned on or off in response to a chemical or physical stimulus, including the presence of a regulatory compound. Regulatory sequences in prokaryotic systems include the *lac, tac,* and *trp* operator systems. In yeast, the ADH2 system or GAL1 system may be used. In filamentous fungi, the *Aspergillus niger* glucoamylase promoter, *Aspergillus oryzae* TAKA alpha-amylase promoter, and *Aspergillus oryzae* glucoamylase promoter, *Trichoderma reesei* cellobiohydrolase I promoter, and *Trichoderma reesei* cellobiohydrolase II promoter may be used. Other examples of regulatory sequences are those that allow for gene amplification. In eukaryotic systems, these regulatory sequences include the dihydrofolate reductase gene that is amplified in the presence of methotrexate, and the metallothionein genes that are amplified with heavy

metals. In these cases, the polynucleotide encoding the polypeptide would be operably linked to the regulatory sequence.

**Expression Vectors**

The present invention also relates to recombinant expression vectors comprising a GH5 polynucleotide of the present invention, a promoter, and transcriptional and translational stop signals. The various nucleotide and control sequences may be joined together to produce a recombinant expression vector that may include one or more convenient restriction sites to allow for insertion or substitution of the polynucleotide encoding the polypeptide at such sites. Alternatively, the polynucleotide may be expressed by inserting the polynucleotide or a nucleic acid construct comprising the polynucleotide into an appropriate vector for expression. In creating the expression vector, the coding sequence is located in the vector so that the coding sequence is operably linked with the appropriate control sequences for expression.

The recombinant expression vector may be any vector (e.g., a plasmid or virus) that can be conveniently subjected to recombinant DNA procedures and can bring about expression of the polynucleotide. The choice of the vector will typically depend on the compatibility of the vector with the host cell into which the vector is to be introduced. The vector may be a linear or closed circular plasmid.

The vector may be an autonomously replicating vector, i.e., a vector that exists as an extrachromosomal entity, the replication of which is independent of chromosomal replication, *e.g.*, a plasmid, an extrachromosomal element, a minichromosome, or an artificial chromosome. The vector may contain any means for assuring self-replication. Alternatively, the vector may be one that, when introduced into the host cell, is integrated into the genome and replicated together with the chromosome(s) into which it has been integrated. Furthermore, a single vector or plasmid or two or more vectors or plasmids that together contain the total DNA to be introduced into the genome of the host cell, or a transposon, may be used.

The vector preferably contains one or more selectable markers that permit easy selection of transformed, transfected, transduced, or the like cells. A selectable marker is a gene the product of which provides for biocide or viral resistance, resistance to heavy metals, prototrophy to auxotrophs, and the like.

Examples of bacterial selectable markers are *Bacillus licheniformis* or *Bacillus subtilis dal* genes, or markers that confer antibiotic resistance such as ampicillin, chloramphenicol, kanamycin, neomycin, spectinomycin, or tetracycline resistance. Suitable markers for yeast host cells include, but are not limited to, ADE2, HIS3, LEU2, LYS2, MET3, TRP1, and URA3. Selectable markers for use in a filamentous fungal host cell include, but are not limited to, *adeA* (phosphoribosylaminoimidazole-succinocarboxamide synthase), *adeB* (phosphoribosylaminoimidazole synthase), *amdS* (acetamidase), *argB* (ornithine carbamoyltransferase), *bar* (phosphinothricin acetyltransferase), *hph* (hygromycin phosphotransferase), *niaD* (nitrate reductase), *pyrG* (orotidine-5'-phosphate decarboxylase), sC (sulfate adenyltransferase), and *trpC* (anthranilate synthase), as well as equivalents thereof. Preferred for use in an *Aspergillus* cell are *Aspergillus nidulans* or *Aspergillus oryzae amdS* and *pyrG* genes and a *Streptomyces hygroscopicus* bargene. Preferred for use in a *Trichoderma* cell are *adeA, adeB, amdS, hph,* and *pyrG* genes.

The selectable marker may be a dual selectable marker system as described in WO 2010/039889. In one aspect, the dual selectable marker is an *hph-tk* dual selectable marker system.

The vector preferably contains an element(s) that permits integration of the vector into the host cell's genome or autonomous replication of the vector in the cell independent of the genome.

For integration into the host cell genome, the vector may rely on the polynucleotide's sequence encoding the polypeptide or any other element of the vector for integration into the genome by homologous or non-homologous recombination. Alternatively, the vector may contain additional polynucleotides for directing integration by homologous recombination into the genome of the host cell at a precise location(s) in the chromosome(s). To increase the likelihood of integration at a precise location, the integrational elements should contain a sufficient number of nucleic acids, such as 100 to 10,000 base pairs, 400 to 10,000 base pairs, and 800 to 10,000 base pairs, which have a high degree of sequence identity to the corresponding target sequence to enhance the probability of homologous recombination. The integrational elements may be any sequence that is homologous with the target sequence in the genome of the host cell. Furthermore, the integrational elements may be non-encoding or encoding polynucleotides. On the other hand, the vector may be integrated into the genome of the host cell by non-homologous recombination.

For autonomous replication, the vector may further comprise an origin of replication enabling the vector to replicate autonomously in the host cell in question. The origin of replication may be any plasmid replicator mediating autonomous replication that functions in a cell. The term "origin of replication" or "plasmid replicator" means a polynucleotide that enables a plasmid or vector to replicate *in vivo.*

Examples of bacterial origins of replication are the origins of replication of plasmids pBR322, pUC19, pACYC177, and pACYC184 permitting replication in *E. coli,* and pUB110, pE194, pTA1060, and pAMß1 permitting replication in *Bacillus.*

Examples of origins of replication for use in a yeast host cell are the 2 micron origin of replication, ARS1, ARS4, the combination of ARS1 and CEN3, and the combination of ARS4 and CEN6.

Examples of origins of replication useful in a filamentous fungal cell are AMA1 and ANS1 (Gems et al., 1991, Gene 98: 61-67; Cullen et al., 1987, Nucleic Acids Res. 15: 9163-9175; WO 00/24883). Isolation of the AMA1 gene and construction of plasmids or vectors comprising the gene can be accomplished according to the methods disclosed in WO 00/24883.

More than one copy of a GH5 polynucleotide of the present invention may be inserted into a host cell to increase production of a polypeptide. An increase in the copy number of the polynucleotide can be obtained by integrating at least one additional copy of the sequence into the host cell genome or by including an amplifiable selectable marker gene with the polynucleotide where cells containing amplified copies of the selectable marker gene, and thereby additional copies of the polynucleotide, can be selected for by cultivating the cells in the presence of the appropriate selectable agent.

The procedures used to ligate the elements described above to construct the recombinant expression vectors of the present invention are well known to one skilled in the art (see, *e.g.*, Sambrook *et al.,* 1989, *supra).*

**Host Cells**

The present invention also relates to recombinant host cells, comprising a GH5 polynucleotide of the present invention operably linked to one or more control sequences that direct the production of a polypeptide of the present invention. A construct or vector comprising a polynucleotide is introduced into a host cell so that the construct or vector is maintained as a chromosomal integrant or as a self-replicating extra-chromosomal vector as described earlier. The term "host cell" encompasses any progeny of a parent cell that is not identical to the parent cell due to mutations that occur during replication. The choice of a host cell will to a large extent depend upon the gene encoding the polypeptide and its source.

The host cell may be any cell useful in the recombinant production of a polypeptide of the present invention, *e.g.*, a prokaryote or a eukaryote.

The prokaryotic host cell may be any Gram-positive or Gram-negative bacterium. Gram-positive bacteria include, but are not limited to, *Bacillus, Clostridium, Enterococcus, Geobacillus, Lactobacillus, Lactococcus, Oceanobacillus, Staphylococcus, Streptococcus,* and *Streptomyces*. Gram-negative bacteria include, but are not limited to, *Campylobacter, E. coli, Flavobacterium, Fusobacterium, Helicobacter, Ilyobacter, Neisseria, Pseudomonas, Salmonella,* and *Ureaplasma.*

The bacterial host cell may be any Bacillus cell including, but not limited to, Bacillus alkalophilus, Bacillus amyloliquefaciens, Bacillus brevis, Bacillus circulans, Bacillus clausii, Bacillus coagulans, Bacillus firmus, Bacillus lautus, Bacillus lentus, Bacillus licheniformis, Bacillus megaterium, Bacillus pumilus, Bacillus stearothermophilus, Bacillus subtilis, and Bacillus thuringiensis cells.

The bacterial host cell may also be any Streptococcus cell including, but not limited to, Streptococcus equisimilis, Streptococcus pyogenes, Streptococcus uberis, and Streptococcus equi subsp. Zooepidemicus cells.

The bacterial host cell may also be any Streptomyces cell including, but not limited to, Streptomyces achromogenes, Streptomyces avermitilis, Streptomyces coelicolor, Streptomyces griseus, and Streptomyces lividans cells.

The introduction of DNA into a *Bacillus* cell may be effected by protoplast transformation (see, *e.g.,* Chang and Cohen, 1979, Mol. Gen. Genet. 168: 111-115), competent cell transformation (see, *e.g.,* Young and Spizizen, 1961, J. Bacteriol. 81: 823-829, or Dubnau and Davidoff-Abelson, 1971, J. Mol. Biol. 56: 209-221), electroporation (see, *e.g.,* Shigekawa and Dower, 1988, Biotechniques 6: 742-751), or conjugation (see, *e.g.,* Koehler and Thorne, 1987, J. Bacteriol. 169: 5271-5278). The introduction of DNA into an *E. coli* cell may be effected by protoplast transformation (see, *e.g.,* Hanahan, 1983, J. Mol. Biol. 166: 557-580) or electroporation (see, *e.g.,* Dower et al., 1988, Nucleic Acids Res. 16: 6127-6145). The introduction of DNA into a *Streptomyces* cell may be effected by protoplast transformation, electroporation (see, *e.g.,* Gong et al., 2004, Folia Microbiol. (Praha) 49: 399-405), conjugation (see, *e.g.,* Mazodier et al., 1989, J. Bacteriol. 171: 3583-3585), or transduction (see, *e.g.,* Burke et al., 2001, Proc. Natl. Acad. Sci. USA 98: 6289-6294). The introduction of DNA into a *Pseudomonas* cell may be effected by electroporation (see, *e.g.,* Choi et al., 2006, J. Microbiol. Methods 64: 391-397) or conjugation (see, *e.g.,* Pinedo and Smets, 2005, Appl. Environ. Microbiol. 71: 51-57). The introduction of DNA into a *Streptococcus* cell may be effected by natural competence (see, *e.g.,* Perry and Kuramitsu, 1981, Infect. Immun. 32: 1295-1297), protoplast transformation (see, *e.g.,* Catt and Jollick, 1991, Microbios 68: 189-207), electroporation (see, *e.g.,* Buckley et al., 1999, Appl. Environ. Microbiol. 65: 3800-3804), or conjugation (see, *e.g.,* Clewell, 1981, Microbiol. Rev. 45: 409-436). However, any method known in the art for introducing DNA into a host cell can be used.

The host cell may also be a eukaryote, such as a mammalian, insect, plant, or fungal cell.

The host cell may be a fungal cell. "Fungi" as used herein includes the phyla *Ascomycota, Basidiomycota, Chytridiomycota,* and *Zygomycota* as well as the *Oomycota* and all mitosporic fungi (as defined by Hawksworth et al., In, Ainsworth and Bisby's Dictionary of The Fungi, 8th edition, 1995, CAB International, University Press, Cambridge, UK).

The fungal host cell may be a yeast cell. "Yeast" as used herein includes ascosporogenous yeast (*Endomycetales*), basidiosporogenous yeast, and yeast belonging to the *Fungi Imperfecti* (*Blastomycetes*). Since the classification

of yeast may change in the future, for the purposes of this invention, yeast shall be defined as described in Biology and Activities of Yeast (Skinner, Passmore, and Davenport, editors, Soc. App. Bacteriol. Symposium Series No. 9, 1980).

The yeast host cell may be a *Candida, Hansenula, Kluyveromyces, Pichia, Saccharomyces, Schizosaccharomyces,* or *Yarrowia* cell, such as a *Kluyveromyces lactis, Saccharomyces carlsbergensis, Saccharomyces cerevisiae, Saccharomyces diastaticus, Saccharomyces douglasii, Saccharomyces kluyveri, Saccharomyces norbensis, Saccharomyces oviformis, or Yarrowia lipolytica* cell.

The fungal host cell may be a filamentous fungal cell. "Filamentous fungi" include all filamentous forms of the subdivision Eumycota and Oomycota (as defined by Hawksworth *et al.,* 1995, *supra*). The filamentous fungi are generally characterized by a mycelial wall composed of chitin, cellulose, glucan, chitosan, mannan, and other complex polysaccharides. Vegetative growth is by hyphal elongation and carbon catabolism is obligately aerobic. In contrast, vegetative growth by yeasts such as *Saccharomyces cerevisiae* is by budding of a unicellular thallus and carbon catabolism may be fermentative.

The filamentous fungal host cell may be an *Acremonium, Aspergillus, Aureobasidium, Bjerkandera, Ceriporiopsis, Chrysosporium, Coprinus, Coriolus, Cryptococcus, Filibasidium, Fusarium, Humicola, Magnaporthe, Mucor, Myceliophthora, Neocallimastix, Neurospora, Paecilomyces, Penicillium, Phanerochaete, Phlebia, Piromyces, Pleurotus, Schizophyllum, Talaromyces, Thermoascus, Thielavia, Tolypocladium, Trametes,* or *Trichoderma* cell.

For example, the filamentous fungal host cell may be an *Aspergillus awamori, Aspergillus foetidus, Aspergillus fumigatus, Aspergillus japonicus, Aspergillus nidulans, Aspergillus niger, Aspergillus oryzae, Bjerkandera adusta, Ceriporiopsis aneirina, Ceriporiopsis caregiea, Ceriporiopsis gilvescens, Ceriporiopsis pannocinta, Ceriporiopsis rivulosa, Ceriporiopsis subrufa, Ceriporiopsis subvermispora, Chrysosporium inops, Chrysosporium keratinophilum, Chrysosporium lucknowense, Chrysosporium merdarium, Chrysosporium pannicola, Chrysosporium queenslandicum, Chrysosporium tropicum, Chrysosporium zonatum, Coprinus cinereus, Coriolus hirsutus, Fusarium bactridioides, Fusarium cerealis, Fusarium crookwellense, Fusarium culmorum, Fusarium graminearum, Fusarium graminum, Fusarium heterosporum, Fusarium negundi, Fusarium oxysporum, Fusarium reticulatum, Fusarium roseum, Fusarium sambucinum, Fusarium sarcochroum, Fusarium sporotrichioides, Fusarium sulphureum, Fusarium torulosum, Fusarium trichothecioides, Fusarium venenatum, Humicola insolens, Humicola lanuginosa, Mucor miehei, Myceliophthora thermophila, Neurospora crassa, Penicillium purpurogenum, Phanerochaete chrysosporium, Phlebia radiata, Pleurotus eryngii, Thielavia terrestris, Trametes villosa, Trametes versicolor, Trichoderma harzianum, Trichoderma koningii, Trichoderma longibrachiatum, Trichoderma reesei,* or *Trichoderma viride* cell.

Fungal cells may be transformed by a process involving protoplast formation, transformation of the protoplasts, and regeneration of the cell wall in a manner known *per se.* Suitable procedures for transformation of *Aspergillus* and *Trichoderma* host cells are described in EP 238023, Yelton et al., 1984, Proc. Natl. Acad. Sci. USA 81: 1470-1474, and Christensen et al., 1988, Bio/Technology 6: 1419-1422. Suitable methods for transforming *Fusarium* species are described by Malardier et al., 1989, Gene 78: 147-156, and WO 96/00787. Yeast may be transformed using the procedures described by Becker and Guarente, In Abelson, J.N. and Simon, M.I., editors, Guide to Yeast Genetics and Molecular Biology, Methods in Enzymology, Volume 194, pp 182-187, Academic Press, Inc., New York; Ito et al., 1983, J. Bacteriol. 153: 163; and Hinnen et al., 1978, Proc. Natl. Acad. Sci. USA 75: 1920.

**Methods of Production**

The present invention also relates to methods of producing a polypeptide of the present invention, comprising (a) cultivating a cell, which in its wild-type form produces the polypeptide, under conditions conducive for production of the polypeptide; and optionally, (b) recovering the polypeptide. In one aspect, the cell is an *Opitutaceae* species cell.

The present invention also relates to methods of producing a polypeptide of the present invention, comprising (a) cultivating a recombinant host cell of the present invention under conditions conducive for production of the polypeptide; and optionally, (b) recovering the polypeptide.

The host cells are cultivated in a nutrient medium suitable for production of the polypeptide using methods known in the art. For example, the cells may be cultivated by shake flask cultivation, or small-scale or large-scale fermentation (including continuous, batch, fed-batch, or solid state fermentations) in laboratory or industrial fermentors in a suitable medium and under conditions allowing the polypeptide to be expressed and/or isolated. The cultivation takes place in a suitable nutrient medium comprising carbon and nitrogen sources and inorganic salts, using procedures known in the art. Suitable media are available from commercial suppliers or may be prepared according to published compositions (*e.g*., in catalogues of the American Type Culture Collection). If the polypeptide is secreted into the nutrient medium, the polypeptide can be recovered directly from the medium. If the polypeptide is not secreted, it can be recovered from cell lysates.

The polypeptide may be detected using methods known in the art that are specific for the polypeptides. These detection methods include, but are not limited to, use of specific antibodies, formation of an enzyme product, or disappearance of an enzyme substrate. For example, an enzyme assay may be used to determine the activity of the polypeptide.

The polypeptide may be recovered using methods known in the art. For example, the polypeptide may be recovered from the nutrient medium by conventional procedures including, but not limited to, collection, centrifugation, filtration, extraction, spray-drying, evaporation, or precipitation. In one aspect, a fermentation broth comprising the polypeptide is recovered.

The polypeptide may be purified by a variety of procedures known in the art including, but not limited to, chromatography (*e.g.*, ion exchange, affinity, hydrophobic, chromatofocusing, and size exclusion), electrophoretic procedures (*e.g.*, preparative isoelectric focusing), differential solubility (*e.g.,* ammonium sulfate precipitation), SDS-PAGE, or extraction (see, *e.g.,* Protein Purification, Janson and Ryden, editors, VCH Publishers, New York, 1989) to obtain substantially pure polypeptides.

In an alternative aspect, the polypeptide is not recovered, but rather a host cell of the present invention expressing the polypeptide is used as a source of the polypeptide.

**Fermentation Broth Formulations or Cell Compositions**

The present invention also relates to a fermentation broth formulation or a cell composition comprising a polypeptide of the present invention. The fermentation broth product further comprises additional ingredients used in the fermentation process, such as, for example, cells (including, the host cells containing the gene encoding the polypeptide of the present invention which are used to produce the polypeptide of interest), cell debris, biomass, fermentation media and/or fermentation products. In some embodiments, the composition is a cell-killed whole broth containing organic acid(s), killed cells and/or cell debris, and culture medium.

The term "fermentation broth" as used herein refers to a preparation produced by cellular fermentation that undergoes no or minimal recovery and/or purification. For example, fermentation broths are produced when microbial cultures are grown to saturation, incubated under carbon-limiting conditions to allow protein synthesis (*e.g.*, expression of enzymes by host cells) and secretion into cell culture medium. The fermentation broth can contain unfractionated or fractionated contents of the fermentation materials derived at the end of the fermentation. Typically, the fermentation broth is unfractionated and comprises the spent culture medium and cell debris present after the microbial cells (*e.g.,* filamentous fungal cells) are removed, *e.g.,* by centrifugation. In some embodiments, the fermentation broth contains spent cell culture medium, extracellular enzymes, and viable and/or nonviable microbial cells.

In an embodiment, the fermentation broth formulation and cell compositions comprise a first organic acid component comprising at least one 1-5 carbon organic acid and/or a salt thereof and a second organic acid component comprising at least one 6 or more carbon organic acid and/or a salt thereof. In a specific embodiment, the first organic acid component is acetic acid, formic acid, propionic acid, a salt thereof, or a mixture of two or more of the foregoing and the second organic acid component is benzoic acid, cyclohexanecarboxylic acid, 4-methylvaleric acid, phenylacetic acid, a salt thereof, or a mixture of two or more of the foregoing.

In one aspect, the composition contains an organic acid(s), and optionally further contains killed cells and/or cell debris. In one embodiment, the killed cells and/or cell debris are removed from a cell-killed whole broth to provide a composition that is free of these components.

The fermentation broth formulations or cell compositions may further comprise a preservative and/or antimicrobial (*e.g.*, bacteriostatic) agent, including, but not limited to, sorbitol, sodium chloride, potassium sorbate, and others known in the art.

The cell-killed whole broth or composition may contain the unfractionated contents of the fermentation materials derived at the end of the fermentation. Typically, the cell-killed whole broth or composition contains the spent culture medium and cell debris present after the microbial cells (*e.g.*, filamentous fungal cells) are grown to saturation, incubated under carbon-limiting conditions to allow protein synthesis. In some embodiments, the cell-killed whole broth or composition contains the spent cell culture medium, extracellular enzymes, and killed filamentous fungal cells. In some embodiments, the microbial cells present in the cell-killed whole broth or composition can be permeabilized and/or lysed using methods known in the art.

A whole broth or cell composition as described herein is typically a liquid, but may contain insoluble components, such as killed cells, cell debris, culture media components, and/or insoluble enzyme(s). In some embodiments, insoluble components may be removed to provide a clarified liquid composition.

The whole broth formulations and cell compositions of the present invention may be produced by a method described in WO 90/15861 or WO 2010/096673.

**Detergent composition**

The polypeptide of the present invention may be added to a detergent composition in an amount corresponding to 0.0001-200 mg of enzyme protein, such as 0.0005-100 mg of enzyme protein, preferably 0.001-30 mg of enzyme protein, more preferably 0.005-8 mg of enzyme protein, even more preferably 0.01-2 mg of enzyme protein per litre of

wash liquor.

A composition for use in automatic dishwash (ADW), for example, may include 0.0001%-50%, such as 0.001%-20%, such as 0.01%-10%, such as 0.05-5% of enzyme protein by weight of the composition.

A composition for use in laundry powder, for example, may include 0.0001%-50%, such as 0.001%-20%, such as 0.01%-10%, such as 0.05%-5% of enzyme protein by weight of the composition.

A composition for use in laundry liquid, for example, may include 0.0001%-10%, such as 0.001-7%, such as 0.1%-5% of enzyme protein by weight of the composition.

The enzyme(s) of the detergent composition may be stabilized using conventional stabilizing agents, e.g., a polyol such as propylene glycol or glycerol, a sugar or sugar alcohol, lactic acid, boric acid, or a boric acid derivative, e.g., an aromatic borate ester, or a phenyl boronic acid derivative such as 4-formylphenyl boronic acid, and the composition may be formulated as described in, for example, WO92/19709 and WO92/19708.

In certain markets different wash conditions and, as such, different types of detergents are used. This is disclosed in e.g. EP 1 025 240. For example, In Asia (Japan) a low detergent concentration system is used, while the United States uses a medium detergent concentration system, and Europe uses a high detergent concentration system.

A detergent composition may comprise an enzyme of the present invention in combination with one or more additional cleaning composition components. The choice of additional components is within the skill of the artisan and includes conventional ingredients, including the exemplary non-limiting components set forth below.

The choice of components may include, for textile care, the consideration of the type of textile to be cleaned, the type and/or degree of soiling, the temperature at which cleaning is to take place, and the formulation of the detergent product. Although components mentioned below are categorized by general header according to a particular functionality, this is not to be construed as a limitation, as a component may comprise additional functionalities as will be appreciated by the skilled artisan.

An ADW (Automatic Dish Wash) composition may comprise an enzyme of the present invention in combination with one or more additional ADW composition components. The choice of additional components is within the skill of the artisan and includes conventional ingredients, including the exemplary non-limiting components set forth below.

**Surfactants**

The detergent composition may comprise one or more surfactants, which may be anionic and/or cationic and/or non-ionic and/or semi-polar and/or zwitterionic, or a mixture thereof. In a particular embodiment, the detergent composition includes a mixture of one or more nonionic surfactants and one or more anionic surfactants. The surfactant(s) is typically present at a level of from about 0.1% to 60% by weight, such as about 1% to about 40%, or about 3% to about 20%, or about 3% to about 10%. The surfactant(s) is chosen based on the desired cleaning application, and may include any conventional surfactant(s) known in the art.

When included therein the detergent will usually contain from about 1% to about 40% by weight of an anionic surfactant, such as from about 5% to about 30%, including from about 5% to about 15%, or from about 15% to about 20%, or from about 20% to about 25% of an anionic surfactant. Non-limiting examples of anionic surfactants include sulfates and sulfonates, in particular, linear alkylbenzenesulfonates (LAS), isomers of LAS, branched alkylbenzenesulfonates (BABS), phenylalkanesulfonates, alpha-olefinsulfonates (AOS), olefin sulfonates, alkene sulfonates, alkane-2,3-diylbis(sulfates), hydroxyalkanesulfonates and disulfonates, alkyl sulfates (AS) such as sodium dodecyl sulfate (SDS), fatty alcohol sulfates (FAS), primary alcohol sulfates (PAS), alcohol ethersulfates (AES or AEOS or FES, also known as alcohol ethoxysulfates or fatty alcohol ether sulfates), secondary alkanesulfonates (SAS), paraffin sulfonates (PS), ester sulfonates, sulfonated fatty acid glycerol esters, alpha-sulfo fatty acid methyl esters (alpha-SFMe or SES) including methyl ester sulfonate (MES), alkyl- or alkenylsuccinic acid, dodecenyl/tetradecenyl succinic acid (DTSA), fatty acid derivatives of amino acids, diesters and monoesters of sulfo-succinic acid or salt of fatty acids (soap), and combinations thereof.

When included therein the detergent will usually contain from about 1% to about 40% by weigh of a cationic surfactant, for example from about 0.5% to about 30%, in particular from about 1% to about 20%, from about 3% to about 10%, such as from about 3% to about 5%, from about 8% to about 12% or from about 10% to about 12%. Non-limiting examples of cationic surfactants include alkyldimethylethanolamine quat (ADMEAQ), cetyltrimethylammonium bromide (CTAB), dimethyldistearylammonium chloride (DSDMAC), and alkylbenzyldimethylammonium, alkyl quaternary ammonium compounds, alkoxylated quaternary ammonium (AQA) compounds, ester quats, and combinations thereof.

When included therein the detergent will usually contain from about 0.2% to about 40% by weight of a nonionic surfactant, for example from about 0.5% to about 30%, in particular from about 1% to about 20%, from about 3% to about 10%, such as from about 3% to about 5%, from about 8% to about 12%, or from about 10% to about 12%. Non-limiting examples of nonionic surfactants include alcohol ethoxylates (AE or AEO), alcohol propoxylates, propoxylated fatty alcohols (PFA), alkoxylated fatty acid alkyl esters, such as ethoxylated and/or propoxylated fatty acid alkyl esters, alkylphenol ethoxylates (APE), nonylphenol ethoxylates (NPE), alkylpolyglycosides (APG), alkoxylated amines, fatty

acid monoethanolamides (FAM), fatty acid diethanolamides (FADA), ethoxylated fatty acid monoethanolamides (EFAM), propoxylated fatty acid monoethanolamides (PFAM), polyhydroxyalkyl fatty acid amides, or *N*-acyl *N*-alkyl derivatives of glucosamine (glucamides, GA, or fatty acid glucamides, FAGA), as well as products available under the trade names SPAN and TWEEN, and combinations thereof.

When included therein the detergent will usually contain from about 0% to about 10% by weight of a semipolar surfactant. Non-limiting examples of semipolar surfactants include amine oxides (AO) such as alkyldimethylamineoxide, *N*-(coco alkyl)-*N*,*N*-dimethylamine oxide and N-(tallow-alkyl)-*N*,*N*-bis(2-hydroxyethyl)amine oxide, , and combinations thereof.

When included therein the detergent will usually contain from about 0% to about 10% by weight of a zwitterionic surfactant. Non-limiting examples of zwitterionic surfactants include betaines such as alkyldimethylbetaines, sulfobetaines, and combinations thereof.

**Hydrotropes**

A hydrotrope is a compound that solubilises hydrophobic compounds in aqueous solutions (or oppositely, polar substances in a non-polar environment). Typically, hydrotropes have both hydrophilic and a hydrophobic character (so-called amphiphilic properties as known from surfactants); however the molecular structure of hydrotropes generally do not favor spontaneous self-aggregation, see e.g. review by Hodgdon and Kaler (2007), Current Opinion in Colloid & Interface Science 12: 121-128. Hydrotropes do not display a critical concentration above which self-aggregation occurs as found for surfactants and lipids forming miceller, lamellar or other well defined meso-phases. Instead, many hydrotropes show a continuous-type aggregation process where the sizes of aggregates grow as concentration increases. However, many hydrotropes alter the phase behavior, stability, and colloidal properties of systems containing substances of polar and non-polar character, including mixtures of water, oil, surfactants, and polymers. Hydrotropes are classically used across industries from pharma, personal care, food, to technical applications. Use of hydrotropes in detergent compositions allow for example more concentrated formulations of surfactants (as in the process of compacting liquid detergents by removing water) without inducing undesired phenomena such as phase separation or high viscosity.

The detergent may contain 0-10% by weight, for example 0-5% by weight, such as about 0.5 to about 5%, or about 3% to about 5%, of a hydrotrope. Any hydrotrope known in the art for use in detergents may be utilized. Non-limiting examples of hydrotropes include sodium benzenesulfonate, sodium p-toluene sulfonate (STS), sodium xylene sulfonate (SXS), sodium cumene sulfonate (SCS), sodium cymene sulfonate, amine oxides, alcohols and polyglycolethers, sodium hydroxynaphthoate, sodium hydroxynaphthalene sulfonate, sodium ethylhexyl sulfate, and combinations thereof.

**Builders and Co-Builders**

The detergent composition may contain about 0-65% by weight, such as about 5% to about 50% of a detergent builder or co-builder, or a mixture thereof. In a dish wash detergent, the level of builder is typically 40-65%, particularly 50-65%. The builder and/or co-builder may particularly be a chelating agent that forms water-soluble complexes with Ca and Mg. Any builder and/or co-builder known in the art for use in detergents may be utilized. Non-limiting examples of builders include zeolites, diphosphates (pyrophosphates), triphosphates such as sodium triphosphate (STP or STPP), carbonates such as sodium carbonate, soluble silicates such as sodium metasilicate, layered silicates (e.g., SKS-6 from Hoechst), ethanolamines such as 2-aminoethan-1-ol (MEA), diethanolamine (DEA, also known as 2,2'-iminodiethan-1-ol), triethanolamine (TEA, also known as 2,2',2"-nitrilotriethan-1-ol), and (carboxymethyl)inulin (CMI), and combinations thereof.

The detergent composition may also contain 0-50% by weight, such as about 5% to about 30%, of a detergent co-builder. The detergent composition may include include a co-builder alone, or in combination with a builder, for example a zeolite builder. Non-limiting examples of co-builders include homopolymers of polyacrylates or copolymers thereof, such as poly(acrylic acid) (PAA) or copoly(acrylic acid/maleic acid) (PAA/PMA). Further non-limiting examples include citrate, chelators such as aminocarboxylates, aminopolycarboxylates and phosphonates, and alkyl- or alkenylsuccinic acid. Additional specific examples include 2,2',2"-nitrilotriacetic acid (NTA), ethylenediaminetetraacetic acid (EDTA), diethylenetriaminepentaacetic acid (DTPA), iminodisuccinic acid (IDS), ethylenediamine-*N*,*N'*-disuccinic acid (EDDS), methylglycinediacetic acid (MGDA), glutamic acid-*N,N*-diacetic acid (GLDA), 1-hydroxyethane-1,1-diphosphonic acid (HEDP), ethylenediaminetetra(methylenephosphonic acid) (EDTMPA), diethylenetriaminepentakis(methylenephosphonic acid) (DTMPA or DTPMPA), N-(2-hydroxyethyl)iminodiacetic acid (EDG), aspartic acid-*N*-monoacetic acid (ASMA), aspartic acid-N,N-diacetic acid (ASDA), aspartic acid-*N*-monopropionic acid (ASMP), iminodisuccinic acid (IDA), N-(2-sulfomethyl)-aspartic acid (SMAS), *N*-(2-sulfoethyl)-aspartic acid (SEAS), *N*-(2-sulfomethyl)-glutamic acid (SMGL), *N*-(2-sulfoethyl)-glutamic acid (SEGL), *N*-methyliminodiacetic acid (MIDA), alpha-alanine-*N,N*-diacetic acid (α-ALDA), serine-*N,N*-diacetic acid (SEDA), isoserine-*N,N*-diacetic acid (ISDA), phenylalanine-*N,N*-diacetic acid (PHDA),

anthranilic acid-*N,N*-diacetic acid (ANDA), sulfanilic acid-*N,N*-diacetic acid (SLDA), taurine-*N,N*-diacetic acid (TUDA) and sulfomethyl-*N,N*-diacetic acid (SMDA), *N*-(2-hydroxyethyl)ethylenediamine-*N,N',N"*-triacetic acid (HEDTA), diethanolglycine (DEG), diethylenetriamine penta(methylenephosphonic acid) (DTPMP), aminotris(methylenephosphonic acid) (ATMP), and combinations and salts thereof. Further exemplary builders and/or co-builders are described in, e.g., WO 09/102854, US 5977053

**Bleaching Systems**

The detergent may contain 0-30% by weight, such as about 1% to about 20%, of a bleaching system. Any bleaching system known in the art for use in detergents may be utilized. Suitable bleaching system components include bleaching catalysts, photobleaches, bleach activators, sources of hydrogen peroxide such as sodium percarbonate, sodium perborates and hydrogen peroxide-urea (1:1), preformed peracids and mixtures thereof. Suitable preformed peracids include, but are not limited to, peroxycarboxylic acids and salts, diperoxydicarboxylic acids, perimidic acids and salts, peroxymonosulfuric acids and salts, for example, Oxone (R), and mixtures thereof. Non-limiting examples of bleaching systems include peroxide-based bleaching systems, which may comprise, for example, an inorganic salt, including alkali metal salts such as sodium salts of perborate (usually mono- or tetra-hydrate), percarbonate, persulfate, perphosphate, persilicate salts, in combination with a peracid-forming bleach activator. The term bleach activator is meant herein as a compound which reacts with hydrogen peroxide to form a peracid via perhydrolysis. The peracid thus formed constitutes the activated bleach. Suitable bleach activators to be used herein include those belonging to the class of esters, amides, imides or anhydrides. Suitable examples are tetraacetylethylenediamine (TAED), sodium 4-[(3,5,5-trimethylhexanoyl)oxy]benzene-1-sulfonate (ISONOBS), 4-(dodecanoyloxy)benzene-1-sulfonate (LOBS), 4-(decanoyloxy)benzene-1-sulfonate, 4-(decanoyloxy)benzoate (DOBS or DOBA), 4-(nonanoyloxy)benzene-1-sulfonate (NOBS), and/or those disclosed in WO98/17767. A particular family of bleach activators of interest was disclosed in EP624154 and particularly preferred in that family is acetyl triethyl citrate (ATC). ATC or a short chain triglyceride like triacetin has the advantage that it is environmentally friendly Furthermore acetyl triethyl citrate and triacetin have good hydrolytical stability in the product upon storage and are efficient bleach activators. Finally ATC is multifunctional, as the citrate released in the perhydrolysis reaction may function as a builder. Alternatively, the bleaching system may comprise peroxyacids of, for example, the amide, imide, or sulfone type. The bleaching system may also comprise peracids such as 6-(phthalimido)peroxyhexanoic acid (PAP). The bleaching system may also include a bleach catalyst. In some embodiments the bleach component may be an organic catalyst selected from the group consisting of organic catalysts having the following formulae:

(i)

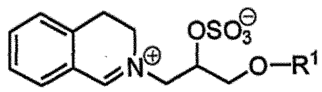

(ii)

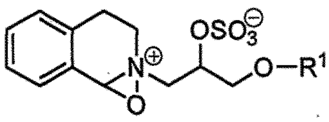

(iii) and mixtures thereof;

wherein each $R^1$ is independently a branched alkyl group containing from 9 to 24 carbons or linear alkyl group containing from 11 to 24 carbons, preferably each $R^1$ is independently a branched alkyl group containing from 9 to 18 carbons or linear alkyl group containing from 11 to 18 carbons, more preferably each $R^1$ is independently selected from the group consisting of 2-propylheptyl, 2-butyloctyl, 2-pentylnonyl, 2-hexyldecyl, dodecyl, tetradecyl, hexadecyl, octadecyl, isononyl, isodecyl, isotridecyl and isopentadecyl. Other exemplary bleaching systems are described, e.g. in WO2007/087258, WO2007/087244, WO2007/087259, EP1867708 (Vitamin K) and WO2007/087242. Suitable photobleaches may for example be sulfonated zinc or aluminium phthalocyanines.

Preferably the bleach component comprises a source of peracid in addition to bleach catalyst, particularly organic bleach catalyst. The source of peracid may be selected from (a) preformed peracid; (b) percarbonate, perborate or persulfate salt (hydrogen peroxide source) preferably in combination with a bleach activator; and (c) perhydrolase enzyme and an ester for forming peracid in situ in the presence of water in a textile or hard surface treatment step.

**Polymers**

The detergent may contain 0-10% by weight, such as 0.5-5%, 2-5%, 0.5-2% or 0.2-1% of a polymer. Any polymer known in the art for use in detergents may be utilized. The polymer may function as a co-builder as mentioned above, or may provide antiredeposition, fiber protection, soil release, dye transfer inhibition, grease cleaning and/or anti-foaming properties. Some polymers may have more than one of the above-mentioned properties and/or more than one of the below-mentioned motifs. Exemplary polymers include (carboxymethyl)cellulose (CMC), poly(vinyl alcohol) (PVA), poly(vinylpyrrolidone) (PVP), poly(ethyleneglycol) or poly(ethylene oxide) (PEG), ethoxylated poly(ethyleneimine), carboxymethyl inulin (CMI), and polycarboxylates such as PAA, PAA/PMA, poly-aspartic acid, and lauryl methacrylate/acrylic acid copolymers , hydrophobically modified CMC (HM-CMC) and silicones, copolymers of terephthalic acid and oligomeric glycols, copolymers of poly(ethylene terephthalate) and poly(oxyethene terephthalate) (PET-POET), PVP, poly(vinylimidazole) (PVI), poly(vinylpyridine-*N*-oxide) (PVPO or PVPNO) and polyvinylpyrrolidone-vinylimidazole (PVPVI). Further exemplary polymers include sulfonated polycarboxylates, polyethylene oxide and polypropylene oxide (PEO-PPO) and diquaternium ethoxy sulfate. Other exemplary polymers are disclosed in, e.g., WO 2006/130575. Salts of the above-mentioned polymers are also contemplated.

**Fabric hueing agents**

The detergent compositions may also include fabric hueing agents such as dyes or pigments, which when formulated in detergent compositions can deposit onto a fabric when said fabric is contacted with a wash liquor comprising said detergent compositions and thus altering the tint of said fabric through absorption/reflection of visible light. Fluorescent whitening agents emit at least some visible light. In contrast, fabric hueing agents alter the tint of a surface as they absorb at least a portion of the visible light spectrum. Suitable fabric hueing agents include dyes and dye-clay conjugates, and may also include pigments. Suitable dyes include small molecule dyes and polymeric dyes. Suitable small molecule dyes include small molecule dyes selected from the group consisting of dyes falling into the Colour Index (C.I.) classifications of Direct Blue, Direct Red, Direct Violet, Acid Blue, Acid Red, Acid Violet, Basic Blue, Basic Violet and Basic Red, or mixtures thereof, for example as described in WO2005/03274, WO2005/03275, WO2005/03276 and EP1876226. The detergent composition preferably comprises from about 0.00003 wt% to about 0.2 wt%, from about 0.00008 wt% to about 0.05 wt%, or even from about 0.0001 wt% to about 0.04 wt% fabric hueing agent. The composition may comprise from 0.0001 wt% to 0.2 wt% fabric hueing agent, this may be especially preferred when the composition is in the form of a unit dose pouch. Suitable hueing agents are also disclosed in, e.g. WO 2007/087257 and WO2007/087243.

**Additional enzymes**

The detergent additive as well as the detergent composition may comprise one or more additional enzymes such as a protease, a lipase, a cutinase, an amylase, a carbohydrase, a cellulase, a pectinase, a mannanase, an arabinase, a galactanase, a xylanase, an oxidase, *e.g*., a laccase, and/or a peroxidase and/or a xanthan lyase.

In general the properties of the selected enzyme(s) should be compatible with the selected detergent, (*i.e*., pH-optimum, compatibility with other enzymatic and non-enzymatic ingredients, etc.), and the enzyme(s) should be present in effective amounts.

**Cellulases**

Suitable cellulases include those of bacterial or fungal origin. Chemically modified or protein engineered mutants are included. Suitable cellulases include cellulases from the genera *Bacillus, Pseudomonas, Humicola, Fusarium, Thielavia, Acremonium, e.g.,* the fungal cellulases produced from *Humicola insolens, Myceliophthora thermophila* and *Fusarium oxysporum* disclosed in US 4,435,307, US 5,648,263, US 5,691,178, US 5,776,757 and WO 89/09259.

Especially suitable cellulases are the alkaline or neutral cellulases having colour care benefits. Examples of such cellulases are cellulases described in EP 0 495 257, EP 0 531 372, WO 96/11262, WO 96/29397, WO 98/08940. Other examples are cellulase variants such as those described in WO 94/07998, EP 0 531 315, US 5,457,046, US 5,686,593, US 5,763,254, WO 95/24471, WO 98/12307 and WO99/001544.

Other cellulases are endo-beta-1,4-glucanase enzyme having a sequence of at least 97% identity to the amino acid sequence of position 1 to position 773 of SEQ ID NO:2 of WO 2002/099091 or a family 44 xyloglucanase, which a xyloglucanase enzyme having a sequence of at least 60% identity to positions 40-559 of SEQ ID NO: 2 of WO 2001/062903.

Commercially available cellulases include Celluzyme™, and Carezyme™ (Novozymes A/S) Carezyme Premium™ (Novozymes A/S), Celluclean™ (Novozymes A/S), Celluclean Classic™ (Novozymes A/S), Cellusoft™

(Novozymes A/S), Whitezyme™ (Novozymes A/S), Clazinase™, and Puradax HA™ (Genencor International Inc.), and KAC-500(B)™ (Kao Corporation).

**Mannanases**

Suitable mannanases include those of bacterial or fungal origin. Chemically or genetically modified mutants are included. The mannanase may be an alkaline mannanase of Family 5 or 26. It may be a wild-type from *Bacillus* or *Humicola,* particularly *B. agaradhaerens, B. licheniformis, B. halodurans, B. clausii,* or *H. insolens.* Suitable mannanases are described in WO 1999/064619. A commercially available mannanase is Mannaway (Novozymes A/S).

**Xanthan lyases**

Suitable xanthan lyases include those of plant, bacterial or fungal origin. Chemically modified or protein engineered mutants are included. Examples of useful enzymes include the xanthan lyases disclosed in WO2013167581 and shown herein as SEQ ID NO:21, 22, 23 and 24.

**Proteases**

Suitable proteases include those of bacterial, fungal, plant, viral or animal origin e.g. vegetable or microbial origin. Microbial origin is preferred. Chemically modified or protein engineered mutants are included. It may be an alkaline protease, such as a serine protease or a metalloprotease. A serine protease may for example be of the S1 family, such as trypsin, or the S8 family such as subtilisin. A metalloproteases protease may for example be a thermolysin from e.g. family M4 or other metalloprotease such as those from M5, M7 or M8 families.

The term "subtilases" refers to a sub-group of serine protease according to Siezen et al., Protein Engng. 4 (1991) 719-737 and Siezen et al. Protein Science 6 (1997) 501-523. Serine proteases are a subgroup of proteases characterized by having a serine in the active site, which forms a covalent adduct with the substrate. The subtilases may be divided into 6 sub-divisions, i.e. the Subtilisin family, the Thermitase family, the Proteinase K family, the Lantibiotic peptidase family, the Kexin family and the Pyrolysin family.

Examples of subtilases are those derived from *Bacillus* such as *Bacillus lentus, B. alkalophilus, B. subtilis, B. amyloliquefaciens, Bacillus pumilus* and *Bacillus gibsonii* described in; US7262042 and WO09/021867, and *subtilisin lentus, subtilisin Novo, subtilisin Carlsberg, Bacillus licheniformis, subtilisin BPN', subtilisin 309, subtilisin 147* and *subtilisin 168* described in WO89/06279 and protease PD138 described in (WO93/18140). Other useful proteases may be those described in WO92/175177, WO01/016285, WO02/026024 and WO02/016547. Examples of trypsin-like proteases are trypsin (e.g. of porcine or bovine origin) and the *Fusarium* protease described in WO89/06270, WO94/25583 and WO05/040372, and the chymotrypsin proteases derived from *Cellumonas* described in WO05/052161 and WO05/052146.

A further preferred protease is the alkaline protease from *Bacillus lentus* DSM 5483, as described for example in WO95/23221, and variants thereof which are described in WO92/21760, WO95/23221, EP1921147 and EP1921148.

Examples of metalloproteases are the neutral metalloprotease as described in WO07/044993 (Genencor Int.) such as those derived from *Bacillus amyloliquefaciens.*

Examples of useful proteases are the variants described in: WO92/19729, WO96/034946, WO98/20115, WO98/20116, WO99/011768, WO01/44452, WO03/006602, WO04/03186, WO04/041979, WO07/006305, WO11/036263, WO11/036264, especially the variants with substitutions in one or more of the following positions: 3, 4, 9, 15, 27, 36, 57, 68, 76, 87, 95, 96, 97, 98, 99, 100, 101, 102, 103, 104, 106, 118, 120, 123, 128, 129, 130, 160, 167, 170, 194, 195, 199, 205, 206, 217, 218, 222, 224, 232, 235, 236, 245, 248, 252 and 274 using the BPN' numbering. More preferred the subtilase variants may comprise the mutations: S3T, V4I, S9R, A15T, K27R, *36D, V68A, N76D, N87S,R, *97E, A98S, S99G,D,A, S99AD, S101G,M,R S103A, V104I,Y,N, S106A, G118V,R, H120D,N, N123S, S128L, P129Q, S130A, G160D, Y167A, R170S, A194P, G195E, V199M, V205I, L217D, N218D, M222S, A232V, K235L, Q236H, Q245R, N252K, T274A (using BPN' numbering).

Suitable commercially available protease enzymes include those sold under the trade names Alcalase®, Duralase$^{Tm}$, Durazym$^{Tm}$, Relase®, Relase® Ultra, Savinase®, Savinase® Ultra, Primase®, Polarzyme®, Kannase®, Liquanase®, Liquanase® Ultra, Ovozyme®, Coronase®, Coronase® Ultra, Blaze®, Neutrase®, Everlase® and Esperase® (Novozymes A/S), those sold under the tradename Maxatase®, Maxacal®, Maxapem®, Purafect®, Purafect Prime®,, Purafect MA®, Purafect Ox®, Purafect OxP®, Puramax®, Properase®, , FN2®, FN3® , FN4®, Excellase®, Eraser®, Opticlean® and Optimase® (Danisco/DuPont), Axapem™ (Gist-Brocases N.V.), BLAP (sequence shown in Figure 29 of US5352604) and variants hereof (Henkel AG) and KAP (*Bacillus alkalophilus* subtilisin) from Kao.

**Lipases and cutinases**

Suitable lipases and cutinases include those of bacterial or fungal origin. Chemically modified or protein engineered mutant enzymes are included. Examples include lipase from *Thermomyces,* e.g. from *T. lanuginosus* (previously named *Humicola lanuginosa*) as described in EP258068 and EP305216, cutinase from *Humicola,* e.g. *H. insolens* (WO96/13580), lipase from strains of *Pseudomonas* (some of these now renamed to *Burkholderia*), e.g. *P. alcaligenes* or *P. pseudoalcaligenes* (EP218272), *P. cepacia* (EP331376), *P. sp.* strain SD705 (WO95/06720 & WO96/27002), *P. wisconsinensis* (WO96/12012), GDSL-type *Streptomyces* lipases (WO10/065455), cutinase from *Magnaporthe grisea* (WO10/107560), cutinase from *Pseudomonas mendocina* (US5,389,536), lipase from *Thermobifida fusca* (WO11/084412), *Geobacillus stearothermophilus* lipase (WO11/084417), lipase from *Bacillus subtilis* (WO11/084599), and lipase from *Streptomyces griseus* (WO11/150157) and *S. pristinaespiralis* (WO12/137147).

Other examples are lipase variants such as those described in EP407225, WO92/05249, WO94/01541, WO94/25578, WO95/14783, WO95/30744, WO95/35381, WO95/22615, WO96/00292, WO97/04079, WO97/07202, WO00/34450, WO00/60063, WO01/92502, WO07/87508 and WO09/109500.

Preferred commercial lipase products include Lipolase™, Lipex™; Lipolex™ and Lipoclean™ (Novozymes A/S), Lumafast (originally from Genencor) and Lipomax (originally from Gist-Brocades).

Still other examples are lipases sometimes referred to as acyltransferases or perhydrolases, e.g. acyltransferases with homology to *Candida antarctica* lipase A (WO10/111143), acyltransferase from *Mycobacterium smegmatis* (WO05/56782), perhydrolases from the CE 7 family (WO09/67279), and variants of the *M. smegmatis* perhydrolase in particular the S54V variant used in the commercial product Gentle Power Bleach from Huntsman Textile Effects Pte Ltd (WO10/100028).

**Amylases**

Suitable amylases which can be used together with the enzyme of the invention may be an alpha-amylase or a glucoamylase and may be of bacterial or fungal origin. Chemically modified or protein engineered mutants are included. Amylases include, for example, alpha-amylases obtained from *Bacillus, e.g.,* a special strain of *Bacillus licheniformis,* described in more detail in GB 1,296,839.

Suitable amylases include amylases having SEQ ID NO: 2 in WO 95/10603 or variants having 90% sequence identity to SEQ ID NO: 3 thereof. Preferred variants are described in WO 94/02597, WO 94/18314, WO 97/43424 and SEQ ID NO: 4 of WO 99/019467, such as variants with substitutions in one or more of the following positions: 15, 23, 105, 106, 124, 128, 133, 154, 156, 178, 179, 181, 188, 190, 197, 201, 202, 207, 208, 209, 211, 243, 264, 304, 305, 391, 408, and 444.

Different suitable amylases include amylases having SEQ ID NO: 6 in WO 02/010355 or variants thereof having 90% sequence identity to SEQ ID NO: 6. Preferred variants of SEQ ID NO: 6 are those having a deletion in positions 181 and 182 and a substitution in position 193.

Other amylases which are suitable are hybrid alpha-amylase comprising residues 1-33 of the alpha-amylase derived from *B. amyloliquefaciens* shown in SEQ ID NO: 6 of WO 2006/066594 and residues 36-483 of the *B. licheniformis* alpha-amylase shown in SEQ ID NO: 4 of WO 2006/066594 or variants having 90% sequence identity thereof. Preferred variants of this hybrid alpha-amylase are those having a substitution, a deletion or an insertion in one of more of the following positions: G48, T49, G107, H156, A181, N190, M197, 1201, A209 and Q264. Most preferred variants of the hybrid alpha-amylase comprising residues 1-33 of the alpha-amylase derived from *B. amyloliquefaciens* shown in SEQ ID NO: 6 of WO 2006/066594 and residues 36-483 of SEQ ID NO: 4 are those having the substitutions:

M197T;
H156Y+A181T+N190F+A209V+Q264S; or
G48A+T49I+G107A+H156Y+A181T+N190F+I201F+A209V+Q264S.

Further amylases which are suitable are amylases having SEQ ID NO: 6 in WO 99/019467 or variants thereof having 90% sequence identity to SEQ ID NO: 6. Preferred variants of SEQ ID NO: 6 are those having a substitution, a deletion or an insertion in one or more of the following positions: R181, G182, H183, G184, N195, I206, E212, E216 and K269. Particularly preferred amylases are those having deletion in positions R181 and G182, or positions H183 and G184.

Additional amylases which can be used are those having SEQ ID NO: 1, SEQ ID NO: 3, SEQ ID NO: 2 or SEQ ID NO: 7 of WO 96/023873 or variants thereof having 90% sequence identity to SEQ ID NO: 1, SEQ ID NO: 2, SEQ ID NO: 3 or SEQ ID NO: 7. Preferred variants of SEQ ID NO: 1, SEQ ID NO: 2, SEQ ID NO: 3 or SEQ ID NO: 7 are those having a substitution, a deletion or an insertion in one or more of the following positions: 140, 181, 182, 183, 184, 195, 206, 212, 243, 260, 269, 304 and 476, using SEQ ID 2 of WO 96/023873 for numbering. More preferred variants are

those having a deletion in two positions selected from 181, 182, 183 and 184, such as 181 and 182, 182 and 183, or positions 183 and 184. Most preferred amylase variants of SEQ ID NO: 1, SEQ ID NO: 2 or SEQ ID NO: 7 are those having a deletion in positions 183 and 184 and a substitution in one or more of positions 140, 195, 206, 243, 260, 304 and 476.

Other amylases which can be used are amylases having SEQ ID NO: 2 of WO 08/153815, SEQ ID NO: 10 in WO 01/66712 or variants thereof having 90% sequence identity to SEQ ID NO: 2 of WO 08/153815 or 90% sequence identity to SEQ ID NO: 10 in WO 01/66712. Preferred variants of SEQ ID NO: 10 in WO 01/66712 are those having a substitution, a deletion or an insertion in one of more of the following positions: 176, 177, 178, 179, 190, 201, 207, 211 and 264.

Further suitable amylases are amylases having SEQ ID NO: 2 of WO 09/061380 or variants having 90% sequence identity to SEQ ID NO: 2 thereof. Preferred variants of SEQ ID NO: 2 are those having a truncation of the C-terminus and/or a substitution, a deletion or an insertion in one of more of the following positions: Q87, Q98, S125, N128, T131, T165, K178, R180, S181, T182, G183, M201, F202, N225, S243, N272, N282, Y305, R309, D319, Q320, Q359, K444 and G475. More preferred variants of SEQ ID NO: 2 are those having the substitution in one of more of the following positions: Q87E,R, Q98R, S125A, N128C, T131I, T165I, K178L, T182G, M201L, F202Y, N225E,R, N272E,R, S243Q,A,E,D, Y305R, R309A, Q320R, Q359E, K444E and G475K and/or deletion in position R180 and/or S181 or of T182 and/or G183. Most preferred amylase variants of SEQ ID NO: 2 are those having the substitutions:

N128C+K178L+T182G+Y305R+G475K;
N128C+K178L+T182G+F202Y+Y305R+D319T+G475K;
S125A+N128C+K178L+T182G+Y305R+G475K; or
S125A+N128C+T131I+T165I+K178L+T182G+Y305R+G475K wherein the variants are C-terminally truncated and optionally further comprises a substitution at position 243 and/or a deletion at position 180 and/or position 181.

Further suitable amylases are amylases having SEQ ID NO: 1 of WO13184577 or variants having 90% sequence identity to SEQ ID NO: 1 thereof. Preferred variants of SEQ ID NO: 1 are those having a substitution, a deletion or an insertion in one of more of the following positions: K176, R178, G179, T180, G181, E187, N192, M199, I203, S241, R458, T459, D460, G476 and G477. More preferred variants of SEQ ID NO: 1 are those having the substitution in one of more of the following positions: K176L, E187P, N192FYH, M199L, I203YF, S241QADN, R458N, T459S, D460T, G476K and G477K and/or deletion in position R178 and/or S179 or of T180 and/or G181. Most preferred amylase variants of SEQ ID NO: 1 are those having the substitutions:

E187P+I203Y+G476K
E187P+1203Y+R458N+T459S+D460T+G476K
wherein the variants optionally further comprises a substitution at position 241 and/or a deletion at position 178 and/or position 179.

Further suitable amylases are amylases having SEQ ID NO: 1 of WO10104675 or variants having 90% sequence identity to SEQ ID NO: 1 thereof. Preferred variants of SEQ ID NO: 1 are those having a substitution, a deletion or an insertion in one of more of the following positions: N21, D97, V128 K177, R179, S180, 1181, G182, M200, L204, E242, G477 and G478. More preferred variants of SEQ ID NO: 1 are those having the substitution in one of more of the following positions: N21D, D97N, V128I K177L, M200L, L204YF, E242QA, G477K and G478K and/or deletion in position R179 and/or S180 or of 1181 and/or G182. Most preferred amylase variants of SEQ ID NO: 1 are those having the substitutions:

N21D+D97N+V128I
wherein the variants optionally further comprises a substitution at position 200 and/or a deletion at position 180 and/or position 181.

Other suitable amylases are the alpha-amylase having SEQ ID NO: 12 in WO01/66712 or a variant having at least 90% sequence identity to SEQ ID NO: 12. Preferred amylase variants are those having a substitution, a deletion or an insertion in one of more of the following positions of SEQ ID NO: 12 in WO01/66712: R28, R118, N174; R181, G182, D183, G184, G186, W189, N195, M202, Y298, N299, K302, S303, N306, R310, N314; R320, H324, E345, Y396, R400, W439, R444, N445, K446, Q449, R458, N471, N484. Particular preferred amylases include variants having a deletion of D183 and G184 and having the substitutions R118K, N195F, R320K and R458K, and a variant additionally having substitutions in one or more position selected from the group: M9, G149, G182, G186, M202, T257, Y295, N299, M323, E345 and A339, most preferred a variant that additionally has substitutions in all these positions.

Other examples are amylase variants such as those described in WO2011/098531, WO2013/001078 and WO2013/001087.

Commercially available amylases are Duramyl™, Termamyl™, Fungamyl™, Stainzyme ™, Stainzyme Plus™, Natalase™, Liquozyme X and BAN™ (from Novozymes A/S), and Rapidase™ , Purastar™/Effectenz™, Powerase, Preferenz S1000, Preferenz S100 and Preferenz S110 (from Genencor International Inc./DuPont).

**Peroxidases/Oxidases**

A peroxidase according to the invention is a peroxidase enzyme comprised by the enzyme classification EC 1.11.1.7, as set out by the Nomenclature Committee of the International Union of Biochemistry and Molecular Biology (IUBMB), or any fragment derived therefrom, exhibiting peroxidase activity.

Suitable peroxidases include those of plant, bacterial or fungal origin. Chemically modified or protein engineered mutants are included. Examples of useful peroxidases include peroxidases from *Coprinopsis, e.g.,* from *C. cinerea* (EP 179,486), and variants thereof as those described in WO 93/24618, WO 95/10602, and WO 98/15257.

A peroxidase according to the invention also include a haloperoxidase enzyme, such as chloroperoxidase, bromoperoxidase and compounds exhibiting chloroperoxidase or bromoperoxidase activity. Haloperoxidases are classified according to their specificity for halide ions. Chloroperoxidases (E.C. 1.11.1.10) catalyze formation of hypochlorite from chloride ions.

In an embodiment, the haloperoxidase is a chloroperoxidase. Preferably, the haloperoxidase is a vanadium haloperoxidase, *i.e.,* a vanadate-containing haloperoxidase. In a preferred method of the present invention the vanadate-containing haloperoxidase is combined with a source of chloride ion.

Haloperoxidases have been isolated from many different fungi, in particular from the fungus group dematiaceous hyphomycetes, such as *Caldariomyces, e.g., C. fumago, Alternaria, Curvularia, e.g., C. verruculosa* and *C. inaequalis, Drechslera, Ulocladium* and *Botrytis.*

Haloperoxidases have also been isolated from bacteria such as *Pseudomonas, e.g., P. pyrrocinia* and *Streptomyces, e.g., S. aureofaciens.*

In an preferred embodiment, the haloperoxidase is derivable from *Curvularia* sp., in particular *Curvularia verruculosa* or *Curvularia inaequalis,* such as *C. inaequalis* CBS 102.42 as described in WO 95/27046; or *C. verruculosa* CBS 147.63 or *C. verruculosa* CBS 444.70 as described in WO 97/04102; or from *Drechslera hartlebii* as described in WO 01/79459, *Dendryphiella salina* as described in WO 01/79458, *Phaeotrichoconis crotalarie* as described in WO 01/79461, or *Geniculosporium* sp. as described in WO 01/79460.

An oxidase according to the invention include, in particular, any laccase enzyme comprised by the enzyme classification EC 1.10.3.2, or any fragment derived therefrom exhibiting laccase activity, or a compound exhibiting a similar activity, such as a catechol oxidase (EC 1.10.3.1), an o-aminophenol oxidase (EC 1.10.3.4), or a bilirubin oxidase (EC 1.3.3.5).

Preferred laccase enzymes are enzymes of microbial origin. The enzymes may be derived from plants, bacteria or fungi (including filamentous fungi and yeasts).

Suitable examples from fungi include a laccase derivable from a strain of *Aspergillus, Neurospora, e.g., N. crassa, Podospora, Botrytis, Collybia, Fomes, Lentinus, Pleurotus, Trametes, e.g., T. villosa* and *T. versicolor, Rhizoctonia, e.g., R. solani, Coprinopsis, e.g., C. cinerea, C. comatus, C. friesii,* and *C. plicatilis, Psathyrella, e.g., P. condelleana, Panaeolus, e.g., P. papilionaceus, Myceliophthora, e.g., M. thermophila, Schytalidium, e.g., S. thermophilum, Polyporus, e.g., P. pinsitus, Phlebia, e.g., P. radiata* (WO 92/01046), or *Coriolus, e.g., C. hirsutus* (JP 2238885).

Suitable examples from bacteria include a laccase derivable from a strain of *Bacillus.*

A laccase derived from *Coprinopsis* or *Myceliophthora* is preferred; in particular a laccase derived from *Coprinopsis cinerea,* as disclosed in WO 97/08325; or from *Myceliophthora thermophila,* as disclosed in WO 95/33836.

The detergent enzyme(s) may be included in a detergent composition by adding separate additives containing one or more enzymes, or by adding a combined additive comprising all of these enzymes. A detergent additive, *i.e.,* a separate additive or a combined additive, can be formulated, for example, as a granulate, liquid, slurry, etc. Preferred detergent additive formulations are granulates, in particular non-dusting granulates, liquids, in particular stabilized liquids, or slurries.

Non-dusting granulates may be produced, *e.g.* as disclosed in US 4,106,991 and 4,661,452 and may optionally be coated by methods known in the art. Examples of waxy coating materials are polyethyleneglycol (PEG) with mean molar weights of 1000 to 20000; ethoxylated nonylphenols having from 16 to 50 ethylene oxide units; ethoxylated fatty alcohols in which the alcohol contains from 12 to 20 carbon atoms and in which there are 15 to 80 ethylene oxide units; fatty alcohols; fatty acids; and mono- and di- and triglycerides of fatty acids. Examples of film-forming coating materials suitable for application by fluid bed techniques are given in GB 1483591. Liquid enzyme preparations may, for instance, be stabilized by adding a polyol such as propylene glycol, a sugar or sugar alcohol, lactic acid or boric acid according to established methods. Protected enzymes may be prepared according to the method disclosed in EP 238,216.

## Adjunct materials

Any detergent components known in the art for use in detergents may also be utilized. Other optional detergent components include anti-corrosion agents, anti-shrink agents, anti-soil redeposition agents, anti-wrinkling agents, bactericides, binders, corrosion inhibitors, disintegrants/disintegration agents, dyes, enzyme stabilizers (including boric acid, borates, CMC, and/or polyols such as propylene glycol), fabric conditioners including clays, fillers/processing aids, fluorescent whitening agents/optical brighteners, foam boosters, foam (suds) regulators, perfumes, soil-suspending agents, softeners, suds suppressors, tarnish inhibitors, and wicking agents, either alone or in combination. Any ingredient known in the art for use in detergents may be utilized. The choice of such ingredients is well within the skill of the artisan.

## Dispersants

The detergent compositions can also contain dispersants. In particular powdered detergents may comprise dispersants. Suitable water-soluble organic materials include the homo- or co-polymeric acids or their salts, in which the polycarboxylic acid comprises at least two carboxyl radicals separated from each other by not more than two carbon atoms. Suitable dispersants are for example described in Powdered Detergents, Surfactant science series volume 71, Marcel Dekker, Inc.

## Dye transfer inhibiting agents

The detergent compositions may also include one or more dye transfer inhibiting agents. Suitable polymeric dye transfer inhibiting agents include, but are not limited to, polyvinylpyrrolidone polymers, polyamine *N*-oxide polymers, copolymers of *N*-vinylpyrrolidone and *N*-vinylimidazole, polyvinyloxazolidones and polyvinylimidazoles or mixtures thereof. When present in a subject composition, the dye transfer inhibiting agents may be present at levels from about 0.0001 % to about 10%, from about 0.01% to about 5% or even from about 0.1% to about 3% by weight of the composition.

## Fluorescent whitening agent

The detergent compositions will preferably also contain additional components that may tint articles being cleaned, such as fluorescent whitening agent or optical brighteners. Where present the brightener is preferably at a level of about 0.01% to about 0.5%. Any fluorescent whitening agent suitable for use in a laundry detergent composition may be used in the composition. The most commonly used fluorescent whitening agents are those belonging to the classes of diaminostilbene-sulfonic acid derivatives, diarylpyrazoline derivatives and bisphenyl-distyryl derivatives. Examples of the diaminostilbene-sulfonic acid derivative type of fluorescent whitening agents include the sodium salts of: 4,4'-bis-(2-diethanolamino-4-anilino-s-triazin-6-ylamino) stilbene-2,2'-disulfonate, 4,4'-bis-(2,4-dianilino-s-triazin-6-ylamino) stilbene-2.2'-disulfonate, 4,4'-bis-(2-anilino-4-(*N*-methyl-*N*-2-hydroxy-ethylamino)-s-triazin-6-ylamino) stilbene-2,2'-disulfonate, 4,4'-bis-(4-phenyl-1,2,3-triazol-2-yl)stilbene-2,2'-disulfonate and sodium 5-(2*H*-naphtho[1,2-*d*][1,2,3]triazol-2-yl)-2-[(*E*)-2-phenylvinyl]benzenesulfonate. Preferred fluorescent whitening agents are Tinopal DMS and Tinopal CBS available from Ciba-Geigy AG, Basel, Switzerland. Tinopal DMS is the disodium salt of 4,4'-bis-(2-morpholino-4-anilino-s-triazin-6-ylamino) stilbene-2,2'-disulfonate. Tinopal CBS is the disodium salt of 2,2'-bis-(phenyl-styryl)-disulfonate. Also preferred are fluorescent whitening agents is the commercially available Parawhite KX, supplied by Paramount Minerals and Chemicals, Mumbai, India. Other fluorescers suitable for use in the invention include the 1-3-diaryl pyrazolines and the 7-alkylaminocoumarins.

Suitable fluorescent brightener levels include lower levels of from about 0.01, from 0.05, from about 0.1 or even from about 0.2 wt % to upper levels of 0.5 or even 0.75 wt%.

## Soil release polymers

The detergent compositions may also include one or more soil release polymers which aid the removal of soils from fabrics such as cotton and polyester based fabrics, in particular the removal of hydrophobic soils from polyester based fabrics. The soil release polymers may for example be nonionic or anionic terephthalte based polymers, polyvinyl caprolactam and related copolymers, vinyl graft copolymers, polyester polyamides see for example Chapter 7 in Powdered Detergents, Surfactant science series volume 71, Marcel Dekker, Inc. Another type of soil release polymers are amphiphilic alkoxylated grease cleaning polymers comprising a core structure and a plurality of alkoxylate groups attached to that core structure. The core structure may comprise a polyalkylenimine structure or a polyalkanolamine structure as described in detail in WO 2009/087523. Furthermore random graft co-polymers are suitable soil release polymers. Suitable graft co-polymers are described in more detail in WO 2007/138054, WO 2006/108856 and WO 2006/113314. Other soil release polymers are substituted polysaccharide structures especially substituted cellulosic structures such

as modified cellulose deriviatives such as those described in EP 1867808 or WO 2003/040279. Suitable cellulosic polymers include cellulose, cellulose ethers, cellulose esters, cellulose amides and mixtures thereof. Suitable cellulosic polymers include anionically modified cellulose, nonionically modified cellulose, cationically modified cellulose, zwitterionically modified cellulose, and mixtures thereof. Suitable cellulosic polymers include methyl cellulose, carboxy methyl cellulose, ethyl cellulose, hydroxyl ethyl cellulose, hydroxyl propyl methyl cellulose, ester carboxy methyl cellulose, and mixtures thereof.

**Anti-redeposition agents**

The detergent compositions may also include one or more anti-redeposition agents such as carboxymethylcellulose (CMC), polyvinyl alcohol (PVA), polyvinylpyrrolidone (PVP), polyoxyethylene and/or polyethyleneglycol (PEG), homopolymers of acrylic acid, copolymers of acrylic acid and maleic acid, and ethoxylated polyethyleneimines. The cellulose based polymers described under soil release polymers above may also function as anti-redeposition agents.

**Rheology Modifiers**

The detergent compositions may also include one or more rheology modifiers, structurants or thickeners, as distinct from viscosity reducing agents. The rheology modifiers are selected from the group consisting of non-polymeric crystalline, hydroxy-functional materials, polymeric rheology modifiers which impart shear thinning characteristics to the aqueous liquid matrix of a liquid detergent composition. The rheology and viscosity of the detergent can be modified and adjusted by methods known in the art, for example as shown in EP 2169040.

**Formulation of detergent products**

The detergent composition may be in any convenient form, e.g., a bar, a homogenous tablet, a tablet having two or more layers, a pouch having one or more compartments, a regular or compact powder, a granule, a paste, a gel, or a regular, compact or concentrated liquid.

Pouches can be configured as single or multicompartments. It can be of any form, shape and material which is suitable for hold the composition, e.g. without allowing the release of the composition to release of the composition from the pouch prior to water contact. The pouch is made from water soluble film which encloses an inner volume. Said inner volume can be divided into compartments of the pouch. Preferred films are polymeric materials preferably polymers which are formed into a film or sheet. Preferred polymers, copolymers or derivates thereof are selected polyacrylates, and water soluble acrylate copolymers, methyl cellulose, carboxy methyl cellulose, sodium dextrin, ethyl cellulose, hydroxyethyl cellulose, hydroxypropyl methyl cellulose, malto dextrin, poly methacrylates, most preferably polyvinyl alcohol copolymers and, hydroxypropyl methyl cellulose (HPMC). Preferably the level of polymer in the film for example PVA is at least about 60%. Preferred average molecular weight will typically be about 20,000 to about 150,000. Films can also be of blended compositions comprising hydrolytically degradable and water soluble polymer blends such as polylactide and polyvinyl alcohol (known under the Trade reference M8630 as sold by MonoSol LLC, Indiana, USA) plus plasticisers like glycerol, ethylene glycerol, propylene glycol, sorbitol and mixtures thereof. The pouches can comprise a solid laundry cleaning composition or part components and/or a liquid cleaning composition or part components separated by the water soluble film. The compartment for liquid components can be different in composition than compartments containing solids: US2009/0011970 A1.

Detergent ingredients can be separated physically from each other by compartments in water dissolvable pouches or in different layers of tablets. Thereby negative storage interaction between components can be avoided. Different dissolution profiles of each of the compartments can also give rise to delayed dissolution of selected components in the wash solution.

A liquid or gel detergent, which is not unit dosed, may be aqueous, typically containing at least 20% by weight and up to 95% water, such as up to about 70% water, up to about 65% water, up to about 55% water, up to about 45% water, up to about 35% water. Other types of liquids, including without limitation, alkanols, amines, diols, ethers and polyols may be included in an aqueous liquid or gel. An aqueous liquid or gel detergent may contain from 0-30% organic solvent.

A liquid or gel detergent may be non-aqueous.

**Laundry soap bars**

The enzymes of the invention may be added to laundry soap bars and used for hand washing laundry, fabrics and/or textiles. The term laundry soap bar includes laundry bars, soap bars, combo bars, syndet bars and detergent bars. The types of bar usually differ in the type of surfactant they contain, and the term laundry soap bar includes those

containing soaps from fatty acids and/or synthetic soaps. The laundry soap bar has a physical form which is solid and not a liquid, gel or a powder at room temperature. The term solid is defined as a physical form which does not significantly change over time, i.e. if a solid object (e.g. laundry soap bar) is placed inside a container, the solid object does not change to fill the container it is placed in. The bar is a solid typically in bar form but can be in other solid shapes such as round or oval.

The laundry soap bar may contain one or more additional enzymes, protease inhibitors such as peptide aldehydes (or hydrosulfite adduct or hemiacetal adduct), boric acid, borate, borax and/or phenylboronic acid derivatives such as 4-formylphenylboronic acid, one or more soaps or synthetic surfactants, polyols such as glycerine, pH controlling compounds such as fatty acids, citric acid, acetic acid and/or formic acid, and/or a salt of a monovalent cation and an organic anion wherein the monovalent cation may be for example $Na^+$, $K^+$ or $NH_4^+$ and the organic anion may be for example formate, acetate, citrate or lactate such that the salt of a monovalent cation and an organic anion may be, for example, sodium formate.

The laundry soap bar may also contain complexing agents like EDTA and HEDP, perfumes and/or different type of fillers, surfactants e.g. anionic synthetic surfactants, builders, polymeric soil release agents, detergent chelators, stabilizing agents, fillers, dyes, colorants, dye transfer inhibitors, alkoxylated polycarbonates, suds suppressers, structurants, binders, leaching agents, bleaching activators, clay soil removal agents, anti-redeposition agents, polymeric dispersing agents, brighteners, fabric softeners, perfumes and/or other compounds known in the art.

The laundry soap bar may be processed in conventional laundry soap bar making equipment such as but not limited to: mixers, plodders, e.g a two stage vacuum plodder, extruders, cutters, logo-stampers, cooling tunnels and wrappers. The invention is not limited to preparing the laundry soap bars by any single method. The premix may be added to the soap at different stages of the process. For example, the premix containing a soap, the enzyme of the invention, optionally one or more additional enzymes, a protease inhibitor, and a salt of a monovalent cation and an organic anion may be prepared and and the mixture is then plodded. The enzyme of the invention and optional additional enzymes may be added at the same time as the protease inhibitor for example in liquid form. Besides the mixing step and the plodding step, the process may further comprise the steps of milling, extruding, cutting, stamping, cooling and/or wrapping.

**Formulation of enzyme in co-granule**

The enzyme of the invention may be formulated as a granule for example as a co-granule that combines one or more enzymes. Each enzyme will then be present in more granules securing a more uniform distribution of enzymes in the detergent. This also reduces the physical segregation of different enzymes due to different particle sizes. Methods for producing multi-enzyme co-granulates for the detergent industry is disclosed in the IP.com disclosure IPCOM000200739D.

Another example of formulation of enzymes by the use of co-granulates are disclosed in WO 2013/188331, which relates to a detergent composition comprising (a) a multi-enzyme co-granule; (b) less than 10 wt zeolite (anhydrous basis); and (c) less than 10 wt phosphate salt (anhydrous basis), wherein said enzyme co-granule comprises from 10 to 98 wt% moisture sink component and the composition additionally comprises from 20 to 80 wt% detergent moisture sink component.

WO 2013/188331 also relates to a method of treating and/or cleaning a surface, preferably a fabric surface comprising the steps of (i) contacting said surface with the detergent composition as claimed and described herein in aqueous wash liquor, (ii) rinsing and/or drying the surface.

The multi-enzyme co-granule may comprise an enzyme of the invention and (a) one or more enzymes selected from the group consisting of first- wash lipases, cleaning cellulases, xyloglucanases, perhydrolases, peroxidases, lipoxygenases, laccases and mixtures thereof; and (b) one or more enzymes selected from the group consisting of hemicellulases, proteases, care cellulases, cellobiose dehydrogenases, xylanases, phospholipases, esterases, cutinases, pectinases, mannanases, pectate lyases, keratinases, reductases, oxidases, phenoloxidases, ligninases, pullulanases, tannases, pentosanases, lichenases glucanases, arabinosidases, hyaluronidase, chondroitinase, amylases, and mixtures thereof.

**Use in degrading xanthan gum**

Xanthan gum is use as an ingredient in many consumer products including foods and cosmetics as well as in the oil and drilling industry. Therefore enzymes having xanthan degrading activity can be applied in improved cleaning processes, such as the easier removal of stains containing xanthan gum, as well as the degradation of xanthan gum which is often used in the oil and drilling industry. Thus the present invention is directed to the use of enzymes of the invention or compositions thereof to degrade xanthan gum. The present invention is also directed to the use of compositions comprising an enzyme of the invention and a xanthan lyase to degrade xanthan gum.

Degradation of xanthan gum may be measured using the viscosity reduction assay as described herein on xanthan gum. Xanthan degrading activity may alternatively be measured as reducing ends on xanthan gum using the colorimetric assay developed by Lever (1972), Anal. Biochem. 47: 273-279, 1972.

## Use in detergents

The enzymes of the invention or compositions thereof may be used in cleaning processes such as the laundering of textiles and fabrics (e.g. household laundry washing and industrial laundry washing), as well as household and industrial hard surface cleaning, such as dish wash. The enzymes of the invention may be added to a detergent composition comprising of one or more detergent components.

An embodiment is the use of enzymes of the invention together with xanthan lyases or compositions thereof in cleaning processes such as the laundering of textiles and fabrics (e.g. household laundry washing and industrial laundry washing), as well as household and industrial hard surface cleaning, such as dish wash. The enzymes of the invention together with xanthan lyases may be added to a detergent composition comprising of one or more detergent components.

The invention also relates to methods for degrading xanthan gum on the surface of a textile or hard surface, such as dish wash, comprising applying a composition comprising one or more enzymes of the invention to xanthan gum. The invention further relates to methods for degrading xanthan gum on the surface of a textile or hard surface, such as dish wash, comprising applying a composition comprising one or more xanthan lyase to xanthan gum. An embodiment is a method for degrading xanthan gum on the surface of a textile or hard surface, such as dish wash, comprising applying a composition comprising one or more enzymes of the invention together with one or more xanthan lyase to xanthan gum. An embodiment is the composition comprising one or more detergent components as described above.

## Use in the fracturing of a subterranean formation (oil and/or gas drilling)

Hydraulic fracturing is used to create subterranean fractures that extend from the borehole into rock formation in order to increase the rate at which fluids can be produced by the formation. Generally, a high viscosity fracturing fluid is pumped into the well at sufficient pressure to fracture the subterranean formation. In order to maintain the increased exposure to the formation, a solid proppant is added to the fracturing fluid which is carried into the fracture by the high pressure applied to the fluid. Once the high viscosity fracturing fluid has carried the proppant into the formation, breakers are used to reduce the fluid's viscosity which allows the proppant to settle into the fracture and thereby increase the exposure of the formation to the well. Breakers work by reducing the molecular weight of the polymers, thus 'breaking' or degrading the polymer. The fracture then becomes a high permeability conduit for fluids and gas to be produced back to the well. Such processes are further disclosed in US patent nos. 7,360,593, 5,806,597, 5,562,160, 5,201,370 and 5,067,566.

Thus the invention relates to the use of an enzyme of the invention as enzyme breakers. An embodiment of the invention is the use of an enzyme of the invention together with a xanthan lyase as enzyme breakers.

Accordingly, the invention provides a method for breaking xanthan gum in a well bore comprising: (i) blending together a gellable fracturing fluid comprising aqueous fluid, one or more hydratable polymers, suitable cross-linking agents for cross-linking the hydratable polymer to form a polymer gel and one or more enzymes of the invention (i.e. the enzyme breaker); (ii) pumping the cross-linked polymer gel into the well bore under sufficient pressure to fracture the surrounding formation; and (iii) allowing the enzyme breaker to degrade the cross-linked polymer to reduce the viscosity of the fluid so that the fluid can be pumped from the formation back to the well surface. As such, the enzymes of the invention can be used to control the viscosity of fracturing fluids. Furthermore, one or more enzymes of the invention together with one or more xanthan lyase can be used to control the viscosity of fracturing fluids.

The enzyme breaker of the present invention may be an ingredient of a fracturing fluid or a breaker-crosslinker-polymer complex which further comprises a hydratable polymer and a crosslinking agent. The fracturing fluid or complex may be a gel or may be gellable. The complex is useful in a method for using the complex in a fracturing fluid to fracture a subterranean formation that surrounds a well bore by pumping the fluid to a desired location within the well bore under sufficient pressure to fracture the surrounding subterranean formation. The complex may be maintained in a substantially non-reactive state by maintaining specific conditions of pH and temperature, until a time at which the fluid is in place in the well bore and the desired fracture is completed. Once the fracture is completed, the specific conditions at which the complex is inactive are no longer maintained. When the conditions change sufficiently, the complex becomes active and the breaker begins to catalyze polymer degradation causing the fracturing fluid to become sufficiently fluid to be pumped from the subterranean formation to the well surface.

Method of degrading xanthan gum wherein the xanthan gum is used in fracturing of a subterranean formation perpetrated by a well bore

When a well is drilled, reservoir drilling fluid (RDF) is circulated within the drilling equipment to cool down and clean the drill bit, remove the drill cuttings out of the well bore, reduce friction between the drill string and the sides of the borehole, and form a filtercake in order to prevent fluid leak off into the formation. The driving force for the formation of the filtercake is the higher wellbore pressure applied to maintain the borehole stability. This filtercake restricts the inflow of reservoir fluids into the wellbore during the drilling process and placement of the completion. If the filtercake damage that is created during the drilling process is not removed prior to or during completion of the well, a range of issues can arise when the well is put on production, i.e., completion equipment failures and impaired reservoir productivity.

Drilling fluid (mud), also called reservoir drilling fluid (RDF), can be synthetic/oil based or water based. To minimize invasion of the drilling fluid into the formation, both oil based and water based mud filtercakes typically contain a bridging or weighting agent, usually particles of calcium carbonate, barite or a mixture of the two, that bridge at the pore throats of the formation and thereby form a relatively low permeability filtercake. Both oil based and water based mud filtercakes also contain solids called cuttings that have been picked up during drilling, as opposed to the bridging/weighting agents that are added in the formulation of the drilling fluid. These solids can be quartz (sand), silts and/or shales, depending on the reservoir formation as well as the formations traversed by the drilling path to the reservoir. In addition, oil based drilling muds contain water droplets that become trapped in the pore space of the filtercake, while water based mud filtercakes contain polymers, such as starch and xanthan gum, and other inorganic salts.

The formation of a mud filtercake is often necessary for drilling, particularly in unconsolidated formations with wellbore stability problems and typically high permeabilities. The filtercake is then treated with various chemicals, such as chelates or acids to dissolve the calcite component; and/or enzymes or oxidizers to degrade the polymer component to recover permeability.

In one aspect, the invention provides a method for degrading xanthan gum wherein xanthan gum is used in fracturing of a subterranean formation perpetrated by a well bore by applying a composition comprising one of more enzymes of the invention. The method can include the steps of: (i) pumping a treatment fluid comprising one or more enzymes of the invention into the borehole in contact with the filtercake to be removed to establish a differential pressure between the treatment fluid and the formation adjacent the filtercake and (ii) evenly propagating treatment of the filtercake during the differential pressure period to delay breakthrough by the treatment fluid.

In one embodiment, the method can include establishing permeability through the treated filtercake between the formation and the borehole. In another embodiment, the filtercake can include drilling solids and clays, and may be formed from an aqueous drilling fluid. If desired, the treatment fluid for treating the aqueous drilling fluid filtercake can also include an oxidizer and/or a chelate, or it can be substantially free of chelate and oxidizer additives. In another example, the filtercake can be formed from an oil or invert emulsion drilling fluid. If desired, the treatment fluid for treating the oil or invert emulsion drilling fluid filtercake can also include a mutual solvent, a water-wetting agent or a combination thereof to disperse hydrophobic components in the filtercake.

In one embodiment, the treatment fluid comprises one or more GH5 polypeptides of the invention. In another embodiment, the treatment fluid comprises one or more xanthan lyase. In a preferred embodiment, the treatment fluid comprises one or more GH5 polypeptides and one or more xanthan lyase.

Method of degrading xanthan gum wherein the xanthan gum is a component in a borehole filtercake

In one aspect, the invention provides a method for cleaning borehole filtercake, comprising polymers, such as xanthan gum and drilling fluid solids once the filtercake has been pumped to the surface. Drilling mud is pumped from mud pits to the drill bit and then back out to the surface, carrying out amongst other things crushed or cut rock (cuttings) in the process. The cuttings are filtered out and the mud is returned to the mud pits where fines can settle and/or chemicals or enzymes (breakers) can be added.

The method for degrading xanthan gum wherein the xanthan gum is a component in borehole filtercake can include the steps of (i) treating the borehole filtercake with a treatment fluid comprising one or more enzymes of the invention and (ii) separating the solids from the fluids. In a preferred embodiment, the treatment fluid comprises one or more enzymes of the invention and one or more xanthan lyase.

The borehole filtercake may be treated in mud pits with one or more enzymes of the invention and the drilling fluid can be re-circulated. Alternatively, once the filtercake has been treated with one or more enzymes of the invention, the solids and fluid are separated using solid-liquid separation processes, such as centrifugation.

## Examples

### Activity assays

### Xanthan lyase activity assay

0.8 mL 100 mM HEPES buffer, pH 6.0 was mixed with 0.2 mL Xanthan gum (5 mg/mL) dissolved in water in a 1 mL 1 cm cuvette. The cuvette was inserted into a spectrophotometer (Agilent G1103A 8453A, CA, USA) with temperature control set at 40 °C. The solution was preincubated for 10 min and 0.1 mL sample was added and the solution was mixed by aspiring and dispensing the solution for at least 5 times using a pipette. Total reaction volume was 1.1 mL. Absorbance at 235 nm was collected for 10 min using a 30 sec measuring interval. Initial activity was calculated by using the software (UV-Visible Chemstation Rev A.10.01 [81], Agilent).

### Example 1: Strain and DNA

The DNA in SEQ ID NO: 1 encoding the GH5 polypeptide EXa of SEQ ID NO: 2 was obtained from an *Opitutaceae* species isolated from an environmental soil sample collected in Denmark.

The DNA SEQ ID NO: 3 encoding the GH5 polypeptide EXb of SEQ ID NO: 4 was isolated from an environmental sample collected in Denmark.

The DNA SEQ ID NO: 5 encoding the GH5 polypeptide EXc of SEQ ID NO: 5 was isolated from an environmental sample collected in Denmark.

The DNA SEQ ID NO: 7 encoding the GH5 polypeptide EXd of SEQ ID NO: 8 was obtained from the public database (UNIPROT M2V1S3) but originates from a strain of *Pseudomonas stutzeri* collected from a Galapagos Rift hydrothermal vent, Ecuador.

Codon optimized synthetic DNA encoding the mature peptide sequences of the four polypeptides were prepared (SEQ ID NO: 9; SEQ ID NO: 10, SEQ ID NO: 11; SEQ ID NO: 12).

### Example 2: Cloning and expression of GH5 polypeptides

The GH5 encoding genes were either cloned by conventional techniques from the strains indicated above or from the synthetic DNA and inserted into a suitable plasmid as described below.

### Example 2a: Cloning and expression of GH5 polypeptides in *E.coli*

The mature peptide encoding part of the GH5 endo-glucanase genes, SEQ ID NO: 1, 3, 5 and 7 was inserted with an N-terminal poly histidine tag with an extra proline and arginine (HHHHHHPR) (SEQ ID NO: 19) after the methionine in the E.coli pET-32a(+) vector from Novagen with standard recombinant techniques. The expression plasmid containing the insert was purified from an *E.coli* transformant harboring the plasmid and transformed into E.coli Xjb (DE3) host cells (from Zymo Research). A fresh clone of *E.coli* Xjb (DE3) containing the pET32-GH5 vector, was grown overnight in Terrific Broth containing 100 ug/ml ampicillin. Next day, a fresh 500 ml culture was inoculated with 1 ml overnight culture and cells were cultured (37 °C, 250 rpm) to an optical density (OD600) between 6-8. Protein expression was induced by 1 mM isopropylthio-D-galactosidase (IPTG) and 6 mM arabinose for 4.5 hours at 20 °C. After continued culture, cells were harvested by centrifugation and lysed by Bugbuster® (Novagen). The soluble fraction was used for polyhistidine tag purification of the GH5 polypeptides SEQ ID NO: 13, 14 and 15 as described in example 4.

### Example 2b: Cloning and expression of GH5 polypeptides in *Bacillus subtilis*

The synthetic codon optimized genes SEQ ID NO: 10, 11 and 12 were cloned into the *Bacillus* expression vector described in WO 2012/025577. The genes were expressed by replacing the native secretion signal sequence with the *Bacillus clausii* secretion signal MKKPLGKIVASTALLISVAFSSSIASA (SEQ ID NO: 20) with an extra affinity tag sequence (HHHHHHPR) (SEQ ID NO: 19) at the C-terminal of the signal peptide, to facilitate the purification process. This resulted in a recombinant mature polypeptide with a His tag at the front of the N-terminal of the mature wild type sequence (SEQ ID NO: 16, 17 and 18).

One clone with the correct recombinant gene sequence was selected and the corresponding plasmid was integrated by homologous recombination into the *Bacillus subtilis* host cell genome (pectate lyase locus) and the gene construct was expressed under the control of a triple promoter system as described in WO99/43835. The gene coding for chloramphenicol acetyltransferase was used as a marker (as described in Diderichsen et al., 1993, Plasmid 30:312-315).

Chloramphenicol resistant transformants were analyzed by PCR to verify the correct size of the amplified fragment. A recombinant *B. subtilis* clone containing the integrated expression construct was selected and cultivated on a rotary shaking table in 500 mL baffled Erlenmeyer flasks each containing 100 ml yeast extract-based media. The clone was cultivated for 5 days at 30°C. The enzyme containing supernatants were harvested and the enzyme purified as described in Example 5.

**Example 3: Purification of wild type GH5 polypeptide from the natural *Opitutaceae* strain**

The *Opitutaceae* strain was cultivated on a rotary shaking table in 500 mL baffled Erlenmeyer flasks each containing 100 ml mineral solution with 0.5% xanthan gum. The strain was cultivated for 20 days at 30°C. A total of 2.0 L supernatant was harvested by centrifugation and was filtered using a 0.2 μm bottle top filter (Nalgene Nunc). The broth was concentrated to 300 mL using ultra-filtration (Sartorius) with 30 kDa cut-off. Equal volume of 3.2 M ammonium sulphate in 40 mM Tris-HCl, pH 7.9 was slowly added with continuous stirring. The sample was filtered using Whatman glass filters (1.7 μm - 0.7 μm) to remove larger particles. The sample was applied on a 20 mL Phenyl-sepharose high performance column (GE Healthcare) pre-equilibrated with 1.6 M ammonium sulphate in 20 mM Tris-HCl, pH 7.9 (equilibration buffer). Unbound protein was eluted by two column volumes of equilibration buffer. Elution was done by a 12 column volume linear gradient from 1.6 M to 0.0 M ammonium sulphate in 20 mM Tris-HCl, pH 7.9. A last elution step of 4 column volume with equilibration buffer was used to elute tightly bound protein. The absorbance at 280 nm was recorded during the entire purification. Protein containing fractions identified by the absorbance at 280 nm in the chromatogram were analyzed by SDS-PAGE (NuPAGE, Invitrogen). Fractions judged as pure were pooled. The sample was concentration from 30 to 4 mL using Macrosep ultra filtration device with 3 kDa cut-off (Pall). The protein concentration was determined by measuring the absorbance at 280 nm using the calculated extinction coefficient where 1 mg/mL equaled 1.89 absorbance units.

**Example 4: Purification of recombinant GH5 polypeptide produced in *E.coli***

200 mL lysed cells (grown as example 2a) were filtered through Fast PES 0.2 μm bottle-top filters to remove debris and unbroken cells. 200 mL of equilibration buffer (20 mM Tris-HCl, pH 7.5 + 500 mM NaCl) was added to the crude protein solution. A 20 mL HisPrep column loaded with $Ni^{2+}$ was equilibrated with equilibration buffer until a stable UV baseline was obtained. The absorbance at 280 nm was continuously monitored throughout the purification. Crude protein was loaded on the column using a flow rate of 4 mL/min. Unbound protein was removed by washing the column with equilibration buffer until a stable UV baseline was obtained. Elution was carried out by a two-step linear gradient using 20 mM Tris-HCl, pH 7.5 + 500 mM NaCl + 500 mM Imidazole (elution buffer). First elution gradient was 10 column volumes 0 to 40 % elution buffer followed by 4 column volumes from 40% to 100 %. Peaks absorbing at 280 nm were analyzed by SDS-PAGE (NuPAGE, Invitrogen). Fractions containing protein with the correct apparent molecular weight were pooled. The pool was desalted and buffer exchanged using a Sephadex G-25 super fine desalting column equilibrated with 20 mM Tris-HCl, pH 8.0. The pool was applied on a 20 mL Source15Q column pre-equillibrated with 20 mM Tris-HCl, pH 8.0. Unbound protein was washed out using 20 mM Tris-HCl, pH 8.0 until a stable UV baseline was obtained. Elution was done by a 10 column volume linear NaCl gradient from 0 to 500 mM NaCl in 20 mM Tris-HCl, pH 8.0. Protein containing fractions were analyzed by SDS-PAGE and fractions judged as pure were pooled. Protein concentration was measured using absorbance at 280 nm using a calculated extinction coefficient where 1 mg/mL corresponded to 1.86 absorbance units.

**Example 5: Purification of recombinant GH5 polypeptide produced in *B. subtilis***

All His-tagged enzymes were purified by immobilized metal chromatography (IMAC) using $Ni^{2+}$ as the metal ion on 5 mL HisTrap Excel columns (GE Healthcare Life Sciences). The purification was done at pH 8 and the bound proteins were eluted with imidazole. The purity of the purified enzymes was checked by SDS-PAGE and the concentration of each enzyme determined by Abs 280 nm after a buffer exchange.

**Example 6: Xanthan degrading activity of GH5 polypeptide and xanthan lyase on xanthan gum by measurement of viscosity reduction**

The viscosity reduction measurements were performed using the viscosity pressure assay described in WO2011/107472 and following the method described in WO2013167581. Results presented are the average of three measurements and are shown in table 1 and 2 below.

A sample size of was 400 μL was used. The hydrolysis conditions were as follows: 30 °C, either 0.25% or 0.5% xanthan gum (XG) in 50 mM MES buffer + 0.01% triton x-100 pH 7.0 or 100mM CHES buffer + 0.01% triton x-100 pH10.

Enzyme was added upon thermal equilibration. Prior to use all enzymes were buffer changed to the MES buffer using NAP 5 columns (GE Healthcare).

The purified enzyme preparations of Example 5 were used for the analysis at a concentration of 31.25 mg/L.

Table 1: Viscosity measurements (Pa) of EXa (SEQ ID NO:13) and/or Xanthan Lyase (SEQ ID NO: 21) on 0.5% xanthan gum at pH 7.

| | T= 0 minutes | T= 30 minutes | T= 1 hour | T= 2 hours | T= 3 hours | T= 4 hours |
|---|---|---|---|---|---|---|
| Water (control) | 430±44 | 504±50 | 470±75 | 483±86 | 466±60 | 504±82 |
| Xanthan gum (control) | 1703±132 | 1738±26 | 1837±122 | 1803±64 | 1739±84 | 1757±21 |
| Xanthan gum + EXa SEQ ID NO:13 | 1586±101 | 1154±38 | 1270±67 | 1230±36 | 1156±49 | 1184±44 |
| Xanthan gum + XLa SEQ ID NO:21 | 1963±93 | 1884±67 | 1890±84 | 1840±131 | 1886±50 | 1950±25 |
| Xanthan gum + EXa SEQ ID NO:13 + XLa SEQ ID NO:21 | 1370±197 | 861±23 | 973±59 | 840±62 | 916±47 | 904±79 |

The results presented above show that the GH5 polypeptide alone and in combination with xanthan lyase can degrade the xanthan gum present in the media at pH 7, thus leading to viscosity reduction. A synergistic effect is obtained with combination of GH5 and xanthan lyase.

Table 2: Viscosity measurements (Pa) of EXa (SEQ ID NO:13) and/or Xanthan Lyase (SEQ ID NO: 23) on 0.5% xanthan gum at pH10

| | T=0 | T=0.5 hours | T=1 hours | T=2 hours | T=3,5 hours |
|---|---|---|---|---|---|
| Water | 370±10 | 454±15 | 519±60 | 411±29 | 554±180 |
| Xanthan gum (XG) control | 1740±151 | 1734±21 | 1819±67 | 1795±29 | 1898±75 |
| XG + EXa SEQ ID NO:13 | 1676±50 | 1324±58 | 1223±12 | 1251±31 | 1318±62 |
| XG + XLc SEQ ID NO:23 | 2046±112 | 1811±82 | 1773±64 | 1781±92 | 1704±67 |
| XG + EXa SEQ ID NO:13 + XLc SEQ ID NO:23 | 1573±227 | 1057±21 | 1153±12 | 1161±40 | 1188±89 |

The results presented above show that the GH5 polypeptide in alone or combination with xanthan lyase can degrade the xanthan gum present in the media at pH 10, thus leading to viscosity reduction.

Table 3: Viscosity measurements (Pa) of EXa (SEQ ID NO:13), EXd (SEQ ID NO:18) and/or Xanthan Lyase (XLa, SEQ ID NO: 21) on 0.5% xanthan gum at pH 7.

| | T=0 | T= 0.5 hours | T= 1 hours | T= 2 hours | T= 3 hours |
|---|---|---|---|---|---|
| Water control | 440 | 410 | 333 | 413 | 469 |
| Xanthan gum (XG) control | 1626 | 1590 | 1546 | 1566 | 1659 |
| XG + EXa SEQ ID NO:13 | 1220 | 1080 | 1046 | 1040 | 1079 |
| XG + EXa SEQ ID NO:13 + XLa SEQ ID NO:21 | 1263 | 850 | 786 | 793 | 815 |
| XG + EXd SEQ ID NO:18 | 1476 | 1406 | 1313 | 1283 | 1245 |

(continued)

| Table 3: Viscosity measurements (Pa) of EXa (SEQ ID NO:13), EXd (SEQ ID NO:18) and/or Xanthan Lyase (XLa, SEQ ID NO: 21) on 0.5% xanthan gum at pH 7. | | | | | |
|---|---|---|---|---|---|
| | T=0 | T= 0.5 hours | T= 1 hours | T= 2 hours | T= 3 hours |
| XG + EXd SEQ ID NO:18 + XLa SEQ ID NO:21 | 1490 | 1056 | 1023 | 933 | 912 |

The results presented above show that the GH5 polypeptide alone and in combination with xanthan lyase can degrade the xanthan gum present in the media at pH 7, thus leading to viscosity reduction.

| Table 4: Viscosity measurements (Pa) of EXa, EXb, EXc recombinantly expressed in E.coli (SEQ ID NO:13; SEQ ID NO:14, SEQ ID NO:15) and/orXanthan Lyase (XLb, SEQ ID NO: 22) on 0.5% xanthan gum at pH7. T=00 is before addition of enzyme and T=0 is right after. | | | | | | | |
|---|---|---|---|---|---|---|---|
| | T=00 | T=0 | T=30 min | T=1hr | T=2hrs | T=3hrs | T=4hrs |
| Water | 541±21 | 544±119 | 519±142 | 545±70 | 399±80 | 422±114 | 326±25 |
| Xanthan gum control | 1878±20 | 1444±15 | 1599±91 | 1571±64 | 1605±38 | 1586±40 | 1566±32 |
| XG + XLb SEQ ID NO:22 | 1898±26 | 1511±12 | 1522±56 | 1505±20 | 1579±80 | 1516±21 | 1559±38 |
| XG + EXb SEQ ID NO:14 | 1884±31 | 1281±55 | 1202±120 | 1145±52 | 1132±70 | 1096±60 | 1116±114 |
| XG + EXc SEQ ID NO:15 | 1931±45 | 1444±80 | 1122±36 | 1108±42 | 1105±45 | 1019±10 | 1059±15 |
| XG + EXa SEQ ID NO:13 | 1891±12 | 1441±38 | 1102±17 | 1051±25 | 1005±6 | 969±26 | 1036±25 |
| XG + EXb SEQ ID NO:14 +XLb SEQ ID NO:22 | 1918±61 | 1121±6 | 862±17 | 731±31 | 689±25 | 652±40 | 576±40 |
| XG + EXc SEQ ID NO:15 +XLb SEQ ID NO:22 | 1911± | 1111± | 935± | 848± | 832± | 822± | 789± |
| XG + EXa SEQ ID NO:13 +XLb SEQ ID NO:22 | 1934±31 | 1198±36 | 855±40 | 831±40 | 785±23 | 909±26 | 819±64 |

The results presented above show that the GH5 polypeptides EXa, EXb and EXc alone and in combination with xanthan lyase can degrade the xanthan gum present in the media at pH 7, thus leading to viscosity reduction. A synergistic effect is obtained with combination of GH5 polypeptide and xanthan lyase

| Table 5: Viscosity measurements (Pa) of EXa, recombinantly expressed in E. coli (SEQ ID NO:13) and EXb and EXc recombinantly expressed in B. subtilis (SEQ ID NO:16 and SEQ ID NO:17 ) and/or Xanthan Lyase (XLb, SEQ ID NO: 22) on 0.5% xanthan gum at pH 7. T=00 is before addition of enzyme and T=0 is right after. | | | | | | | |
|---|---|---|---|---|---|---|---|
| | T=00 | T=0 | T=30 min | T=1 hour | T=2 hours | T=3 hours | T=4 hours |
| Water | 441±25 | 421±40 | 646±44 | 535±59 | 599±74 | 492±15 | 494±32 |
| Xanthan gum(XG) | 2027±2 3 | 1707±3 5 | 1949±5 9 | 1785±11 6 | 1746±7 5 | 1726±1 0 | 1867±6 |
| XG + EXa SEQ ID NO:13 | 2054±4 4 | 1514±1 7 | 1299±2 1 | 1112±57 | 1089±4 5 | 1046±0 | 1027±6 |

(continued)

| Table 5: Viscosity measurements (Pa) of EXa, recombinantly expressed in E. coli (SEQ ID NO:13) and EXb and EXc recombinantly expressed in B. subtilis (SEQ ID NO:16 and SEQ ID NO:17 ) and/or Xanthan Lyase (XLb, SEQ ID NO: 22) on 0.5% xanthan gum at pH 7. T=00 is before addition of enzyme and T=0 is right after. | | | | | | | |
|---|---|---|---|---|---|---|---|
| | T=00 | T=0 | T=30 min | T=1 hour | T=2 hours | T=3 hours | T=4 hours |
| XG + EXb SEQ ID NO:16 | 2067±1 5 | 1527±8 1 | 1393±1 2 | 1229±53 | 1159±1 2 | 1136±0 | 1134±6 |
| XG + EXc SEQ ID NO:17 | 2061±3 1 | 1501±5 5 | 1416±4 4 | 1175±6 | 1183±7 8 | 1169±4 0 | 1147±1 5 |
| XG + EXa SEQ ID NO:13 + XLb SEQ ID NO:22 | 2061±6 | 1274±1 7 | 1063±4 7 | 812±59 | 769±46 | 729±15 | 671±26 |
| XG + EXb SEQ ID NO:20 + XLb SEQ ID NO:22 | 2074±2 6 | 1411±6 5 | 1079±1 5 | 945±92 | 809±12 | 796±10 | 781±10 |
| XG + EXc SEQ ID NO:17 + XLb SEQ ID NO:22 | 2094±3 0 | 1491±2 5 | 1166±0 | 959±46 | 889±40 | 846±0 | 847±57 |
| XG + XLb SEQ ID NO:22 | 2097±4 9 | 1794±6 2 | 1863±2 3 | 1685±15 | 1653±1 0 | 1679±6 | 1667±2 9 |
| XG + EXa SEQ ID NO:13 + XLa SEQ ID NO:21 | 2131±1 5 | 1227±8 1 | 1143±8 1 | 789±62 | 739±25 | 716±44 | 677±55 |
| XG + EXb SEQ ID NO:16 +XLa SEQ ID NO:21 | 2104±7 9 | 1324±1 7 | 1096±4 4 | 795±31 | 803±26 | 792±21 | 767±12 |
| XG + EXc SEQ ID NO:17 +XLa SEQ ID NO:21 | 2107±1 2 | 1241±5 0 | 1163±3 2 | 802±15 | 826±15 | 846±0 | 894±15 |
| XG + XLa SEQ ID NO:21 | 2134±2 0 | 1741±5 7 | 1933±2 9 | 1639±30 | 1659±2 3 | 1666±1 7 | 1637±1 2 |

The results presented above show that the GH5 polypeptides EXa, EXb and EXc alone and in combination with xanthan lyase can degrade the xanthan gum present in the media at pH 7, thus leading to viscosity reduction. A synergistic effect is obtained with combination of GH5 polypeptide and xanthan lyase.

| Table 6: Viscosity measurements (Pa) of EXa, EXb, EXc recombinantly expressed in E.coli (SEQ ID NO:13; SEQ ID NO: 14 or SEQ ID NO: 15) and/or Xanthan Lyase (XLc, SEQ ID NO: 23 or SEQ ID NO:24) on 0.5% xanthan gum at pH 10. T=00 is before addition of enzyme and T=0 is right after. | | | | | | |
|---|---|---|---|---|---|---|
| | *T=00* | *T=0* | *T=30'* | *T=1hr* | *T=2hrs* | *T=3hrs* |
| Water | 429±66 | 502±110 | 504±50 | 434±29 | 478±42 | 479±26 |
| Xanthan gum (XG) | 1932±31 | 1485±81 | 1678±12 | 1641±70 | 1642±38 | 1592±92 |
| XG + EXa SEQ ID NO:13 | 1992±13 8 | 1332±6 | 1254±21 | 1147±51 | 1192±35 | 1215±31 |
| XG + EXb SEQ ID NO:14 | 1989±85 | 1415±50 | 1351±66 | 1321±17 | 1358±51 | 1252±21 |
| XG + EXc SEQ ID NO:17 | 1892±45 | 1442±10 0 | 1408±21 | 1341±50 | 1332±31 | 1262±51 |

(continued)

| Table 6: Viscosity measurements (Pa) of EXa, EXb, EXc recombinantly expressed in E.coli (SEQ ID NO:13; SEQ ID NO: 14 or SEQ ID NO: 15) and/or Xanthan Lyase (XLc, SEQ ID NO: 23 or SEQ ID NO:24) on 0.5% xanthan gum at pH 10. T=00 is before addition of enzyme and T=0 is right after. | | | | | | |
|---|---|---|---|---|---|---|
| | *T=00* | *T=0* | *T=30'* | *T=1hr* | *T=2hrs* | *T=3hrs* |
| XG + EXa SEQ ID NO:13 +XLc SEQ ID NO:23 | 1899±69 | 1429±62 | 1084±76 | 1131±17 | 1092±25 | 1112±40 |
| XG + EXb SEQ ID NO: 14 +XLc SEQ ID NO: 23 | 2019±62 | 1465±13 2 | 1144±23 | 1121±53 | 1108±81 | 1012±59 |
| XG + EXc SEQ ID NO: 15 +XLc SEQ ID NO:23 | 2085±80 | 1602±38 | 1344±15 | 1321±10 | 1262±55 | 1319±10 |
| XG + XLc SEQ ID NO:23 | 2005±47 | 1702±75 | 1588±6 | 1524±67 | 1588±60 | 1569±36 |
| XG + EXa SEQ ID NO:13 +XLd SEQ ID NO:24 | 1959±72 | 1462±11 0 | 1158±38 | 1144±40 | 1148±72 | 1005±45 |
| XG + EXb SEQ ID NO:14 +XLd SEQ ID NO:24 | 1975±25 | 1442±35 | 1211±26 | 1177±15 | 1192±72 | 1182±67 |
| XG + EXc SEQ ID NO: 15 +XLd SEQ ID NO:24 | 1925±13 3 | 1422±95 | 1238±12 | 1274±58 | 1208±81 | 1215±67 |
| XG + XLd SEQ ID NO:24 | 1839±40 | 1525±61 | 1488±21 | 1447±42 | 1432±15 | 1425±76 |

The results presented above show that the GH5 polypeptides GH5, EXb and EXc in combination with xanthan lyase can degrade the xanthan gum present in the media at pH 10, thus leading to viscosity reduction.

| Table 7: Viscosity measurements (Pa) of GH5 polypeptide purified from supernatant of the *Opitutaceae* sp strain and/or Xanthan Lyase (XLa, SEQ ID NO: 21) on 0.25% xanthan gum at pH7 | | | | | |
|---|---|---|---|---|---|
| | T=0 | T=0.5 hour | T= 1 hour | T= 2 hours | T= 3 hours |
| Water | 471±99 | 390±46 | 423±61 | 433±64 | 438±36 |
| Xanthan gum (XG) | 898±12 | 880±40 | 900±17 | 820±40 | 908±50 |
| XG + EXa SEQ ID NO:1 | 856±34 | 743±46 | 723±34 | 672±38 | 644±55 |
| XG + XLa SEQ ID NO: 21 | 908±29 | 865±22 | 860±35 | 857±32 | 856±61 |
| XG + EXa SEQ ID NO: 1 + XLa SEQ ID NO:21 | 800±28 | 597±30 | 612±31 | 577±45 | 648±89 |

**Example 8: Xanthan degrading activity of GH5 polypeptide and xanthan lyase on xanthan gum by measurement of viscosity reduction**

The viscosity measurements were performed using the viscosity pressure assay described in WO2011/107472. 150 μL of each 1 mL hydrolysis or control was the sample size. Results presented are the average of four measurements and are shown in table 8 and 9 below.

Modified xanthan gum was prepared by an adaption of Nankai et al. 1999. "Microbial system for polysaccharide

depolymerization: enzymatic route for xanthan depolymerization by Bacillus sp strain GL1." Applied and Environmental Microbiology 65(6): 2520-2526.

2.5 g of xanthan gum (CP Kelco) was wetted with 5 mL of 96 % ethanol in a 2 L beaker. 500 mL of 100 mM ACES buffer pH 7.00 was added and the solution stirred at ambient temperature for 2h. 250 μL of xanthan lyase (*Bacillus* sp., Megazyme) was added and the solution incubated for 20 h at 50 °C. The sample was then cooled by placing the beaker on ice. After hydrolysis was 1400 mL of ice cold 96 % ethanol was added to the 500 mL sample, under stirring. Precipitation occurs, and after approximately 5 min the ethanol was decanted removing the pyruvated mannose residues. The sample was vacuum filtered and transferred to a glass plate. The glasses were dried at 50 °C for 20 h. The sample was collected, weighed, and grinded.

The hydrolysis conditions were as follows: 40 °C, 0.35 % xanthan gum (XG) in 50 mM HEPES buffer + 0.01 % triton X-100 pH 7.0. The modified xanthan gum powder (mXG) was prepared as described above and a 0.7 % solution was prepared using the same procedure as outlined for XG. Enzyme was added upon thermal equilibration. The initial viscosity is measured prior to enzyme addition, after thermal equilibration. Controls are the same with buffer added instead of enzyme. Buffer was monitored to determine the ultimate end point of a total hydrolysis.

Table 8. Viscosity measurements (Pa). EXc SEQ ID and XLb (SEQ ID NO:22). Each enzyme dosed in 1.5 ppm. pH 7.0

| **Time (Minutes)** | **0** | **15** | **30** | **45** | **60** | **75** | **90** |
|---|---|---|---|---|---|---|---|
| Buffer 50 mM HEPES Control | 645 | 610 | 521 | 502 | 620 | 632 | 600 |
| | | | | | | | |
| Xanthan Gum + Buffer Control | 2140 | 2075 | 1948 | 2092 | 2033 | 2077 | 2005 |
| Xanthan Gum + EXc | 2120 | 1295 | 991 | 957 | 935 | 1112 | 917 |
| Xanthan Gum + EXc + Xanthan Lyase | 1977 | 808 | 811 | 837 | 773 | 807 | 777 |
| Xanthan Gum + Xanthan lyase | 1972 | 1853 | 1838 | 1802 | 1750 | 1737 | 1677 |
| | | | | | | | |
| Modified Xanthan Gum + Buffer Control | 2262 | 2100 | 2143 | 2134 | 2118 | 2150 | 2097 |
| Modified Xanthan Gum + EXc | 2217 | 1225 | 1173 | 1157 | 1130 | 1155 | 1130 |

**Example 9: Wash performance of GH5 polypeptide and xanthan lyase**

The wash performance of the GH5 enzyme was assessed in laundry wash experiments using a Mini wash assay, which is a test method where soiled textile is continuously lifted up and down into the test solution and subsequently rinsed. The wash experiment was conducted under the experimental conditions specified in Table 10.

The textiles were subsequently air-dried and the wash performance was measured as the brightness of the color of the textiles. Brightness can be expressed as the Remission (R), which is a measure for the light reflected or emitted from the test material when illuminated with white light. The Remission (R) of the textiles was measured at 460 nm using a Zeiss MCS 521 VIS spectrophotometer. The measurements were done according to the manufacturer's protocol. The performance of the new enzyme (combination) was compared to the performance of detergent alone (blank). An enzyme (combination) is considered to exhibit improved wash performance, if it performs better than the detergent alone (i.e. $R_{ENZYME} > R_{BLANK}$) (see Table 13 and 14).

| Table 10: Experimental setup of Mini wash assay | |
|---|---|
| Detergent | Liquid Model detergent A or Model detergent T (see Table 11 and 12) |
| Detergent dose | 3.33 g/l |
| pH | "as is" in the current detergent solution and was not adjusted |

(continued)

| Table 10: Experimental setup of Mini wash assay | |
|---|---|
| Water hardness | 16°dH, adjusted by adding $CaCl_2*2H_2O$, $MgCl_2*6H_2O$ and $NaHCO_3$ (5:1:3) to milli-Q water. |
| Enzymes | EXc (SEQ ID NO:17), xanthan lyase (XLb, SEQ ID NO:22 or XLc SEQ ID NO:23) |
| Enzyme dosage | Dosage of GH5: 0.05 mg EP/L (enzyme protein), 0.10 mg EP/L, 0.2 mg EP/L, 0.5 mg EP, 1.0 mg EP/L; experiments with combinations of GH5 and XL were conducted with a fixed concentration of 1.0 mg EP/L XL |
| Volume of test solution | 50 ml |
| Test material | Xanthan Gum with carbon black DN-31D textile swatches (23x3 cm).<br>The test material was obtained from Center for Testmaterials BV, P.O. Box 120, 3133 KT Vlaardingen, the Netherlands, and WFK Testgewebe GmbH, Christenfeld 10, D-41379 Brüggen, Germany |
| Temperature | 40°C |
| Wash time | 30 min |
| Rinse time | 5 min |
| Test system | Soiled textile continuously lifted up and down into the test solutions, 50 times per minute (up-time 0.4 sec, down-time 0.4 sec, lift time 0.4 sec). The test solutions are kept in 125 ml glass beakers.<br>After wash of the textiles are continuously lifted up and down into tap water, 50 times per minute (up-time 0.4 sec, down-time 0.4 sec, lift time 0.4 sec). |

| Table 11: Composition of Model Detergent A (Liquid) [1)] | |
|---|---|
| **Detergent ingredients** | **Wt %** |
| Linear alkylbenzenesulfonic acid (LAS) (Marlon AS3) | 13 |
| Sodium alkyl(C12)ether sulfate (AEOS) (STEOL CS-370 E) | 10 |
| Coco soap (Radiacid 631) | 2.75 |
| Soy soap (Edenor SJ) | 2.75 |
| Alcohol ethoxylate (AEO) (Bio-Soft N25-7) | 11 |
| Sodium hydroxide | 2 |
| Ethanol | 3 |
| Propane-1,2-diol (MPG) | 6 |
| Glycerol | 2 |
| Triethanolamine (TEA) | 3 |
| Sodium formate | 1 |
| Sodium citrate | 2 |
| Diethylenetriaminepentakis(methylenephosphonic acid) (DTMPA) | 0.2 |
| Polycarboxylate polymer (PCA) (Sokalan CP-5) | 0.2 |
| Water | Up to 100 |
| [1)]The pH of the detergent was adjusted to pH 8 with sodium hydroxide or citric acid. | |

| Table 12. Composition of Model detergent T (powder) | |
|---|---|
| **Detergent ingredients** | **Wt %** |
| LAS, sodium salt | 11.72 |
| AS, sodium salt | 2.0 |
| Soap, sodium salt | 2.15 |
| AEO | 3.0 |
| Soda ash | 14.98 |
| Hydrous sodium silicate | 3.12 |
| Zeolite A | 18.75 |
| HEDP-Na4 | 0.15 |
| Sodium citrate | 2.0 |
| PCA, copoly(acrylic acid/maleic acid), sodium salt | 1.65 |
| SRP | 0.5 |
| Sodium sulfate | 13.53 |
| Sodium percarbonate | 22.20 |
| TAED | 3.25 |
| Foam regulator | 1.0 |

Table 13: Remission (R) values obtained in Mini Wash using EXc with and without xanthan lyase (XLb) in liquid model A detergent

| Enzyme dosage | No enzyme | EXc | EXc + xanthan lyase |
|---|---|---|---|
| 0.05 mg EP/L | 29.5 | 32.8 | 35.1 |
| 0.1 mg EP/L | 29.5 | 33.6 | 35.4 |
| 0.2 mg EP/L | 29.5 | 34.3 | 35.9 |
| 0.5 mg EP/L | 29.5 | 35.1 | 36.7 |
| 1.0 mg EP/L | 29.5 | 35.4 | 37.3 |

**Table 14 Remission (R) values obtained in** Mini Wash using EXc with and without Xanthan Lyase (XLc)in powder model T detergent

| Enzyme dosage | No enzyme | EXc | EXc + xanthan lyase |
|---|---|---|---|
| 0.05 mg EP/L | 29.8 | 29.7 | 29.7 |
| 0.1 mg EP/L | 29.8 | 29.8 | 29.8 |
| 0.2 mg EP/L | 29.8 | 30.0 | 30.0 |
| 0.5 mg EP/L | 29.8 | 30.6 | 30.9 |
| 1.0 mg EP/L | 29.8 | 31.0 | 31.2 |

**Example 10: Wash performance of combinations of a GH5 polypeptide and xanthan lyase was tested on specific stains**

The wash performance of variants in liquid and powder detergents was determined by using the following standardized stains, all obtainable from CFT (Center for Testmaterials) B.V., Vlaardingen, Netherlands:

A: Fluid make-up: product no. PCS17

B: Fluid make-up: product no. CS17

For the tests in liquid detergents, a liquid washing agent with the following composition was used as base formulation (all values in weight percent): 0 to 0.5% xanthan gum, 0.2 to 0.4% antifoaming agent, 6 to 7% glycerol, 0.3 to 0.5% ethanol, 0 to 7% FAEOS (fatty alcohol ether sulfate), 10 to 28% nonionic surfactants, 0.5-1% boric acid, 1 to 2% sodium citrate (dihydrate), 2 to 4% soda, 0 to 16% coconut fatty acid, 0.5% HEDP (1-hydroxyethane-(1,1-diphosphonic acid)), 0 to 0.4% PVP (polyvinylpyrrolidone), 0 to 0.05% optical brighteners, 0 to 0.001% dye, remainder deionized water.

Based on this base formulation, detergent was prepared by adding the respective enzyme combination as indicated in table 15. As a reference, the detergent composition without addition of the enzyme combinations was used.

The dosing ratio of the liquid washing agent was 4.7 grams per liter of washing liquor and the washing procedure was performed for 60 minutes at a temperature of 40°C, the water having a water hardness between 15.5 and 16.5° (German degrees of hardness).

For the tests in solid detergents, a European premium detergent was used as base formulation.

The whiteness, i.e. the brightening of the stains, was determined photometrically as an indication of wash performance. A Minolta CM508d spectrometer device was used, which was calibrated beforehand using a white standard provided with the unit.

The results obtained are the difference values between the remission units obtained with the detergents and the remission units obtained with the detergent containing the enzyme combinations. A positive value therefore indicates an improved wash performance due to the enzyme combinations present in the detergent. It is evident from table 15 that enzyme combinations according to the invention show improved wash performance.

Table 15: Wash performance in liquid detergent

| Enzyme combination | | A | B |
|---|---|---|---|
| XLb SEQ ID NO:22+ EXc SEQ ID NO:17 | Diff | 3.3 | 6.4 |
| | HSD | 2.4 | 1.2 |

Table 16: Wash performance in solid detergent

| Enzyme combination | | B |
|---|---|---|
| XLb SEQ ID NO:22 + EXc SEQ ID | Diff | 1.9 |
| | HSD | 1.2 |

**Example 11: Wash performance of GH5 polypeptides with and without Xanthan Lyase**

In this example wash performance of GH5 polypeptides was evaluated in a liquid model detergent A washed in the Automatic Mechanical Stress Assay (AMSA) at 20°C or 40°C. The wash performance of the enzymes was evaluated either alone or in combination with a Xanthan Lyase. The wash conditions used are specified in Table 17 below.

Table 17. Wash conditions used in the example 11:

| | |
|---|---|
| Detergent | Liquid model detergent A |
| Detergent conc. | 3.3 g/L |
| pH | "as is" in the current detergent solution and was not adjusted |
| Temperature | 20°C or 40°C |
| Dosages in AMSA-plate | 140μL detergent per slot; 20μL enzyme per slot |
| Water hardness | 16°dH, adjusted by adding $CaCl_2*2H_2O$, $MgCl_2*6H_2O$ and $NaHCO_3$ (5:1:3) to milli-Q water |
| Enzymes | EXb (SEQ ID NO:16); EXc (SEQ ID NO:17), xanthan lyase (XLb, SEQ ID NO:22) |
| Enzyme dosage | EXb and EXc concentrations: 0.7, 1.5, 20, 125 ppb XLb concentration: 400 ppb |
| Test solution volume | 160 micro L |

(continued)

| | |
|---|---|
| Detergent | Liquid model detergent A |
| Wash time | 20 minutes |
| Stain/ swatch | Mayonaise with carbon black C-S-05 S from CFT, Center for |
| | Testmaterials BV. |

The enzyme and wash liquid were dosed into the AMSA plate and washed according to conditions listed in Table 17. After wash the fabric was flushed in tap water and air-dried. The performance of the enzyme was subsequently measured as the brightness of the colour of the textile samples. Brightness was measured as the intensity of the light reflected from the textile sample when illuminated with white light. Intensity was measured with a professional flatbed scanner EPSON EXPRESSION 10000XL with special designed software that extracted the intensity value from the scanned imagine through standard vector calculations.

The performance of the enzyme (or combination of enzymes) was compared to the performance of detergent alone (blank) or detergent with the Xanthan lyase (XL). An enzyme (or combination of enzymes) was considered to exhibit improved wash performance if it performed better than the detergent alone (i.e., $R_{ENZYME} > R_{BLANK}$) (see Tables 18, 19, 20 and 21).

Table 18. Intensity and delta intensity of GH5 polypeptides EXb (SEQ ID NO:16) and EXc (SEQ ID NO:17) tested in AMSA at 20°C in model detergent A.

| | Intensity | | | | Delta intensity | | | |
|---|---|---|---|---|---|---|---|---|
| Concentration [ppb] | 0.7 | 1.5 | 20 | 125 | 0.7 | 1.5 | 20 | 125 |
| Blank | 210.4 | 210.4 | 210.4 | 210.4 | | | | |
| EXb (SEQ ID NO:16) | 210.8 | 212.8 | 217.2 | 217.8 | 0.4 | 2.4 | 6.8 | 7.5 |
| EXc (SEQ ID NO:17) | 212.0 | 214.4 | 216.5 | 218.4 | 1.6 | 4.1 | 6.2 | 8.0 |

Table 19. Intensity and delta intensity of GH5 polypeptides EXb (SEQ ID NO:16) and EXc (SEQ ID NO:17) tested in AMSA at 40°C in model detergent A.

| | Intensity | | | | Delta intensity | | | |
|---|---|---|---|---|---|---|---|---|
| Concentration [ppb] | 0.7 | 1.5 | 20 | 125 | 0.7 | 1.5 | 20 | 125 |
| Blank | 220.0 | 220.0 | 220.0 | 220.0 | | | | |
| EXb (SEQ ID NO: 16) | 221.9 | 222.9 | 229.4 | 230.2 | 1.9 | 3.0 | 9.4 | 10.2 |
| EXc (SEQ ID NO: 17) | 223.2 | 225.4 | 228.3 | 229.0 | 3.3 | 5.4 | 8.3 | 9.0 |

Table 20. Intensity and delta intensity of GH5 polypeptides EXb (SEQ ID NO:16) and EXc (SEQ ID NO:17) with Xanthan lyase (XLb (SEQ ID NO:22) tested in AMSA at 20°C in model detergent A.

| | Intensity | | | | Delta intensity | | | |
|---|---|---|---|---|---|---|---|---|
| Concentration [ppb] | 0.7 | 1.5 | 20 | 125 | 0.7 | 1.5 | 20 | 125 |
| Blank with XLb (SEQ ID NO: 22) | 214.0 | 214.0 | 214.0 | 214.0 | | | | |

(continued)

| | Intensity | | | | Delta intensity | | | |
|---|---|---|---|---|---|---|---|---|
| EXb (SEQ ID NO:16 with XLb (SEQ ID NO:22) | 213.0 | 215.3 | 220.4 | 223.7 | -1.0 | 1.3 | 6.4 | 9.7 |
| EXc (SEQ ID NO:17) with XLb (SEQ ID NO:22) | 212.4 | 215.1 | 220.2 | 221.4 | -1.6 | 1.1 | 6.2 | 7.4 |

Table 21. Intensity and delta intensity of GH5 polypeptides EXb (SEQ ID NO:16) and EXc (SEQ ID NO:17) with Xanthan lyase (XLb (SEQ ID NO:22) tested in AMSA at 40°C in model detergent A.

| | Intensity | | | | Delta intensity | | | |
|---|---|---|---|---|---|---|---|---|
| Concentration [ppb] | 0.7 | 1.5 | 20 | 125 | 0.7 | 1.5 | 20 | 125 |
| Blank with XLb (SEQ ID NO: 22) | 220.6 | 220.6 | 220.6 | 220.6 | | | | |
| EXb (SEQ ID NO:16 with XLb (SEQ ID NO:22) | 222.0 | 225.0 | 231.0 | 232.6 | 1.3 | 4.4 | 10.3 | 12.0 |
| EXc (SEQ ID NO:17) with XLb (SEQ ID NO:22) | 222.3 | 223.9 | 230.1 | 231.5 | 1.7 | 3.2 | 9.5 | 10.9 |

The results in above tables show that the GH5 polypeptides, e.g., EXb and EXc, have an improved wash performance both when evaluated alone or in combination with the Xanthan Lyase, e.g., XLb.

## Claims

**1.** A polypeptide of glycosyl hydrolase family 5 having xanthan degrading activity, selected from the group consisting of:

(a)

(i) a polypeptide having at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or 100% sequence identity to the mature polypeptide of SEQ ID NO: 2;
(ii) a fragment of the polypeptide of (a)(i) that has xanthan degrading activity; and
(iii) a polypeptide comprising the polypeptide of (a)(i) or (a)(ii) and an N-terminal and/or C-terminal His-tag.;

(b)

(i) a polypeptide having at least 80%, at least 81%, at least 82%, at least 83%, at least 84%, at least 85%,

at least 86%, at least 87%, at least 88%, at least 89%, at least 90%, at least 91%, at least 92%, at least 93%, at least 94%, at least 95%, at least 96%, at least 97%, at least 98%, at least 99%, or 100% sequence identity to the mature polypeptide of SEQ ID NO: 4;
(ii) a fragment of the polypeptide of (b)(i) that has xanthan degrading activity; and
(iii) a polypeptide comprising the polypeptide of (b)(i) or (b)(ii) and an N-terminal and/or C-terminal His-tag;

(c)

(i) a polypeptide having at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or 100% sequence identity to the mature polypeptide of SEQ ID NO: 6;
(ii) a fragment of the polypeptide of (c)(i) that has xanthan degrading activity; and
(iii) a polypeptide comprising the polypeptide of (c)(i) or (c)(ii) and an N-terminal and/or C-terminal His-tag.

**2.** The polypeptide of claim 1, having at least 80%, at least 85%, at least 90%, at least 91%, at least 92%, at least 93%, at least 94%, at least 95%, at least 96%, at least 97%, at least 98%, at least 99% or 100% sequence identity to the mature polypeptide of any of SEQ ID NO: 2, 4, or 6.

**3.** A polypeptide of glycosyl hydrolase family 5 having xanthan degrading activity, consisting of any of SEQ ID NO: 2, 4, or 6 or the mature polypeptide of any of SEQ ID NO: 2, 4, or 6.

**4.** A polypeptide of glycosyl hydrolase family 5 having xanthan degrading activity, comprising any of SEQ ID NO: 2, 4, or 6 or the mature polypeptide of any of SEQ ID NO: 2, 4, or 6.

**5.** A polypeptide of glycosyl hydrolase family 5 having xanthan degrading activity, which is a fragment of any of SEQ ID NO: 2, 4, or 6, wherein the fragment has xanthan degrading activity.

**6.** A polynucleotide encoding the polypeptide of any of claims 1-5.

**7.** A nucleic acid construct or expression vector comprising the polynucleotide of claim 6 operably linked to one or more control sequences that direct the production of the polypeptide in an expression host.

**8.** A recombinant host cell comprising the polynucleotide of claim 6 operably linked to one or more control sequences that direct the production of the polypeptide.

**9.** A method of producing the polypeptide of any of claims 1-5, comprising cultivating a cell, which in its wild-type form produces the polypeptide, under conditions conducive for production of the polypeptide.

**10.** A composition comprising the polypeptide of any of claims 1-5.

**11.** The composition of claim 10, further comprising a polypeptide having xanthan lyase activity.

**12.** The composition of claim 11, wherein the polypeptide having xanthan lyase activity is a polypeptide having the amino acid sequence of any one of SEQ ID NO: 21, 22, 23 or 24.

**13.** Use of a composition according to any of claims 10-12 for degrading xanthan gum.

**14.** A method for degrading xanthan gum comprising applying a composition according to any of claims 10-12 to xanthan gum.

## Patentansprüche

**1.** Polypeptid der Glycosylhydrolase-Familie 5 mit xanthanabbauender Aktivität, ausgewählt aus der Gruppe bestehend aus:

(a)

(i) einem Polypeptid mit mindestens 80%, mindestens 81%, mindestens 82%, mindestens 83%, mindestens 84%, mindestens 85%, mindestens 86%, mindestens 87%, mindestens 88%, mindestens 89%, mindestens 90%, mindestens 91%, mindestens 92%, mindestens 93%, mindestens 94%, mindestens 95%, mindestens 96%, mindestens 97%, mindestens 98%, mindestens 99% oder 100% Sequenzidentität zum reifen Polypeptid von SEQ ID NO: 2;
(ii) einem Fragment des Polypeptids von (a)(i), das xanthanabbauende Aktivität aufweist; und
(iii) einem Polypeptid, das das Polypeptid von (a)(i) oder (a)(ii) und einen N-terminalen und/oder C-terminalen His-Tag umfasst;

(b)

(i) einem Polypeptid mit mindestens 80%, mindestens 81%, mindestens 82%, mindestens 83%, mindestens 84%, mindestens 85%, mindestens 86%, mindestens 87%, mindestens 88%, mindestens 89%, mindestens 90%, mindestens 91%, mindestens 92%, mindestens 93%, mindestens 94%, mindestens 95%, mindestens 96%, mindestens 97%, mindestens 98%, mindestens 99% oder 100% Sequenzidentität zum reifen Polypeptid von SEQ ID NO: 4;
(ii) einem Fragment des Polypeptids von (b)(i), das xanthanabbauende Aktivität aufweist; und
(iii) einem Polypeptid, das das Polypeptid von (b)(i) oder (b)(ii) und einen N-terminalen und/oder C-terminalen His-Tag umfasst;

(c)

(i) einem Polypeptid mit mindestens 80%, mindestens 81%, mindestens 82%, mindestens 83%, mindestens 84%, mindestens 85%, mindestens 86%, mindestens 87%, mindestens 88%, mindestens 89%, mindestens 90%, mindestens 91%, mindestens 92%, mindestens 93%, mindestens 94%, mindestens 95%, mindestens 96%, mindestens 97%, mindestens 98%, mindestens 99% oder 100% Sequenzidentität zum reifen Polypeptid von SEQ ID NO: 6;
(ii) einem Fragment des Polypeptids von (c)(i), das xanthanabbauende Aktivität aufweist; und
(iii) einem Polypeptid, das das Polypeptid von (c)(i) oder (c)(ii) und einen N-terminalen und/oder C-terminalen His-Tag umfasst.

**2.** Polypeptid nach Anspruch 1 mit mindestens 80%, mindestens 85%, mindestens 90%, mindestens 91%, mindestens 92%, mindestens 93%, mindestens 94%, mindestens 95%, mindestens 96%, mindestens 97%, mindestens 98%, mindestens 99% oder 100% Sequenzidentität zum reifen Polypeptid einer beliebigen von SEQ ID NO: 2, 4 oder 6.

**3.** Polypeptid der Glycosylhydrolase-Familie 5 mit xanthanabbauender Aktivität, das aus einer beliebigen von SEQ ID NO: 2, 4 oder 6 oder dem reifen Polypeptid einer beliebigen von SEQ ID NO: 2, 4 oder 6 besteht.

**4.** Polypeptid der Glycosylhydrolase-Familie 5 mit xanthanabbauender Aktivität, das eine beliebige von SEQ ID NO: 2, 4 oder 6 oder das reife Polypeptid einer beliebigen von SEQ ID NO: 2, 4 oder 6 umfasst.

**5.** Polypeptid der Glycosylhydrolase-Familie 5 mit xanthanabbauender Aktivität, das ein Fragment einer beliebigen von SEQ ID NO: 2, 4 oder 6 ist, wobei das Fragment xanthanabbauende Aktivität aufweist.

**6.** Polynukleotid, das das Polypeptid gemäß einem beliebigen der Ansprüche 1-5 kodiert.

**7.** Nukleinsäurekonstrukt oder Expressionsvektor, umfassend das Polynukleotid gemäß Anspruch 6, funktionsfähig verknüpft mit einer oder mehreren Kontrollsequenzen, die die Herstellung des Polypeptids in einem Expressionswirt steuert/steuern.

**8.** Rekombinante Wirtszelle, die das Polynukleotid gemäß Anspruch 6 umfasst, funktionsfähig verknüpft mit einer oder mehreren Kontrollsequenzen, die die Herstellung des Polypeptids steuert/steuern.

**9.** Verfahren zum Herstellen des Polypeptids gemäß einem beliebigen der Ansprüche 1-5, das Kultivieren einer Zelle, die in ihrer Wildtyp-Form das Polypeptid herstellt, unter Bedingungen, die zur Herstellung des Polypeptids förderlich sind, umfasst.

**10.** Zusammensetzung, die das Polypeptid gemäß einem beliebigen der Ansprüche 1-5 umfasst.

**11.** Zusammensetzung nach Anspruch 10, die des Weiteren ein Polypeptid mit Xanthanlyaseaktivität umfasst.

**12.** Zusammensetzung nach Anspruch 11, wobei das Polypeptid mit Xanthanlyaseaktivität ein Polypeptid mit der Aminosäuresequenz einer beliebigen der Sequenzen von SEQ ID NO: 21, 22, 23 oder 24 ist.

**13.** Verwendung einer Zusammensetzung gemäß einem beliebigen der Ansprüche 10-12 zum Abbauen von Xanthangummi.

**14.** Verfahren zum Abbauen von Xanthangummi, das Anwenden einer Zusammensetzung gemäß einem beliebigen der Ansprüche 10-12 auf Xanthangummi umfasst.

**Revendications**

**1.** Polypeptide de la famille des glycosyle hydrolases 5 ayant une activité de dégradation du xanthane, choisi dans le groupe constitué par :

(a)

(i) un polypeptide ayant une identité de séquence d'au moins 80 %, d'au moins 81 %, d'au moins 82 %, d'au moins 83 %, d'au moins 84 %, d'au moins 85 %, d'au moins 86 %, d'au moins 87 %, d'au moins 88 %, d'au moins 89 %, d'au moins 90 %, d'au moins 91 %, d'au moins 92 %, d'au moins 93 %, d'au moins 94 %, d'au moins 95 %, d'au moins 96 %, d'au moins 97 %, d'au moins 98 %, d'au moins 99 % ou de 100 % avec le polypeptide mature de la SEQ ID NO : 2 ;
(ii) un fragment du polypeptide de (a)(i) qui a une activité de dégradation du xanthane ; et
(iii) un polypeptide comprenant le polypeptide de (a)(i) ou (a)(ii) et une étiquette His N-terminale et/ou C-terminale ;

(b)

(i) un polypeptide ayant une identité de séquence d'au moins 80 %, d'au moins 81 %, d'au moins 82 %, d'au moins 83 %, d'au moins 84 %, d'au moins 85 %, d'au moins 86 %, d'au moins 87 %, d'au moins 88 %, d'au moins 89 %, d'au moins 90 %, d'au moins 91 %, d'au moins 92 %, d'au moins 93 %, d'au moins 94 %, d'au moins 95 %, d'au moins 96 %, d'au moins 97 %, d'au moins 98 %, d'au moins 99 % ou de 100 % avec le polypeptide mature de la SEQ ID NO : 4 ;
(ii) un fragment du polypeptide de (b)(i) qui a une activité de dégradation du xanthane ; et
(iii) un polypeptide comprenant le polypeptide de (b)(i) ou (b)(ii) et une étiquette His N-terminale et/ou C-terminale ;

(c)

(i) un polypeptide ayant une identité de séquence d'au moins 80 %, d'au moins 81 %, d'au moins 82 %, d'au moins 83 %, d'au moins 84 %, d'au moins 85 %, d'au moins 86 %, d'au moins 87 %, d'au moins 88 %, d'au moins 89 %, d'au moins 90 %, d'au moins 91 %, d'au moins 92 %, d'au moins 93 %, d'au moins 94 %, d'au moins 95 %, d'au moins 96 %, d'au moins 97 %, d'au moins 98 %, d'au moins 99 % ou de 100 % avec le polypeptide mature de la SEQ ID NO : 6 ;
(ii) un fragment du polypeptide de (c)(i) qui a une activité de dégradation du xanthane ; et
(iii) un polypeptide comprenant le polypeptide de (c)(i) ou (c)(ii) et une étiquette His N-terminale et/ou C-terminale.

**2.** Polypeptide selon la revendication 1, ayant une identité de séquence d'au moins 80 %, d'au moins 85 %, d'au moins 90 %, d'au moins 91 %, d'au moins 92 %, d'au moins 93 %, d'au moins 94 %, d'au moins 95 %, d'au moins 96 %, d'au moins 97 %, d'au moins 98 %, d'au moins 99 % ou de 100 % avec le polypeptide mature de l'une quelconque des SEQ ID NO : 2, 4, et 6.

**3.** Polypeptide de la famille des glycosyle hydrolases 5 ayant une activité de dégradation du xanthane, consistant en l'une quelconque des SEQ ID NO : 2, 4 et 6 ou le polypeptide mature de l'une quelconque des SEQ ID NO : 2, 4 et 6.

4. Polypeptide de la famille des glycosyle hydrolases 5 ayant une activité de dégradation du xanthane, comprenant l'une quelconque des SEQ ID NO : 2, 4 et 6 ou le polypeptide mature de l'une quelconque des SEQ ID NO : 2, 4 et 6.

5. Polypeptide de la famille des glycosyle hydrolases 5 ayant une activité de dégradation du xanthane qui est un fragment de l'une quelconque des SEQ ID NO : 2, 4 et 6, dans lequel le fragment a une activité de dégradation du xanthane.

6. Polynucléotide codant le polypeptide de l'une quelconque des revendications 1 à 5.

7. Produit d'assemblage d'acide nucléique ou vecteur d'expression comprenant le polynucléotide de la revendication 6 lié de manière fonctionnelle à une ou plusieurs séquences de contrôle qui dirigent la production du polypeptide dans un hôte d'expression.

8. Cellule hôte recombinée comprenant le polynucléotide de la revendication 6 lié de manière fonctionnelle à une ou plusieurs séquences de contrôle qui dirigent la production du polypeptide.

9. Méthode de production du polypeptide de l'une quelconque des revendications 1 à 5, comprenant la culture d'une cellule qui, sous sa forme de type sauvage, produit le polypeptide, dans des conditions propices à la production du polypeptide.

10. Composition comprenant le polypeptide de l'une quelconque des revendications 1 à 5.

11. Composition selon la revendication 10, comprenant en outre un polypeptide ayant une activité de xanthane lyase.

12. Composition selon la revendication 11, dans laquelle le polypeptide ayant une activité de xanthane lyase est un polypeptide ayant la séquence d'acides aminés de l'une quelconque des SEQ ID NO : 21, 22, 23 et 24.

13. Utilisation d'une composition de l'une quelconque des revendications 10 à 12 pour dégrader la gomme xanthane.

14. Méthode pour dégrader la gomme xanthane, comprenant l'application d'une composition de l'une quelconque des revendications 10 à 12 à de la gomme xanthane.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2013167581 A
- WO 9943835 A
- WO 9600787 A
- WO 0056900 A
- US 6011147 A
- WO 9425612 A
- WO 9533836 A
- WO 2010039889 A
- WO 0024883 A
- EP 238023 A
- WO 9015861 A
- WO 2010096673 A
- WO 9219709 A
- WO 9219708 A
- EP 1025240 A
- WO 09102854 A
- US 5977053 A
- WO 9817767 A
- EP 624154 A
- WO 2007087258 A
- WO 2007087244 A
- WO 2007087259 A
- EP 1867708 A
- WO 2007087242 A
- WO 2006130575 A
- WO 200503274 A
- WO 200503275 A
- WO 200503276 A
- EP 1876226 A
- WO 2007087257 A
- WO 2007087243 A
- US 4435307 A
- US 5648263 A
- US 5691178 A
- US 5776757 A
- WO 8909259 A
- EP 0495257 A
- EP 0531372 A
- WO 9611262 A
- WO 9629397 A
- WO 9808940 A
- WO 9407998 A
- EP 0531315 A
- US 5457046 A
- US 5686593 A
- US 5763254 A
- WO 9524471 A
- WO 9812307 A
- WO 99001544 A
- WO 2002099091 A
- WO 2001062903 A
- WO 1999064619 A
- US 7262042 B
- WO 09021867 A
- WO 8906279 A
- WO 9318140 A
- WO 92175177 A
- WO 01016285 A
- WO 02026024 A
- WO 02016547 A
- WO 8906270 A
- WO 9425583 A
- WO 05040372 A
- WO 05052161 A
- WO 05052146 A
- WO 9523221 A
- WO 9221760 A
- EP 1921147 A
- EP 1921148 A
- WO 07044993 A
- WO 9219729 A
- WO 96034946 A
- WO 9820115 A
- WO 9820116 A
- WO 99011768 A
- WO 0144452 A
- WO 03006602 A
- WO 0403186 A
- WO 04041979 A
- WO 07006305 A
- WO 11036263 A
- WO 11036264 A
- US 5352604 A
- EP 258068 A
- EP 305216 A
- WO 9613580 A
- EP 218272 A
- EP 331376 A
- WO 9506720 A
- WO 9627002 A
- WO 9612012 A
- WO 10065455 A
- WO 10107560 A
- US 5389536 A
- WO 11084412 A
- WO 11084417 A
- WO 11084599 A
- WO 11150157 A

- WO 12137147 A
- EP 407225 A
- WO 9205249 A
- WO 9401541 A
- WO 9425578 A
- WO 9514783 A
- WO 9530744 A
- WO 9535381 A
- WO 9522615 A
- WO 9600292 A
- WO 9704079 A
- WO 9707202 A
- WO 0034450 A
- WO 0060063 A
- WO 0192502 A
- WO 0787508 A
- WO 09109500 A
- WO 10111143 A
- WO 0556782 A
- WO 0967279 A
- WO 10100028 A
- GB 1296839 A
- WO 9510603 A
- WO 9402597 A
- WO 9418314 A
- WO 9743424 A
- WO 99019467 A
- WO 02010355 A
- WO 2006066594 A
- WO 96023873 A
- WO 08153815 A
- WO 0166712 A
- WO 09061380 A
- WO 13184577 A
- WO 10104675 A
- WO 2011098531 A
- WO 2013001078 A
- WO 2013001087 A
- EP 179486 A
- WO 9324618 A
- WO 9510602 A
- WO 9815257 A
- WO 9527046 A
- WO 9704102 A
- WO 0179459 A
- WO 0179458 A
- WO 0179461 A
- WO 0179460 A
- WO 9201046 A
- JP 2238885 A
- WO 9708325 A
- US 4106991 A
- US 4661452 A
- GB 1483591 A
- EP 238216 A
- WO 2009087523 A
- WO 2007138054 A
- WO 2006108856 A
- WO 2006113314 A
- EP 1867808 A
- WO 2003040279 A
- EP 2169040 A
- US 20090011970 A1
- WO 2013188331 A
- US 7360593 B
- US 5806597 A
- US 5562160 A
- US 5201370 A
- US 5067566 A
- WO 2012025577 A
- WO 2011107472 A

**Non-patent literature cited in the description**

- **RUIJSSENAARS et al.** A pyruvated mannose-specific xanthan lyase involved in xanthan degradation by Paenibacillus alginolyticus XL-1. *Appl. Environ. Microbiol.,* 1999, vol. 65 (6), 2446-2452
- **RUIJSSENAARS et al.** A novel gene encoding xanthan lyase of Paenibacillus alginolyticus strain XL-1. *Appl. Environ. Microbiol.,* 2000, vol. 66 (9), 3945-3950
- **HENRISSAT et al.** A classification of glycosyl hydrolases based on amino-acid sequence similarities. *J. Biochem.,* 1991, vol. 280, 309-316
- **NEEDLEMAN ; WUNSCH.** *J. Mol. Biol.,* 1970, vol. 48, 443-453
- **RICE et al.** Trends Genet. *EMBOSS: The European Molecular Biology Open Software Suite,* 2000, vol. 16, 276-277
- **RICE et al.** *EMBOSS: The European Molecular Biology Open Software Suite,* 2000
- **SAMBROOK et al.** Molecular Cloning, A Laboratory Manual. Cold Spring Harbor, 1989
- **H. NEURATH ; R.L. HILL.** The Proteins. Academic Press, 1979
- **INNIS et al.** PCR: A Guide to Methods and Application. Academic Press, 1990
- **AGAISSE ; LERECLUS.** *Molecular Microbiology,* 1994, vol. 13, 97-107
- **EGON et al.** *Gene,* 1988, vol. 69, 301-315
- **VILLA-KAMAROFF et al.** *Proc. Natl. Acad. Sci. USA,* 1978, vol. 75, 3727-3731
- **DEBOER et al.** *Proc. Natl. Acad. Sci. USA,* 1983, vol. 80, 21-25
- **GILBERT et al.** Useful proteins from recombinant bacteria. *Scientific American,* 1980, vol. 242, 74-94
- **ROMANOS et al.** *Yeast,* 1992, vol. 8, 423-488

- **HUE et al.** *Journal of Bacteriology,* 1995, vol. 177, 3465-3471
- **GUO ; SHERMAN.** *Mol. Cellular Biol.,* 1995, vol. 15, 5983-5990
- **SIMONEN ; PALVA.** *Microbiological Reviews,* 1993, vol. 57, 109-137
- **GEMS et al.** *Gene,* 1991, vol. 98, 61-67
- **CULLEN et al.** *Nucleic Acids Res.,* 1987, vol. 15, 9163-9175
- **CHANG ; COHEN.** *Mol. Gen. Genet.,* 1979, vol. 168, 111-115
- **YOUNG ; SPIZIZEN.** *J. Bacteriol.,* 1961, vol. 81, 823-829
- **DUBNAU ; DAVIDOFF-ABELSON.** *J. Mol. Biol.,* 1971, vol. 56, 209-221
- **SHIGEKAWA ; DOWER.** *Biotechniques,* 1988, vol. 6, 742-751
- **KOEHLER ; THORNE.** *J. Bacteriol.,* 1987, vol. 169, 5271-5278
- **HANAHAN.** *J. Mol. Biol.,* 1983, vol. 166, 557-580
- **DOWER et al.** *Nucleic Acids Res.,* 1988, vol. 16, 6127-6145
- **GONG et al.** *Folia Microbiol,* 2004, vol. 49, 399-405
- **MAZODIER et al.** *J. Bacteriol.,* 1989, vol. 171, 3583-3585
- **BURKE et al.** *Proc. Natl. Acad. Sci. USA,* 2001, vol. 98, 6289-6294
- **CHOI et al.** *J. Microbiol. Methods,* 2006, vol. 64, 391-397
- **PINEDO ; SMETS.** *Appl. Environ. Microbiol.,* 2005, vol. 71, 51-57
- **PERRY ; KURAMITSU.** *Infect. Immun.,* 1981, vol. 32, 1295-1297
- **CATT ; JOLLICK.** *Microbios,* 1991, vol. 68, 189-207
- **BUCKLEY et al.** *Appl. Environ. Microbiol.,* 1999, vol. 65, 3800-3804
- **CLEWELL.** *Microbiol. Rev.,* 1981, vol. 45, 409-436
- **HAWKSWORTH et al.** Ainsworth and Bisby's Dictionary of The Fungi. CAB International, University Press, 1995
- Biology and Activities of Yeast. Soc. App. Bacteriol. Symposium Series No. 9. 1980
- **YELTON et al.** *Proc. Natl. Acad. Sci. USA,* 1984, vol. 81, 1470-1474
- **CHRISTENSEN et al.** *Bio/Technology,* 1988, vol. 6, 1419-1422
- **MALARDIER et al.** *Gene,* 1989, vol. 78, 147-156
- **BECKER ; GUARENTE.** Guide to Yeast Genetics and Molecular Biology, Methods in Enzymology. Academic Press, Inc, vol. 194, 182-187
- **ITO et al.** *J. Bacteriol.,* 1983, vol. 153, 163
- **HINNEN et al.** *Proc. Natl. Acad. Sci. USA,* 1978, vol. 75, 1920
- Protein Purification. VCH Publishers, 1989
- **HODGDON ; KALER.** *Current Opinion in Colloid & Interface Science,* 2007, vol. 12, 121-128
- **SIEZEN et al.** *Protein Engng.,* 1991, vol. 4, 719-737
- **SIEZEN et al.** *Protein Science,* 1997, vol. 6, 501-523
- Powdered Detergents, Surfactant science series. Marcel Dekker, Inc, vol. 71
- **LEVER.** *Anal. Biochem.,* 1972, vol. 47, 273-279
- **DIDERICHSEN et al.** *Plasmid,* 1993, vol. 30, 312-315
- **NANKAI et al.** Microbial system for polysaccharide depolymerization: enzymatic route for xanthan depolymerization by Bacillus sp strain GL1. *Applied and Environmental Microbiology,* 1999, vol. 65 (6), 2520-2526

SEQUENCE LISTING

<110> Novozymes A/S

<120> POLYPEPTIDES HAVING XANTHAN DEGRADING ACTIVITY AND POLYNUCLEOTIDES ENCODING SAME

<130> 12815-WO-PCT

<160> 24

<170> PatentIn version 3.5

<210> 1
<211> 2517
<212> DNA
<213> Opitutaceae sp

<220>
<221> CDS
<222> (1)..(2514)

<220>
<221> sig_peptide
<222> (1)..(108)

<220>
<221> mat_peptide
<222> (109)..(2514)

<400> 1

```
atg caa tca tca agc tca aat tcg gtc gta tcc gct tcc cgg ata ctc       48
Met Gln Ser Ser Ser Ser Asn Ser Val Val Ser Ala Ser Arg Ile Leu
    -35                 -30                 -25

cga cgc ttc tcc ctc ccg ctg ctc gcc gcc gcg ctg ggc ctc gcc gcg       96
Arg Arg Phe Ser Leu Pro Leu Leu Ala Ala Ala Leu Gly Leu Ala Ala
-20                 -15                 -10                 -5

ccc gcc cgc gcc gcc gac tat tac ctg aag gcc agc caa ggc gca tcc      144
Pro Ala Arg Ala Ala Asp Tyr Tyr Leu Lys Ala Ser Gln Gly Ala Ser
            -1  1               5                   10

aac cac tgg tcc tcc cat ctc acc gac tgg acc gcc aac gcc gac ggc      192
Asn His Trp Ser Ser His Leu Thr Asp Trp Thr Ala Asn Ala Asp Gly
        15                  20                  25

acc ggc gcc aac ccg acg gtc atc ggc ctg gcc gac acc ttc gac acc      240
Thr Gly Ala Asn Pro Thr Val Ile Gly Leu Ala Asp Thr Phe Asp Thr
    30                  35                  40

aac aac cgc acg ctt cgc act ccc gcc gtc aac gcc acc acc acc tac      288
Asn Asn Arg Thr Leu Arg Thr Pro Ala Val Asn Ala Thr Thr Thr Tyr
45                  50                  55                  60

ccg ggc ggc gtg ctc cgc ctt tcc ggc ggc gcc ggc gtc atc ggc atg      336
Pro Gly Gly Val Leu Arg Leu Ser Gly Gly Ala Gly Val Ile Gly Met
                65                  70                  75

aag act ggc ggc acc gcc gtc gcc atc gtg ccc aag ctc gtc tcc acc      384
Lys Thr Gly Gly Thr Ala Val Ala Ile Val Pro Lys Leu Val Ser Thr
            80                  85                  90
```

```
gcc ggc acc gtg gac gcc tgg cac acc ggc acc caa tac ttc cgc gcc        432
Ala Gly Thr Val Asp Ala Trp His Thr Gly Thr Gln Tyr Phe Arg Ala
        95                  100                 105

gac gac tgg gag aac ctc gcc tcc ggc acc ggg ttc acc gcg ctc aag        480
Asp Asp Trp Glu Asn Leu Ala Ser Gly Thr Gly Phe Thr Ala Leu Lys
    110                 115                 120

gcc gtc gcc ggc cgc acg ctc aag gtc agc gtc ggc aag ctc acc ggc        528
Ala Val Ala Gly Arg Thr Leu Lys Val Ser Val Gly Lys Leu Thr Gly
125                 130                 135                 140

tcc ggc gag acc cgt ctt cac ggc ggc ggc gcc gtc cgc ctc gac gtc        576
Ser Gly Glu Thr Arg Leu His Gly Gly Gly Ala Val Arg Leu Asp Val
                145                 150                 155

acc gac ggc gaa cgc tac ctc ggc gtc gtc cgc gtc tcc tcc ggc gcg        624
Thr Asp Gly Glu Arg Tyr Leu Gly Val Val Arg Val Ser Ser Gly Ala
            160                 165                 170

gcc gac ttc gac aac aac gtg ttc gtc tcc ggc ccg ctc gtg atc gag        672
Ala Asp Phe Asp Asn Asn Val Phe Val Ser Gly Pro Leu Val Ile Glu
        175                 180                 185

acc ggc gcg acc gtc gtg ctc gac cag gcc gtc tcc ttc gcc ggc ctg        720
Thr Gly Ala Thr Val Val Leu Asp Gln Ala Val Ser Phe Ala Gly Leu
    190                 195                 200

acc gtc gcc ggc acc gag tat tcg ccc ggc aac tac acc ttc gcc gcg        768
Thr Val Ala Gly Thr Glu Tyr Ser Pro Gly Asn Tyr Thr Phe Ala Ala
205                 210                 215                 220

ctc cag gcc gcg cat cct acg gtg ttc acc tcc ggc acc gcc ggc ggc        816
Leu Gln Ala Ala His Pro Thr Val Phe Thr Ser Gly Thr Ala Gly Gly
                225                 230                 235

tcg atc acc gtc cgc gcc ccg cgc acc tgg tat ctc acc gtg aat cag        864
Ser Ile Thr Val Arg Ala Pro Arg Thr Trp Tyr Leu Thr Val Asn Gln
            240                 245                 250

ggc ggc gtg cag aac tgg acc gag acc tac ctt tcg aac tgg aac tcc        912
Gly Gly Val Gln Asn Trp Thr Glu Thr Tyr Leu Ser Asn Trp Asn Ser
        255                 260                 265

gcc gcc aat ggc tcc ggc gtc gcg ccg act tcg atc aac ggc tac gac        960
Ala Ala Asn Gly Ser Gly Val Ala Pro Thr Ser Ile Asn Gly Tyr Asp
    270                 275                 280

ttc tac atc gat cag gtc tcc aac cgc gag atc cgc acg ccc tcc acc       1008
Phe Tyr Ile Asp Gln Val Ser Asn Arg Glu Ile Arg Thr Pro Ser Thr
285                 290                 295                 300

gcc tcc acc ttc ggc ggc ggc gcg ctc gcc ctc gcc agc ggc gcc aag       1056
Ala Ser Thr Phe Gly Gly Gly Ala Leu Ala Leu Ala Ser Gly Ala Lys
                305                 310                 315

ctc acc ctc aag agt tcg ccc ggc gtc gtc agc acc atc ccg gcg ttc       1104
Leu Thr Leu Lys Ser Ser Pro Gly Val Val Ser Thr Ile Pro Ala Phe
            320                 325                 330

gtg aac acg aac tcc ccg atc atc gtg aac ggc ggc ggt agc ttc cgc       1152
Val Asn Thr Asn Ser Pro Ile Ile Val Asn Gly Gly Gly Ser Phe Arg
```

```
        335                 340                 345

caa agt ctc gcc ctc ggt gac tgg gag atc gcc tcc ggc atc acc aag        1200
Gln Ser Leu Ala Leu Gly Asp Trp Glu Ile Ala Ser Gly Ile Thr Lys
    350                 355                 360

ctc tcc gcc ggc tcc ggt cgc agc ctc ggc ttc gac atc gac tac ctc        1248
Leu Ser Ala Gly Ser Gly Arg Ser Leu Gly Phe Asp Ile Asp Tyr Leu
365                 370                 375                 380

ggc ggc gcg ggt ggc ctt gtc acc caa aac ggc ggc tct tac ttc ctc        1296
Gly Gly Ala Gly Gly Leu Val Thr Gln Asn Gly Gly Ser Tyr Phe Leu
                385                 390                 395

agc ctc gac gac ggc tcc ggc tac acc ggc acg ctc aac cac gcg tcc        1344
Ser Leu Asp Asp Gly Ser Gly Tyr Thr Gly Thr Leu Asn His Ala Ser
            400                 405                 410

ggc gcg ctc cgc ttc gag tcc gtc ttc tcc acc gag ggc gcg ctc acc        1392
Gly Ala Leu Arg Phe Glu Ser Val Phe Ser Thr Glu Gly Ala Leu Thr
        415                 420                 425

atc ggc tcc tcg gcg acc gtc cac ctc gac caa cag gtt tac gtc acg        1440
Ile Gly Ser Ser Ala Thr Val His Leu Asp Gln Gln Val Tyr Val Thr
    430                 435                 440

tcg ttc tcc gtc gcc ggt gtc gcc aag gcc gcc ggc atc cac acc tac        1488
Ser Phe Ser Val Ala Gly Val Ala Lys Ala Ala Gly Ile His Thr Tyr
445                 450                 455                 460

gcc tcg ctg aac gcc gcg cat ccc gca cag ttc acc gcc ggc gcc gcg        1536
Ala Ser Leu Asn Ala Ala His Pro Ala Gln Phe Thr Ala Gly Ala Ala
                465                 470                 475

ccc gga ctc gtc gct gtt tac acg ccc gac acc gcc ggc ccc gtc cgc        1584
Pro Gly Leu Val Ala Val Tyr Thr Pro Asp Thr Ala Gly Pro Val Arg
            480                 485                 490

atg aac ggc gtc aat atc tcc ggc ccc gag agc aac acc gcc aac ctc        1632
Met Asn Gly Val Asn Ile Ser Gly Pro Glu Ser Asn Thr Ala Asn Leu
        495                 500                 505

ccc ggc acc tac ggc tac aac tac gtt tac ccc acc gag gcc gac ttc        1680
Pro Gly Thr Tyr Gly Tyr Asn Tyr Val Tyr Pro Thr Glu Ala Asp Phe
    510                 515                 520

gac tac tac gcc tcc aag ggc ctc aac ctc atc cgc att ccc ttc cgc        1728
Asp Tyr Tyr Ala Ser Lys Gly Leu Asn Leu Ile Arg Ile Pro Phe Arg
525                 530                 535                 540

tgg gag cgc atg cag cac ggc ctg aac gtt ccg ctc aac acc gcc cag        1776
Trp Glu Arg Met Gln His Gly Leu Asn Val Pro Leu Asn Thr Ala Gln
                545                 550                 555

ctc ggc tac atg gac acc gcc gtc gcc cgc gcc tcc gcg cgc ggc atg        1824
Leu Gly Tyr Met Asp Thr Ala Val Ala Arg Ala Ser Ala Arg Gly Met
            560                 565                 570

aag gtc atc ctc gat atg cac aac tac gcc cgc tgc aaa gtc ggc gga        1872
Lys Val Ile Leu Asp Met His Asn Tyr Ala Arg Cys Lys Val Gly Gly
        575                 580                 585

gtc acc tac aag ttc ggc gac gcg cag ctc ccc gcc tcg gcc tac gcc        1920
```

```
Val Thr Tyr Lys Phe Gly Asp Ala Gln Leu Pro Ala Ser Ala Tyr Ala
    590                 595                 600

gac gtc tgg cgc cgt ctc gcc gac cac tac aaa aac gag ccc gcc atc      1968
Asp Val Trp Arg Arg Leu Ala Asp His Tyr Lys Asn Glu Pro Ala Ile
605                 610                 615                 620

tac ggc ttc gac atc atg aac gag ccc aac ggc ctc tcc ggc ggc gtc      2016
Tyr Gly Phe Asp Ile Met Asn Glu Pro Asn Gly Leu Ser Gly Gly Val
                625                 630                 635

tgg ccc gcc tac gcc cag gcc gcg gtc aac gcc atc cgc gag gtc aat      2064
Trp Pro Ala Tyr Ala Gln Ala Ala Val Asn Ala Ile Arg Glu Val Asn
            640                 645                 650

ctg tcc acc tgg gtc atc gtc gag ggc gag ttt tgg gcc aac gct tgg      2112
Leu Ser Thr Trp Val Ile Val Glu Gly Glu Phe Trp Ala Asn Ala Trp
        655                 660                 665

ggc ttc gag acc aag aac ccg tat ctg cac aac gtc cgc gat ccc gtc      2160
Gly Phe Glu Thr Lys Asn Pro Tyr Leu His Asn Val Arg Asp Pro Val
    670                 675                 680

ggc cgc ctc atg ttc tcc gcc cac tcc tac tgg agc gac gcc ggc acc      2208
Gly Arg Leu Met Phe Ser Ala His Ser Tyr Trp Ser Asp Ala Gly Thr
685                 690                 695                 700

gat gtt tac aag acc tac gac gaa gag ggc gcc tat ccc gag atg ggc      2256
Asp Val Tyr Lys Thr Tyr Asp Glu Glu Gly Ala Tyr Pro Glu Met Gly
                705                 710                 715

gtg aac aac gtg aag ccc ttc atc gac tgg ctg aag aag cac gac gcc      2304
Val Asn Asn Val Lys Pro Phe Ile Asp Trp Leu Lys Lys His Asp Ala
            720                 725                 730

aag ggc ttc gtc ggc gaa tac ggc gtg ccc aac aac gac ccg cgc tgg      2352
Lys Gly Phe Val Gly Glu Tyr Gly Val Pro Asn Asn Asp Pro Arg Trp
        735                 740                 745

ctc gtc gtg ctg gac aac ttc ctc gcc tac ctc gcg gcc gag ggc gtg      2400
Leu Val Val Leu Asp Asn Phe Leu Ala Tyr Leu Ala Ala Glu Gly Val
    750                 755                 760

agc ggc acc tac tgg gcc ggc ggc gcc tgg tat tcg ggc agc ccg atc      2448
Ser Gly Thr Tyr Trp Ala Gly Gly Ala Trp Tyr Ser Gly Ser Pro Ile
765                 770                 775                 780

agc tgc cac ccg tcc tcc aac tac acc gtg gat cgc gcc gtc atg agc      2496
Ser Cys His Pro Ser Ser Asn Tyr Thr Val Asp Arg Ala Val Met Ser
                785                 790                 795

gtg ctc gaa gac cat cca tga                                          2517
Val Leu Glu Asp His Pro
            800
```

<210> 2
<211> 838
<212> PRT
<213> Opitutaceae sp

<400> 2

```
Met Gln Ser Ser Ser Ser Asn Ser Val Val Ser Ala Ser Arg Ile Leu
    -35                 -30                 -25

Arg Arg Phe Ser Leu Pro Leu Leu Ala Ala Ala Leu Gly Leu Ala Ala
-20                 -15                 -10                 -5

Pro Ala Arg Ala Ala Asp Tyr Tyr Leu Lys Ala Ser Gln Gly Ala Ser
            -1  1               5                   10

Asn His Trp Ser Ser His Leu Thr Asp Trp Thr Ala Asn Ala Asp Gly
        15                  20                  25

Thr Gly Ala Asn Pro Thr Val Ile Gly Leu Ala Asp Thr Phe Asp Thr
    30                  35                  40

Asn Asn Arg Thr Leu Arg Thr Pro Ala Val Asn Ala Thr Thr Thr Tyr
45                  50                  55                  60

Pro Gly Gly Val Leu Arg Leu Ser Gly Gly Ala Gly Val Ile Gly Met
                65                  70                  75

Lys Thr Gly Gly Thr Ala Val Ala Ile Val Pro Lys Leu Val Ser Thr
            80                  85                  90

Ala Gly Thr Val Asp Ala Trp His Thr Gly Thr Gln Tyr Phe Arg Ala
        95                  100                 105

Asp Asp Trp Glu Asn Leu Ala Ser Gly Thr Gly Phe Thr Ala Leu Lys
    110                 115                 120

Ala Val Ala Gly Arg Thr Leu Lys Val Ser Val Gly Lys Leu Thr Gly
125                 130                 135                 140

Ser Gly Glu Thr Arg Leu His Gly Gly Gly Ala Val Arg Leu Asp Val
                145                 150                 155

Thr Asp Gly Glu Arg Tyr Leu Gly Val Val Arg Val Ser Ser Gly Ala
            160                 165                 170

Ala Asp Phe Asp Asn Asn Val Phe Val Ser Gly Pro Leu Val Ile Glu
        175                 180                 185

Thr Gly Ala Thr Val Val Leu Asp Gln Ala Val Ser Phe Ala Gly Leu
    190                 195                 200

Thr Val Ala Gly Thr Glu Tyr Ser Pro Gly Asn Tyr Thr Phe Ala Ala
205                 210                 215                 220
```

```
Leu Gln Ala Ala His Pro Thr Val Phe Thr Ser Gly Thr Ala Gly Gly
                225                 230                 235

Ser Ile Thr Val Arg Ala Pro Arg Thr Trp Tyr Leu Thr Val Asn Gln
            240                 245                 250

Gly Gly Val Gln Asn Trp Thr Glu Thr Tyr Leu Ser Asn Trp Asn Ser
        255                 260                 265

Ala Ala Asn Gly Ser Gly Val Ala Pro Thr Ser Ile Asn Gly Tyr Asp
    270                 275                 280

Phe Tyr Ile Asp Gln Val Ser Asn Arg Glu Ile Arg Thr Pro Ser Thr
285                 290                 295                 300

Ala Ser Thr Phe Gly Gly Gly Ala Leu Ala Leu Ala Ser Gly Ala Lys
                305                 310                 315

Leu Thr Leu Lys Ser Ser Pro Gly Val Val Ser Thr Ile Pro Ala Phe
            320                 325                 330

Val Asn Thr Asn Ser Pro Ile Ile Val Asn Gly Gly Gly Ser Phe Arg
        335                 340                 345

Gln Ser Leu Ala Leu Gly Asp Trp Glu Ile Ala Ser Gly Ile Thr Lys
    350                 355                 360

Leu Ser Ala Gly Ser Gly Arg Ser Leu Gly Phe Asp Ile Asp Tyr Leu
365                 370                 375                 380

Gly Gly Ala Gly Gly Leu Val Thr Gln Asn Gly Gly Ser Tyr Phe Leu
                385                 390                 395

Ser Leu Asp Asp Gly Ser Gly Tyr Thr Gly Thr Leu Asn His Ala Ser
            400                 405                 410

Gly Ala Leu Arg Phe Glu Ser Val Phe Ser Thr Glu Gly Ala Leu Thr
        415                 420                 425

Ile Gly Ser Ser Ala Thr Val His Leu Asp Gln Gln Val Tyr Val Thr
    430                 435                 440

Ser Phe Ser Val Ala Gly Val Ala Lys Ala Ala Gly Ile His Thr Tyr
445                 450                 455                 460

Ala Ser Leu Asn Ala Ala His Pro Ala Gln Phe Thr Ala Gly Ala Ala
                465                 470                 475
```

```
Pro Gly Leu Val Ala Val Tyr Thr Pro Asp Thr Ala Gly Pro Val Arg
            480                 485                 490

Met Asn Gly Val Asn Ile Ser Gly Pro Glu Ser Asn Thr Ala Asn Leu
        495                 500                 505

Pro Gly Thr Tyr Gly Tyr Asn Tyr Val Tyr Pro Thr Glu Ala Asp Phe
    510                 515                 520

Asp Tyr Tyr Ala Ser Lys Gly Leu Asn Leu Ile Arg Ile Pro Phe Arg
525                 530                 535                 540

Trp Glu Arg Met Gln His Gly Leu Asn Val Pro Leu Asn Thr Ala Gln
                545                 550                 555

Leu Gly Tyr Met Asp Thr Ala Val Ala Arg Ala Ser Ala Arg Gly Met
            560                 565                 570

Lys Val Ile Leu Asp Met His Asn Tyr Ala Arg Cys Lys Val Gly Gly
        575                 580                 585

Val Thr Tyr Lys Phe Gly Asp Ala Gln Leu Pro Ala Ser Ala Tyr Ala
    590                 595                 600

Asp Val Trp Arg Arg Leu Ala Asp His Tyr Lys Asn Glu Pro Ala Ile
605                 610                 615                 620

Tyr Gly Phe Asp Ile Met Asn Glu Pro Asn Gly Leu Ser Gly Gly Val
                625                 630                 635

Trp Pro Ala Tyr Ala Gln Ala Ala Val Asn Ala Ile Arg Glu Val Asn
            640                 645                 650

Leu Ser Thr Trp Val Ile Val Glu Gly Glu Phe Trp Ala Asn Ala Trp
        655                 660                 665

Gly Phe Glu Thr Lys Asn Pro Tyr Leu His Asn Val Arg Asp Pro Val
    670                 675                 680

Gly Arg Leu Met Phe Ser Ala His Ser Tyr Trp Ser Asp Ala Gly Thr
685                 690                 695                 700

Asp Val Tyr Lys Thr Tyr Asp Glu Glu Gly Ala Tyr Pro Glu Met Gly
                705                 710                 715

Val Asn Asn Val Lys Pro Phe Ile Asp Trp Leu Lys Lys His Asp Ala
```

720 725 730

Lys Gly Phe Val Gly Glu Tyr Gly Val Pro Asn Asn Asp Pro Arg Trp
735 740 745

Leu Val Val Leu Asp Asn Phe Leu Ala Tyr Leu Ala Ala Glu Gly Val
750 755 760

Ser Gly Thr Tyr Trp Ala Gly Gly Ala Trp Tyr Ser Gly Ser Pro Ile
765 770 775 780

Ser Cys His Pro Ser Ser Asn Tyr Thr Val Asp Arg Ala Val Met Ser
785 790 795

Val Leu Glu Asp His Pro
800

<210> 3
<211> 2496
<212> DNA
<213> Unknown

<220>
<223> Environmental sample

<220>
<221> CDS
<222> (1)..(2493)

<220>
<221> sig_peptide
<222> (1)..(111)

<220>
<221> mat_peptide
<222> (112)..(2493)

<400> 3

```
atg aac acc aca cca caa ccc acc ccc gcc cgc cgg acg cct cga cgc        48
Met Asn Thr Thr Pro Gln Pro Thr Pro Ala Arg Arg Thr Pro Arg Arg
        -35                 -30                 -25

ccg ttc ctc gcc acc ctc gct acc atc ctc ggc ctc gcc gcc tcc gtc        96
Pro Phe Leu Ala Thr Leu Ala Thr Ile Leu Gly Leu Ala Ala Ser Val
    -20                 -15                 -10

tcc tcc gtc tcc gcc gcc gac tgg tat ctc gat aaa aac cag gcc cgc       144
Ser Ser Val Ser Ala Ala Asp Trp Tyr Leu Asp Lys Asn Gln Ala Arg
-5              -1  1               5                   10

tac gcc agc tgg gac acc ctc gcc gac tgg aaa ccc aac ccc gac ggc       192
Tyr Ala Ser Trp Asp Thr Leu Ala Asp Trp Lys Pro Asn Pro Asp Gly
            15                  20                  25

agc ggc tcc aac ccc tcc gcc ctc tcc ccc tcc gac acc tac cac ctc       240
Ser Gly Ser Asn Pro Ser Ala Leu Ser Pro Ser Asp Thr Tyr His Leu
```

```
            30                  35                  40

aac ggc ttc atg ctc cgc acc ccc gag ggc ggc tcc acc tac acc ttc      288
Asn Gly Phe Met Leu Arg Thr Pro Glu Gly Gly Ser Thr Tyr Thr Phe
    45                  50                  55

acc ggc ggc ctc ctc agc ctc gcc aac aac gcc gac aac ttc gcc ctc      336
Thr Gly Gly Leu Leu Ser Leu Ala Asn Asn Ala Asp Asn Phe Ala Leu
60                  65                  70                  75

aag acc acc ggc tcc ggc gtc tcc atc atc ccc gcc ctg cgc acc acc      384
Lys Thr Thr Gly Ser Gly Val Ser Ile Ile Pro Ala Leu Arg Thr Thr
                80                  85                  90

gcc ggc ctc gtc caa aac gtc ggc tcc ggc acg caa aac ctc cag gtt      432
Ala Gly Leu Val Gln Asn Val Gly Ser Gly Thr Gln Asn Leu Gln Val
            95                  100                 105

ggc cac tac caa aac ctc tcc ggc acg acc tcc tac tac gcc cag acc      480
Gly His Tyr Gln Asn Leu Ser Gly Thr Thr Ser Tyr Tyr Ala Gln Thr
        110                 115                 120

ggg cgc ggc ctc aac ctc gcc atc acc acc ctc gtg ggc tcc ggc cag      528
Gly Arg Gly Leu Asn Leu Ala Ile Thr Thr Leu Val Gly Ser Gly Gln
    125                 130                 135

ttc cgc ttc tac ggc ggc ggc acc tac tac ctc tcc ctc gcc aac tcc      576
Phe Arg Phe Tyr Gly Gly Gly Thr Tyr Tyr Leu Ser Leu Ala Asn Ser
140                 145                 150                 155

ccg acc tac gac ggc gac atc tac gtc caa tcc ggc acc atc gat ttc      624
Pro Thr Tyr Asp Gly Asp Ile Tyr Val Gln Ser Gly Thr Ile Asp Phe
                160                 165                 170

aac aac gac ctc gcc acc gcc ggc act ctc acc gtc aac acc ggt gcc      672
Asn Asn Asp Leu Ala Thr Ala Gly Thr Leu Thr Val Asn Thr Gly Ala
            175                 180                 185

aag gtc gcc ctc gac cag gcc gtc acc ttc acc ggc ctc acc ata gcc      720
Lys Val Ala Leu Asp Gln Ala Val Thr Phe Thr Gly Leu Thr Ile Ala
        190                 195                 200

ggc aca gcg tat cca gtt gga aac tac agc tac gcc gcg ctt cag gcc      768
Gly Thr Ala Tyr Pro Val Gly Asn Tyr Ser Tyr Ala Ala Leu Gln Ala
    205                 210                 215

gcc cac ccc gcc gtt ttc gtc tcc ggc acc tcc ggc gga gcc atc aac      816
Ala His Pro Ala Val Phe Val Ser Gly Thr Ser Gly Gly Ala Ile Asn
220                 225                 230                 235

gtc cgc gcc ccg cgc aac tgg tat ctc tcc acc cac caa ccc gtc ggc      864
Val Arg Ala Pro Arg Asn Trp Tyr Leu Ser Thr His Gln Pro Val Gly
                240                 245                 250

gcc agc tgg aac acc ctc gcc cat tgg cgc gcc aac ccc gac ggc acc      912
Ala Ser Trp Asn Thr Leu Ala His Trp Arg Ala Asn Pro Asp Gly Thr
            255                 260                 265

ggc gcc acc gcc gac tcc atc aac tcc ttc gac aac tac atc aac caa      960
Gly Ala Thr Ala Asp Ser Ile Asn Ser Phe Asp Asn Tyr Ile Asn Gln
        270                 275                 280

gtc tcc ggc cgc acc ctg cgc acc ccc gaa acc acc gcc acc ttc gcc     1008
```

```
Val Ser Gly Arg Thr Leu Arg Thr Pro Glu Thr Thr Ala Thr Phe Ala
    285                 290                 295

ggc ggt tcc ctc gtc ctc gcc gac ggc ggc aac ctc tcg ctc aag gcc      1056
Gly Gly Ser Leu Val Leu Ala Asp Gly Gly Asn Leu Ser Leu Lys Ala
300                 305                 310                 315

ccc gcc ggc cac tcc agc acc atc ccc gcc ttc gcc aca tcg gga tcg      1104
Pro Ala Gly His Ser Ser Thr Ile Pro Ala Phe Ala Thr Ser Gly Ser
                320                 325                 330

att tcc atc acc aac ggc ttc agc agc atc acc cag ccc ctc gtc atc      1152
Ile Ser Ile Thr Asn Gly Phe Ser Ser Ile Thr Gln Pro Leu Val Ile
            335                 340                 345

ggc gac tgg cac ctc ggc gcc ggc acc gcc caa gtc tcc gtg cca agc      1200
Gly Asp Trp His Leu Gly Ala Gly Thr Ala Gln Val Ser Val Pro Ser
        350                 355                 360

acc agc acc gtg cag ctc acc gtc gat aaa ctc tcc ggc gac ggc acc      1248
Thr Ser Thr Val Gln Leu Thr Val Asp Lys Leu Ser Gly Asp Gly Thr
    365                 370                 375

ctc cag ttc cag aac ggc ggc aaa tac acc ctc aac atc cgc ggc gcg      1296
Leu Gln Phe Gln Asn Gly Gly Lys Tyr Thr Leu Asn Ile Arg Gly Ala
380                 385                 390                 395

tcc gcc ttc acc ggc acc ctc cgc cac ctc tcc ggc acg ctc acc gta      1344
Ser Ala Phe Thr Gly Thr Leu Arg His Leu Ser Gly Thr Leu Thr Val
                400                 405                 410

gcc tcc cag atc ggc acc ggc ggc acc ctc gtc gtc gaa tcc acc ggc      1392
Ala Ser Gln Ile Gly Thr Gly Gly Thr Leu Val Val Glu Ser Thr Gly
            415                 420                 425

gcg gtg aaa ctc gac cac ccc ggc ttc ttc acc ggc gtc acc gtc gcc      1440
Ala Val Lys Leu Asp His Pro Gly Phe Phe Thr Gly Val Thr Val Ala
        430                 435                 440

ggc acg ccc ctc gcc ccc ggc tac cac acc tac gcc gcg ctc aaa gcc      1488
Gly Thr Pro Leu Ala Pro Gly Tyr His Thr Tyr Ala Ala Leu Lys Ala
    445                 450                 455

gcc cac ccc gcg cgc ttc ccc acc ggc tcc acc aac gcc ttc ctc gcc      1536
Ala His Pro Ala Arg Phe Pro Thr Gly Ser Thr Asn Ala Phe Leu Ala
460                 465                 470                 475

gtc tat ccg ccc gac acc acc ggc ccc gcc cac atg ttc ggc gtc aac      1584
Val Tyr Pro Pro Asp Thr Thr Gly Pro Ala His Met Phe Gly Val Asn
                480                 485                 490

ctc gcc ggc ggc gaa ttc ggc acc ccg atg ccc ggc gtt tac ggc acc      1632
Leu Ala Gly Gly Glu Phe Gly Thr Pro Met Pro Gly Val Tyr Gly Thr
            495                 500                 505

gac tac atc tac ccg agc gcc gcc gcc ttc gat tac tac cac ggc aaa      1680
Asp Tyr Ile Tyr Pro Ser Ala Ala Ala Phe Asp Tyr Tyr His Gly Lys
        510                 515                 520

ggc ctc aaa ctc atc cgc ctc ccc ttt aag tgg gaa cgc ctc cag cac      1728
Gly Leu Lys Leu Ile Arg Leu Pro Phe Lys Trp Glu Arg Leu Gln His
    525                 530                 535
```

```
acc ctc aac gcc ccc ctc aac gcc gcc gag ctc gcc cgc atc gac acc        1776
Thr Leu Asn Ala Pro Leu Asn Ala Ala Glu Leu Ala Arg Ile Asp Thr
540                 545                 550                 555

gtc gtc ggc tac gcc tcc gcg cgc ggc atg aag gtc gtc ctc gac atg        1824
Val Val Gly Tyr Ala Ser Ala Arg Gly Met Lys Val Val Leu Asp Met
                560                 565                 570

cac aac tac gcc cgc cgc aaa gaa agc ggc acc acc tac ctc atc ggc        1872
His Asn Tyr Ala Arg Arg Lys Glu Ser Gly Thr Thr Tyr Leu Ile Gly
            575                 580                 585

acc ggc ccc gtc acc atg gac gcc ttc ggc gac gtc tgg cgt cgc atc        1920
Thr Gly Pro Val Thr Met Asp Ala Phe Gly Asp Val Trp Arg Arg Ile
        590                 595                 600

gcc gat cac tac aag ggc aac ccc gcc atc tac ggc tac ggc atc atg        1968
Ala Asp His Tyr Lys Gly Asn Pro Ala Ile Tyr Gly Tyr Gly Ile Met
    605                 610                 615

aac gag ccc tac tcc acc aac acc acc tgg ccc cag atg gcc cag acc        2016
Asn Glu Pro Tyr Ser Thr Asn Thr Thr Trp Pro Gln Met Ala Gln Thr
620                 625                 630                 635

gcc gtc aac gcc atc cgc acc gtt gac ctc acc acc cac gtc atc gtc        2064
Ala Val Asn Ala Ile Arg Thr Val Asp Leu Thr Thr His Val Ile Val
                640                 645                 650

gcc ggc gac ggc tgg tcc aac gcc acc ggc tgg cgc tcc aag aac ccc        2112
Ala Gly Asp Gly Trp Ser Asn Ala Thr Gly Trp Arg Ser Lys Asn Pro
            655                 660                 665

aac ctc gac acc cag gac ccc gtc ggc cgc ctc atc tac gaa gcc cac        2160
Asn Leu Asp Thr Gln Asp Pro Val Gly Arg Leu Ile Tyr Glu Ala His
        670                 675                 680

tgc tac ttc gat tcc aac ctc tcc ggc acc tac acc caa agc tac gat        2208
Cys Tyr Phe Asp Ser Asn Leu Ser Gly Thr Tyr Thr Gln Ser Tyr Asp
    685                 690                 695

gcc gcc ggc gcc cac ccc atg atc ggc gtg gac cgc gtg cgc gaa ttc        2256
Ala Ala Gly Ala His Pro Met Ile Gly Val Asp Arg Val Arg Glu Phe
700                 705                 710                 715

gtc gag tgg ctt cag gaa acc ggc aac aaa ggc ttc atc ggc gaa tac        2304
Val Glu Trp Leu Gln Glu Thr Gly Asn Lys Gly Phe Ile Gly Glu Tyr
                720                 725                 730

ggc gtc ccc ggc aac gac ccc cgc tgg ctc gtc gtg ctc gac aac ttc        2352
Gly Val Pro Gly Asn Asp Pro Arg Trp Leu Val Val Leu Asp Asn Phe
            735                 740                 745

ctc gcc tac ctc gac gcc aac ggc gtc tcc ggc acc tac tgg gcc ggc        2400
Leu Ala Tyr Leu Asp Ala Asn Gly Val Ser Gly Thr Tyr Trp Ala Gly
        750                 755                 760

ggt cct tgg tgg ggc aac tac ccg ctc agc tgc gaa ccc acc tcc aac        2448
Gly Pro Trp Trp Gly Asn Tyr Pro Leu Ser Cys Glu Pro Thr Ser Asn
    765                 770                 775

tac acc gtg gac aaa ccc cag atg agc gtc ctc gaa aac tac aac tga        2496
Tyr Thr Val Asp Lys Pro Gln Met Ser Val Leu Glu Asn Tyr Asn
780                 785                 790
```

<210> 4
<211> 831
<212> PRT
<213> Unknown

<220>
<223> Synthetic Construct

<400> 4

```
Met Asn Thr Thr Pro Gln Pro Thr Pro Ala Arg Arg Thr Pro Arg Arg
        -35                 -30                 -25

Pro Phe Leu Ala Thr Leu Ala Thr Ile Leu Gly Leu Ala Ala Ser Val
    -20                 -15                 -10

Ser Ser Val Ser Ala Ala Asp Trp Tyr Leu Asp Lys Asn Gln Ala Arg
-5              -1  1               5                   10

Tyr Ala Ser Trp Asp Thr Leu Ala Asp Trp Lys Pro Asn Pro Asp Gly
            15                  20                  25

Ser Gly Ser Asn Pro Ser Ala Leu Ser Pro Ser Asp Thr Tyr His Leu
        30                  35                  40

Asn Gly Phe Met Leu Arg Thr Pro Glu Gly Gly Ser Thr Tyr Thr Phe
    45                  50                  55

Thr Gly Gly Leu Leu Ser Leu Ala Asn Asn Ala Asp Asn Phe Ala Leu
60                  65                  70                  75

Lys Thr Thr Gly Ser Gly Val Ser Ile Ile Pro Ala Leu Arg Thr Thr
                80                  85                  90

Ala Gly Leu Val Gln Asn Val Gly Ser Gly Thr Gln Asn Leu Gln Val
            95                  100                 105

Gly His Tyr Gln Asn Leu Ser Gly Thr Thr Ser Tyr Tyr Ala Gln Thr
        110                 115                 120

Gly Arg Gly Leu Asn Leu Ala Ile Thr Thr Leu Val Gly Ser Gly Gln
    125                 130                 135

Phe Arg Phe Tyr Gly Gly Gly Thr Tyr Tyr Leu Ser Leu Ala Asn Ser
140                 145                 150                 155

Pro Thr Tyr Asp Gly Asp Ile Tyr Val Gln Ser Gly Thr Ile Asp Phe
                160                 165                 170
```

```
Asn Asn Asp Leu Ala Thr Ala Gly Thr Leu Thr Val Asn Thr Gly Ala
            175                 180                 185

Lys Val Ala Leu Asp Gln Ala Val Thr Phe Thr Gly Leu Thr Ile Ala
        190                 195                 200

Gly Thr Ala Tyr Pro Val Gly Asn Tyr Ser Tyr Ala Ala Leu Gln Ala
    205                 210                 215

Ala His Pro Ala Val Phe Val Ser Gly Thr Ser Gly Gly Ala Ile Asn
220                 225                 230                 235

Val Arg Ala Pro Arg Asn Trp Tyr Leu Ser Thr His Gln Pro Val Gly
                240                 245                 250

Ala Ser Trp Asn Thr Leu Ala His Trp Arg Ala Asn Pro Asp Gly Thr
            255                 260                 265

Gly Ala Thr Ala Asp Ser Ile Asn Ser Phe Asp Asn Tyr Ile Asn Gln
        270                 275                 280

Val Ser Gly Arg Thr Leu Arg Thr Pro Glu Thr Thr Ala Thr Phe Ala
    285                 290                 295

Gly Gly Ser Leu Val Leu Ala Asp Gly Gly Asn Leu Ser Leu Lys Ala
300                 305                 310                 315

Pro Ala Gly His Ser Ser Thr Ile Pro Ala Phe Ala Thr Ser Gly Ser
                320                 325                 330

Ile Ser Ile Thr Asn Gly Phe Ser Ser Ile Thr Gln Pro Leu Val Ile
            335                 340                 345

Gly Asp Trp His Leu Gly Ala Gly Thr Ala Gln Val Ser Val Pro Ser
        350                 355                 360

Thr Ser Thr Val Gln Leu Thr Val Asp Lys Leu Ser Gly Asp Gly Thr
    365                 370                 375

Leu Gln Phe Gln Asn Gly Gly Lys Tyr Thr Leu Asn Ile Arg Gly Ala
380                 385                 390                 395

Ser Ala Phe Thr Gly Thr Leu Arg His Leu Ser Gly Thr Leu Thr Val
                400                 405                 410

Ala Ser Gln Ile Gly Thr Gly Gly Thr Leu Val Val Glu Ser Thr Gly
            415                 420                 425
```

Ala Val Lys Leu Asp His Pro Gly Phe Phe Thr Gly Val Thr Val Ala
430 435 440

Gly Thr Pro Leu Ala Pro Gly Tyr His Thr Tyr Ala Ala Leu Lys Ala
445 450 455

Ala His Pro Ala Arg Phe Pro Thr Gly Ser Thr Asn Ala Phe Leu Ala
460 465 470 475

Val Tyr Pro Pro Asp Thr Thr Gly Pro Ala His Met Phe Gly Val Asn
480 485 490

Leu Ala Gly Gly Glu Phe Gly Thr Pro Met Pro Gly Val Tyr Gly Thr
495 500 505

Asp Tyr Ile Tyr Pro Ser Ala Ala Ala Phe Asp Tyr Tyr His Gly Lys
510 515 520

Gly Leu Lys Leu Ile Arg Leu Pro Phe Lys Trp Glu Arg Leu Gln His
525 530 535

Thr Leu Asn Ala Pro Leu Asn Ala Ala Glu Leu Ala Arg Ile Asp Thr
540 545 550 555

Val Val Gly Tyr Ala Ser Ala Arg Gly Met Lys Val Val Leu Asp Met
560 565 570

His Asn Tyr Ala Arg Arg Lys Glu Ser Gly Thr Thr Tyr Leu Ile Gly
575 580 585

Thr Gly Pro Val Thr Met Asp Ala Phe Gly Asp Val Trp Arg Arg Ile
590 595 600

Ala Asp His Tyr Lys Gly Asn Pro Ala Ile Tyr Gly Tyr Gly Ile Met
605 610 615

Asn Glu Pro Tyr Ser Thr Asn Thr Thr Trp Pro Gln Met Ala Gln Thr
620 625 630 635

Ala Val Asn Ala Ile Arg Thr Val Asp Leu Thr Thr His Val Ile Val
640 645 650

Ala Gly Asp Gly Trp Ser Asn Ala Thr Gly Trp Arg Ser Lys Asn Pro
655 660 665

Asn Leu Asp Thr Gln Asp Pro Val Gly Arg Leu Ile Tyr Glu Ala His

```
            670                 675                 680

Cys Tyr Phe Asp Ser Asn Leu Ser Gly Thr Tyr Thr Gln Ser Tyr Asp
    685                 690                 695

Ala Ala Gly Ala His Pro Met Ile Gly Val Asp Arg Val Arg Glu Phe
700                 705                 710                 715

Val Glu Trp Leu Gln Glu Thr Gly Asn Lys Gly Phe Ile Gly Glu Tyr
                720                 725                 730

Gly Val Pro Gly Asn Asp Pro Arg Trp Leu Val Val Leu Asp Asn Phe
            735                 740                 745

Leu Ala Tyr Leu Asp Ala Asn Gly Val Ser Gly Thr Tyr Trp Ala Gly
        750                 755                 760

Gly Pro Trp Trp Gly Asn Tyr Pro Leu Ser Cys Glu Pro Thr Ser Asn
    765                 770                 775

Tyr Thr Val Asp Lys Pro Gln Met Ser Val Leu Glu Asn Tyr Asn
780                 785                 790
```

<210> 5
<211> 2508
<212> DNA
<213> Unknown

<220>
<223> Environmental sample

<220>
<221> CDS
<222> (1)..(2505)

<220>
<221> sig_peptide
<222> (1)..(105)

<220>
<221> mat_peptide
<222> (106)..(2505)

<400> 5

```
atg aaa cac cac cac acc aca cca cac acc ccg cgt cgg acc ctg ctc      48
Met Lys His His His Thr Thr Pro His Thr Pro Arg Arg Thr Leu Leu
-35                 -30                 -25                 -20

cgc tcg ctt gcc ggc ctg ctg gct ctc gcc acc ggc ctc gcc tcc acc      96
Arg Ser Leu Ala Gly Leu Leu Ala Leu Ala Thr Gly Leu Ala Ser Thr
                -15                 -10                 -5

gcc cac gcc gcc gac tac tac ctc aaa gtc aac caa ccc cac ccc aac     144
Ala His Ala Ala Asp Tyr Tyr Leu Lys Val Asn Gln Pro His Pro Asn
```

```
        -1  1               5                   10

agc tgg gcc tca ccc gtc acc gat tgg gcc gcc aac ccc gac ggc acc      192
Ser Trp Ala Ser Pro Val Thr Asp Trp Ala Ala Asn Pro Asp Gly Thr
    15                  20                  25

gga gcc gct ccc gcc gcc atc gcc gcg ccc gac acc ttt tac acc aac      240
Gly Ala Ala Pro Ala Ala Ile Ala Ala Pro Asp Thr Phe Tyr Thr Asn
30                  35                  40                  45

aac cgc acg ctc cgc acc ccc gcc gtc ggc gtc aac gcc acc ttc ccc      288
Asn Arg Thr Leu Arg Thr Pro Ala Val Gly Val Asn Ala Thr Phe Pro
                50                  55                  60

ggc ggc gtc ctc ggc cta aac ggc ggc gtc atc ggc ata aaa acc ggc      336
Gly Gly Val Leu Gly Leu Asn Gly Gly Val Ile Gly Ile Lys Thr Gly
            65                  70                  75

ccc tcc gcc ttc tcc atc gcc ccc aag ctc gtc tcc acc gcc ggc gcc      384
Pro Ser Ala Phe Ser Ile Ala Pro Lys Leu Val Ser Thr Ala Gly Ala
        80                  85                  90

atc gag tcc tgg ggc aca ccc caa aac ttc cgc gcc gac gac tgg gag      432
Ile Glu Ser Trp Gly Thr Pro Gln Asn Phe Arg Ala Asp Asp Trp Glu
    95                  100                 105

agc aac gcc ccc ttc ccc acc ttc acc gga ctg agg acc gcc tcc aac      480
Ser Asn Ala Pro Phe Pro Thr Phe Thr Gly Leu Arg Thr Ala Ser Asn
110                 115                 120                 125

cat acg ctc aag gtc tcc gtc ggc aaa ctc tcc ggc acc ggc gaa atc      528
His Thr Leu Lys Val Ser Val Gly Lys Leu Ser Gly Thr Gly Glu Ile
                130                 135                 140

cgc gtc cac ggc ggc ggc acc gtc ctc ctc gac gtc acc gac gcc gaa      576
Arg Val His Gly Gly Gly Thr Val Leu Leu Asp Val Thr Asp Ala Glu
            145                 150                 155

aac tac ctc ggc acc ctc tgc gtc gcc tcc ggc gcg ttg aac ttc gac      624
Asn Tyr Leu Gly Thr Leu Cys Val Ala Ser Gly Ala Leu Asn Phe Asp
        160                 165                 170

aac gcc gtc ttc tcc tcc ggc ccc ctc gac atc aag acc ggc gcc acc      672
Asn Ala Val Phe Ser Ser Gly Pro Leu Asp Ile Lys Thr Gly Ala Thr
    175                 180                 185

gtc gtc ctc gac cag gcc gtc tcc ttc gcc ggc ctc gcc gtc gga gcc      720
Val Val Leu Asp Gln Ala Val Ser Phe Ala Gly Leu Ala Val Gly Ala
190                 195                 200                 205

acc gag tat cca ccc ggc aac tac acc ctc gcc gcc ctg caa gcc gcc      768
Thr Glu Tyr Pro Pro Gly Asn Tyr Thr Leu Ala Ala Leu Gln Ala Ala
                210                 215                 220

cac ccg ggc gtc ttc acc ggc acc gcc gcc ggc tcc atc acc gtc cgc      816
His Pro Gly Val Phe Thr Gly Thr Ala Ala Gly Ser Ile Thr Val Arg
            225                 230                 235

gcc ccg cgc acc tgg tat ctc acc gtc agc cag ggc tcc cag aac tgg      864
Ala Pro Arg Thr Trp Tyr Leu Thr Val Ser Gln Gly Ser Gln Asn Trp
        240                 245                 250

acc gag gcc ttc ctc tcc aac tgg aac tcc gcc gcc aac ggc tcc ggc      912
```

Thr Glu Ala Phe Leu Ser Asn Trp Asn Ser Ala Ala Asn Gly Ser Gly
255 260 265

gtc gcc ccg aac tac atc aac ggc cac gac atc tac ctc aac cag gtg 960
Val Ala Pro Asn Tyr Ile Asn Gly His Asp Ile Tyr Leu Asn Gln Val
270 275 280 285

aac aac cgc gag ctc cgc acg ccc tac acc gcc agc acc ttc acc ggc 1008
Asn Asn Arg Glu Leu Arg Thr Pro Tyr Thr Ala Ser Thr Phe Thr Gly
290 295 300

ggc acc ctc gcc ctc acc ttc ggc tcg aag ctc gtc gtc aag acc tca 1056
Gly Thr Leu Ala Leu Thr Phe Gly Ser Lys Leu Val Val Lys Thr Ser
305 310 315

ccc aac ctc gtc agc acc atc ccc gcc ctc gtc acc tcc ggc acc ccg 1104
Pro Asn Leu Val Ser Thr Ile Pro Ala Leu Val Thr Ser Gly Thr Pro
320 325 330

cag ttc gcc aac ggc agc ggc agc cgc caa aac ctc gcc atc ggc gac 1152
Gln Phe Ala Asn Gly Ser Gly Ser Arg Gln Asn Leu Ala Ile Gly Asp
335 340 345

tgg gac atc atc tcc ggc acc agc cgc ctc gtc gcc ggc tcc acc cgg 1200
Trp Asp Ile Ile Ser Gly Thr Ser Arg Leu Val Ala Gly Ser Thr Arg
350 355 360 365

tcc ctc ggc ttc gac atc ggc tgg ctc acc ggc gcg ggc aac ctc cag 1248
Ser Leu Gly Phe Asp Ile Gly Trp Leu Thr Gly Ala Gly Asn Leu Gln
370 375 380

acc gaa ggc ggc ggc tcg ttc ttc ctc cgc ctc atc gac ggc tcc ggc 1296
Thr Glu Gly Gly Gly Ser Phe Phe Leu Arg Leu Ile Asp Gly Ser Gly
385 390 395

tac acc ggc gcc atc aac cac aac tcc ggc gcc ctc cgc ttc gag tcc 1344
Tyr Thr Gly Ala Ile Asn His Asn Ser Gly Ala Leu Arg Phe Glu Ser
400 405 410

gtc ttc tcc acc gcc ggt gcc ctc aac atc ggc gcc tcc gcg acc gtc 1392
Val Phe Ser Thr Ala Gly Ala Leu Asn Ile Gly Ala Ser Ala Thr Val
415 420 425

cac ctc gac aag ccc gtc tat gtc agc ggc ctc tcc gtc gcc ggc gtc 1440
His Leu Asp Lys Pro Val Tyr Val Ser Gly Leu Ser Val Ala Gly Val
430 435 440 445

gcc aaa ccc gcc ggc atc cac acc tac gcc tcg ctg aac gcc gcg cat 1488
Ala Lys Pro Ala Gly Ile His Thr Tyr Ala Ser Leu Asn Ala Ala His
450 455 460

ccc gcg cag ttc aac gcc ggc gcc gcg ccc gga ctc gtc gcc gtt tac 1536
Pro Ala Gln Phe Asn Ala Gly Ala Ala Pro Gly Leu Val Ala Val Tyr
465 470 475

aca ccc aac act gcc gcc ccc gtc cgc atg aac ggc gtc aac ctc tcc 1584
Thr Pro Asn Thr Ala Ala Pro Val Arg Met Asn Gly Val Asn Leu Ser
480 485 490

ggc ccc gaa tcc gtc ggc ggc gcc ggc acg ccc ttt ccc ggc acc tac 1632
Gly Pro Glu Ser Val Gly Gly Ala Gly Thr Pro Phe Pro Gly Thr Tyr
495 500 505

```
ggc ttc cag tgg att tac ccc acc gtc gcc gac tac gac tac tac gcc       1680
Gly Phe Gln Trp Ile Tyr Pro Thr Val Ala Asp Tyr Asp Tyr Tyr Ala
510                 515                 520                 525

gcc aag ggc ctt aac ctc atc cgc atc cca ttc cgc tgg gaa cgc atg       1728
Ala Lys Gly Leu Asn Leu Ile Arg Ile Pro Phe Arg Trp Glu Arg Met
                530                 535                 540

caa ggc acc ctt aac ggt ccc ctc atc gcc gcc gaa ctc gct cgc atg       1776
Gln Gly Thr Leu Asn Gly Pro Leu Ile Ala Ala Glu Leu Ala Arg Met
            545                 550                 555

gac aac gcc atc gcc ctc gcc tcc gcg cgc ggc atg aag gtc atc ctc       1824
Asp Asn Ala Ile Ala Leu Ala Ser Ala Arg Gly Met Lys Val Ile Leu
        560                 565                 570

gat atg cat aac tac gcg cgc tac cgc acc ccg acc gcg agc tac gtg       1872
Asp Met His Asn Tyr Ala Arg Tyr Arg Thr Pro Thr Ala Ser Tyr Val
    575                 580                 585

ttt ggt gac gcc cag ctc ccc gcc tcc gcc ttc gcc gac gtc tgg cgc       1920
Phe Gly Asp Ala Gln Leu Pro Ala Ser Ala Phe Ala Asp Val Trp Arg
590                 595                 600                 605

aag ctc gcc gat cac tac aaa aac gaa ccc gcc atc tac ggt ttc gac       1968
Lys Leu Ala Asp His Tyr Lys Asn Glu Pro Ala Ile Tyr Gly Phe Asp
                610                 615                 620

atc atg aac gag ccg cac agc atg ccc acc ccc acc acc tgg ccc acc       2016
Ile Met Asn Glu Pro His Ser Met Pro Thr Pro Thr Thr Trp Pro Thr
            625                 630                 635

tac gcc caa gcc gcc gtc cac gcc atc cgc gag gtc aac ctc gac acc       2064
Tyr Ala Gln Ala Ala Val His Ala Ile Arg Glu Val Asn Leu Asp Thr
        640                 645                 650

tgg atc atc gta gag ggc gag acc tat gcc aac tcc tgg aaa ttc ggg       2112
Trp Ile Ile Val Glu Gly Glu Thr Tyr Ala Asn Ser Trp Lys Phe Gly
    655                 660                 665

gaa aaa aat ccc cac ctc cac aac gtg cgc gac ccc gtc ggc cgc ctc       2160
Glu Lys Asn Pro His Leu His Asn Val Arg Asp Pro Val Gly Arg Leu
670                 675                 680                 685

atg ttc tcc gcc cac tcc tac tgg tgc aaa aac ggc gac gac aga tac       2208
Met Phe Ser Ala His Ser Tyr Trp Cys Lys Asn Gly Asp Asp Arg Tyr
                690                 695                 700

ggc acc tac gac gcg gaa aac ggc cac ccc cag atg ggc gtg gac agc       2256
Gly Thr Tyr Asp Ala Glu Asn Gly His Pro Gln Met Gly Val Asp Ser
            705                 710                 715

ctc aag cac ttc gtt gac tgg ctc cgc aaa cac aac gcc cac ggc ttc       2304
Leu Lys His Phe Val Asp Trp Leu Arg Lys His Asn Ala His Gly Phe
        720                 725                 730

gtc ggc gaa tac ggc gtc ccc aac aac gac ccc cgc tgg ctc gaa gtc       2352
Val Gly Glu Tyr Gly Val Pro Asn Asn Asp Pro Arg Trp Leu Glu Val
    735                 740                 745

ctt gaa aac gcg ctc atc tac ctg gcg aat gaa aac atc agc ggc acc       2400
Leu Glu Asn Ala Leu Ile Tyr Leu Ala Asn Glu Asn Ile Ser Gly Thr
750                 755                 760                 765
```

```
tac tgg gcc ggc ggc gcc tgg ctc gcc ggc agc cac atc agc tgc cac       2448
Tyr Trp Ala Gly Gly Ala Trp Leu Ala Gly Ser His Ile Ser Cys His
                770                 775                 780

ccg tcc tcc aac tac acc gtg gac cgc ccc gtc atg agc gtc ctc caa       2496
Pro Ser Ser Asn Tyr Thr Val Asp Arg Pro Val Met Ser Val Leu Gln
            785                 790                 795

aac tac ccg taa                                                       2508
Asn Tyr Pro
        800
```

<210> 6
<211> 835
<212> PRT
<213> Unknown

<220>
<223> Synthetic Construct

<400> 6

```
Met Lys His His His Thr Thr Pro His Thr Pro Arg Arg Thr Leu Leu
-35                 -30                 -25                 -20

Arg Ser Leu Ala Gly Leu Leu Ala Leu Ala Thr Gly Leu Ala Ser Thr
                -15                 -10                 -5

Ala His Ala Ala Asp Tyr Tyr Leu Lys Val Asn Gln Pro His Pro Asn
        -1  1               5                   10

Ser Trp Ala Ser Pro Val Thr Asp Trp Ala Ala Asn Pro Asp Gly Thr
    15                  20                  25

Gly Ala Ala Pro Ala Ala Ile Ala Ala Pro Asp Thr Phe Tyr Thr Asn
30                  35                  40                  45

Asn Arg Thr Leu Arg Thr Pro Ala Val Gly Val Asn Ala Thr Phe Pro
                50                  55                  60

Gly Gly Val Leu Gly Leu Asn Gly Gly Val Ile Gly Ile Lys Thr Gly
            65                  70                  75

Pro Ser Ala Phe Ser Ile Ala Pro Lys Leu Val Ser Thr Ala Gly Ala
        80                  85                  90

Ile Glu Ser Trp Gly Thr Pro Gln Asn Phe Arg Ala Asp Asp Trp Glu
    95                  100                 105

Ser Asn Ala Pro Phe Pro Thr Phe Thr Gly Leu Arg Thr Ala Ser Asn
110                 115                 120                 125
```

```
His Thr Leu Lys Val Ser Val Gly Lys Leu Ser Gly Thr Gly Glu Ile
                130                 135                 140

Arg Val His Gly Gly Gly Thr Val Leu Leu Asp Val Thr Asp Ala Glu
            145                 150                 155

Asn Tyr Leu Gly Thr Leu Cys Val Ala Ser Gly Ala Leu Asn Phe Asp
        160                 165                 170

Asn Ala Val Phe Ser Ser Gly Pro Leu Asp Ile Lys Thr Gly Ala Thr
    175                 180                 185

Val Val Leu Asp Gln Ala Val Ser Phe Ala Gly Leu Ala Val Gly Ala
190                 195                 200                 205

Thr Glu Tyr Pro Pro Gly Asn Tyr Thr Leu Ala Ala Leu Gln Ala Ala
                210                 215                 220

His Pro Gly Val Phe Thr Gly Thr Ala Ala Gly Ser Ile Thr Val Arg
            225                 230                 235

Ala Pro Arg Thr Trp Tyr Leu Thr Val Ser Gln Gly Ser Gln Asn Trp
        240                 245                 250

Thr Glu Ala Phe Leu Ser Asn Trp Asn Ser Ala Ala Asn Gly Ser Gly
    255                 260                 265

Val Ala Pro Asn Tyr Ile Asn Gly His Asp Ile Tyr Leu Asn Gln Val
270                 275                 280                 285

Asn Asn Arg Glu Leu Arg Thr Pro Tyr Thr Ala Ser Thr Phe Thr Gly
                290                 295                 300

Gly Thr Leu Ala Leu Thr Phe Gly Ser Lys Leu Val Val Lys Thr Ser
            305                 310                 315

Pro Asn Leu Val Ser Thr Ile Pro Ala Leu Val Thr Ser Gly Thr Pro
        320                 325                 330

Gln Phe Ala Asn Gly Ser Gly Ser Arg Gln Asn Leu Ala Ile Gly Asp
    335                 340                 345

Trp Asp Ile Ile Ser Gly Thr Ser Arg Leu Val Ala Gly Ser Thr Arg
350                 355                 360                 365

Ser Leu Gly Phe Asp Ile Gly Trp Leu Thr Gly Ala Gly Asn Leu Gln
                370                 375                 380
```

```
Thr Glu Gly Gly Gly Ser Phe Phe Leu Arg Leu Ile Asp Gly Ser Gly
            385                 390                 395

Tyr Thr Gly Ala Ile Asn His Asn Ser Gly Ala Leu Arg Phe Glu Ser
        400                 405                 410

Val Phe Ser Thr Ala Gly Ala Leu Asn Ile Gly Ala Ser Ala Thr Val
    415                 420                 425

His Leu Asp Lys Pro Val Tyr Val Ser Gly Leu Ser Val Ala Gly Val
430                 435                 440                 445

Ala Lys Pro Ala Gly Ile His Thr Tyr Ala Ser Leu Asn Ala Ala His
                450                 455                 460

Pro Ala Gln Phe Asn Ala Gly Ala Ala Pro Gly Leu Val Ala Val Tyr
            465                 470                 475

Thr Pro Asn Thr Ala Ala Pro Val Arg Met Asn Gly Val Asn Leu Ser
        480                 485                 490

Gly Pro Glu Ser Val Gly Gly Ala Gly Thr Pro Phe Pro Gly Thr Tyr
    495                 500                 505

Gly Phe Gln Trp Ile Tyr Pro Thr Val Ala Asp Tyr Asp Tyr Tyr Ala
510                 515                 520                 525

Ala Lys Gly Leu Asn Leu Ile Arg Ile Pro Phe Arg Trp Glu Arg Met
                530                 535                 540

Gln Gly Thr Leu Asn Gly Pro Leu Ile Ala Ala Glu Leu Ala Arg Met
            545                 550                 555

Asp Asn Ala Ile Ala Leu Ala Ser Ala Arg Gly Met Lys Val Ile Leu
        560                 565                 570

Asp Met His Asn Tyr Ala Arg Tyr Arg Thr Pro Thr Ala Ser Tyr Val
    575                 580                 585

Phe Gly Asp Ala Gln Leu Pro Ala Ser Ala Phe Ala Asp Val Trp Arg
590                 595                 600                 605

Lys Leu Ala Asp His Tyr Lys Asn Glu Pro Ala Ile Tyr Gly Phe Asp
                610                 615                 620

Ile Met Asn Glu Pro His Ser Met Pro Thr Pro Thr Thr Trp Pro Thr
```

```
            625                 630                 635

Tyr Ala Gln Ala Ala Val His Ala Ile Arg Glu Val Asn Leu Asp Thr
        640                 645                 650

Trp Ile Ile Val Glu Gly Glu Thr Tyr Ala Asn Ser Trp Lys Phe Gly
    655                 660                 665

Glu Lys Asn Pro His Leu His Asn Val Arg Asp Pro Val Gly Arg Leu
670                 675                 680                 685

Met Phe Ser Ala His Ser Tyr Trp Cys Lys Asn Gly Asp Asp Arg Tyr
                690                 695                 700

Gly Thr Tyr Asp Ala Glu Asn Gly His Pro Gln Met Gly Val Asp Ser
            705                 710                 715

Leu Lys His Phe Val Asp Trp Leu Arg Lys His Asn Ala His Gly Phe
        720                 725                 730

Val Gly Glu Tyr Gly Val Pro Asn Asn Asp Pro Arg Trp Leu Glu Val
    735                 740                 745

Leu Glu Asn Ala Leu Ile Tyr Leu Ala Asn Glu Asn Ile Ser Gly Thr
750                 755                 760                 765

Tyr Trp Ala Gly Gly Ala Trp Leu Ala Gly Ser His Ile Ser Cys His
                770                 775                 780

Pro Ser Ser Asn Tyr Thr Val Asp Arg Pro Val Met Ser Val Leu Gln
            785                 790                 795

Asn Tyr Pro
        800
```

<210> 7
<211> 2082
<212> DNA
<213> Pseudomonas stutzeri

<220>
<221> CDS
<222> (1)..(2079)

<220>
<221> sig_peptide
<222> (1)..(108)

<220>

<221> mat_peptide
<222> (109)..(2079)

<400> 7

```
atg tcc acc aac ctg ttt tcc ggt gcc cgc aag gca ctc gtc gct tcc        48
Met Ser Thr Asn Leu Phe Ser Gly Ala Arg Lys Ala Leu Val Ala Ser
    -35                 -30                 -25

atc gct gcc gct gtt ctg ctg ggt ggc gcc act gtt gta acc acg cct        96
Ile Ala Ala Ala Val Leu Leu Gly Gly Ala Thr Val Val Thr Thr Pro
-20                 -15                 -10                 -5

tat gcc gct gca tcc tcg gtt gcc gct gta tcg gtt tcc gcc aag atc       144
Tyr Ala Ala Ala Ser Ser Val Ala Ala Val Ser Val Ser Ala Lys Ile
            -1  1               5                   10

aac gcg ttc acc aac agc gat tgg ctg aac ggt atc tgg cgc acc ggc       192
Asn Ala Phe Thr Asn Ser Asp Trp Leu Asn Gly Ile Trp Arg Thr Gly
        15                  20                  25

gcc ggc ttc tcg atc ccc gcc acc tcc gca aac cgc gcc gcg ttc gtg       240
Ala Gly Phe Ser Ile Pro Ala Thr Ser Ala Asn Arg Ala Ala Phe Val
    30                  35                  40

gcc ggc gct tcg gta cga ctg gca gac ggt cag gta cgc aag atc agc       288
Ala Gly Ala Ser Val Arg Leu Ala Asp Gly Gln Val Arg Lys Ile Ser
45                  50                  55                  60

cgc gcg caa atc gtc ggc agc aac atg agc atc ttc ctg gaa ggt gca       336
Arg Ala Gln Ile Val Gly Ser Asn Met Ser Ile Phe Leu Glu Gly Ala
                65                  70                  75

aag ctg gac ggc aac aag gtt ggc gca ccg caa gtg gtc acc atc ggc       384
Lys Leu Asp Gly Asn Lys Val Gly Ala Pro Gln Val Val Thr Ile Gly
            80                  85                  90

agc acg gcc gta acg gcc ccg gac act tct gct ccg atc act aca ccg       432
Ser Thr Ala Val Thr Ala Pro Asp Thr Ser Ala Pro Ile Thr Thr Pro
        95                  100                 105

cct acc gtt act gcg cac tcg acc agc atc aac gca ttc acc aac aat       480
Pro Thr Val Thr Ala His Ser Thr Ser Ile Asn Ala Phe Thr Asn Asn
    110                 115                 120

gat tgg ctc aat ggt gta tgg cgt aag tcg ccg ggc ttc tcc att ccg       528
Asp Trp Leu Asn Gly Val Trp Arg Lys Ser Pro Gly Phe Ser Ile Pro
125                 130                 135                 140

gca agc gct gcc aac aag gct gct ttc aaa gtt gga gcg aca gca aaa       576
Ala Ser Ala Ala Asn Lys Ala Ala Phe Lys Val Gly Ala Thr Ala Lys
                145                 150                 155

ctg gca gat ggc cag gtt cgc aaa att acc cag gta caa gtt gtt ggc       624
Leu Ala Asp Gly Gln Val Arg Lys Ile Thr Gln Val Gln Val Val Gly
            160                 165                 170

gcc aat atg agc gtc tat ctg gaa ggt gcg gca gtt aac gga agt gtc       672
Ala Asn Met Ser Val Tyr Leu Glu Gly Ala Ala Val Asn Gly Ser Val
        175                 180                 185

gtc ggc gca ccc aac aag ttg gcg ctg gct aca act tcg act acc agc       720
Val Gly Ala Pro Asn Lys Leu Ala Leu Ala Thr Thr Ser Thr Thr Ser
    190                 195                 200
```

```
ccg gct ccg act ccg gcg ccc agt gct ccg acc cct tcg gtc atc gcc        768
Pro Ala Pro Thr Pro Ala Pro Ser Ala Pro Thr Pro Ser Val Ile Ala
205                 210                 215                 220

acc agc aac ctg aac aac tac acc aat gct caa tgg ctc aac ggt atg        816
Thr Ser Asn Leu Asn Asn Tyr Thr Asn Ala Gln Trp Leu Asn Gly Met
                225                 230                 235

tac cgt acc gct gca ggc ttc tcc atc cag gca agc agc gcc aac gtg        864
Tyr Arg Thr Ala Ala Gly Phe Ser Ile Gln Ala Ser Ser Ala Asn Val
            240                 245                 250

gcg gca ttc aag gct ggc gct ttg gtg agg ctc gct gat ggt cag acc        912
Ala Ala Phe Lys Ala Gly Ala Leu Val Arg Leu Ala Asp Gly Gln Thr
        255                 260                 265

cgc aag gtg ctg cgc gct cag ctg gtc ggc agc aac atg agc gtc ttt        960
Arg Lys Val Leu Arg Ala Gln Leu Val Gly Ser Asn Met Ser Val Phe
    270                 275                 280

ctt gac ggc gcg gta atc aac ggt acg acc ctg ggc tat ccg aag acc       1008
Leu Asp Gly Ala Val Ile Asn Gly Thr Thr Leu Gly Tyr Pro Lys Thr
285                 290                 295                 300

atc tcg gtg gtc agt acg tcg acc ggc act cct tcg tct cct gct ctg       1056
Ile Ser Val Val Ser Thr Ser Thr Gly Thr Pro Ser Ser Pro Ala Leu
                305                 310                 315

act acc cca ccg gta gag cca gca ccg gct ccg gtg ccc acc gca cct       1104
Thr Thr Pro Pro Val Glu Pro Ala Pro Ala Pro Val Pro Thr Ala Pro
            320                 325                 330

gac acc acc aat ggc aag ccg ctg ctg gtt ggc gtc aat ctg tcc ggc       1152
Asp Thr Thr Asn Gly Lys Pro Leu Leu Val Gly Val Asn Leu Ser Gly
        335                 340                 345

gcc ggc ttc ggt ccc tcg gtt gtt ccc ggc aag cat ggc acc aac tac       1200
Ala Gly Phe Gly Pro Ser Val Val Pro Gly Lys His Gly Thr Asn Tyr
    350                 355                 360

acc tat cct gcc gag tcg tac tac aag aag tat tcc gac ctg ggc atg       1248
Thr Tyr Pro Ala Glu Ser Tyr Tyr Lys Lys Tyr Ser Asp Leu Gly Met
365                 370                 375                 380

ccg ctg gtt cgc ctg ccg ttc ctc tgg gag cgt atc cag ccc aag ctg       1296
Pro Leu Val Arg Leu Pro Phe Leu Trp Glu Arg Ile Gln Pro Lys Leu
                385                 390                 395

aac tct ccg ctg aac gcc gag gag ttc gcc cgt ctg aag cag tcg ctg       1344
Asn Ser Pro Leu Asn Ala Glu Glu Phe Ala Arg Leu Lys Gln Ser Leu
            400                 405                 410

gat ttc gcg cag aag cac aac gtc aag gtg att ctc gac ctg cac aac       1392
Asp Phe Ala Gln Lys His Asn Val Lys Val Ile Leu Asp Leu His Asn
        415                 420                 425

tac tac cgt tat tac ggc aag ctg atc ggc tcc aaa gaa gtg ccc atc       1440
Tyr Tyr Arg Tyr Tyr Gly Lys Leu Ile Gly Ser Lys Glu Val Pro Ile
    430                 435                 440

agt tcc ttc gcc gcg gta tgg aag cag atc gtg cag caa gta gtg aac       1488
Ser Ser Phe Ala Ala Val Trp Lys Gln Ile Val Gln Gln Val Val Asn
445                 450                 455                 460
```

```
cac ccg gcc gtc gaa ggc tac ggc ctg atg aac gag ccg cac tcg acc        1536
His Pro Ala Val Glu Gly Tyr Gly Leu Met Asn Glu Pro His Ser Thr
                465                 470                 475

aac ggg ctc tgg ccg cag gct gcc ctg gcg gct gct cag gca atc cgc        1584
Asn Gly Leu Trp Pro Gln Ala Ala Leu Ala Ala Ala Gln Ala Ile Arg
            480                 485                 490

acc gtc gac tcc aag cgc tgg atc tac gta gca ggc gat cgc tgg tcg        1632
Thr Val Asp Ser Lys Arg Trp Ile Tyr Val Ala Gly Asp Arg Trp Ser
        495                 500                 505

agc gct ttc cac tgg ccg cac tac aac act cag ctg gtc acc aac ccg        1680
Ser Ala Phe His Trp Pro His Tyr Asn Thr Gln Leu Val Thr Asn Pro
    510                 515                 520

tgg atg cgc gat ccg aag aac aat ctg gtt tac gaa gcg cac atg tac        1728
Trp Met Arg Asp Pro Lys Asn Asn Leu Val Tyr Glu Ala His Met Tyr
525                 530                 535                 540

gtg gac aag gat ttc tcg ggc aac tac ttc gac aag gcc gag aag ttc        1776
Val Asp Lys Asp Phe Ser Gly Asn Tyr Phe Asp Lys Ala Glu Lys Phe
                545                 550                 555

gac ccg atg att ggc gtc aac cgc gtc aag ccc ttc gtc gac tgg ctc        1824
Asp Pro Met Ile Gly Val Asn Arg Val Lys Pro Phe Val Asp Trp Leu
            560                 565                 570

aag cag cac aaa ctg cgc ggc tac atc ggt gag cac ggc gta ccg gat        1872
Lys Gln His Lys Leu Arg Gly Tyr Ile Gly Glu His Gly Val Pro Asp
        575                 580                 585

ttc tcg ccc tcg gcc atc gtc gca acc gat aac ctg ctg gcc tac ctg        1920
Phe Ser Pro Ser Ala Ile Val Ala Thr Asp Asn Leu Leu Ala Tyr Leu
    590                 595                 600

cgt cag aac tgc atc ccg agc acc tat tgg gct gcc ggt ccc tgg tgg        1968
Arg Gln Asn Cys Ile Pro Ser Thr Tyr Trp Ala Ala Gly Pro Trp Trp
605                 610                 615                 620

ggc gag tac gcg atg tcc ctg gac gta agc agc ggc aag cac cgt ccg        2016
Gly Glu Tyr Ala Met Ser Leu Asp Val Ser Ser Gly Lys His Arg Pro
                625                 630                 635

cag ctg ccg gtt ctg cag aag cac gcc aaa acc gca aac agc tgc acc        2064
Gln Leu Pro Val Leu Gln Lys His Ala Lys Thr Ala Asn Ser Cys Thr
            640                 645                 650

agc atc ggt ccg ctg taa                                                2082
Ser Ile Gly Pro Leu
        655
```

<210> 8
<211> 693
<212> PRT
<213> Pseudomonas stutzeri

<400> 8

```
Met Ser Thr Asn Leu Phe Ser Gly Ala Arg Lys Ala Leu Val Ala Ser
    -35                 -30                 -25
```

```
Ile Ala Ala Ala Val Leu Leu Gly Gly Ala Thr Val Val Thr Thr Pro
-20                 -15                 -10                 -5

Tyr Ala Ala Ala Ser Ser Val Ala Ala Val Ser Val Ser Ala Lys Ile
            -1  1               5                   10

Asn Ala Phe Thr Asn Ser Asp Trp Leu Asn Gly Ile Trp Arg Thr Gly
        15                  20                  25

Ala Gly Phe Ser Ile Pro Ala Thr Ser Ala Asn Arg Ala Ala Phe Val
    30                  35                  40

Ala Gly Ala Ser Val Arg Leu Ala Asp Gly Gln Val Arg Lys Ile Ser
45                  50                  55                  60

Arg Ala Gln Ile Val Gly Ser Asn Met Ser Ile Phe Leu Glu Gly Ala
                65                  70                  75

Lys Leu Asp Gly Asn Lys Val Gly Ala Pro Gln Val Val Thr Ile Gly
            80                  85                  90

Ser Thr Ala Val Thr Ala Pro Asp Thr Ser Ala Pro Ile Thr Thr Pro
        95                  100                 105

Pro Thr Val Thr Ala His Ser Thr Ser Ile Asn Ala Phe Thr Asn Asn
    110                 115                 120

Asp Trp Leu Asn Gly Val Trp Arg Lys Ser Pro Gly Phe Ser Ile Pro
125                 130                 135                 140

Ala Ser Ala Ala Asn Lys Ala Ala Phe Lys Val Gly Ala Thr Ala Lys
                145                 150                 155

Leu Ala Asp Gly Gln Val Arg Lys Ile Thr Gln Val Gln Val Val Gly
            160                 165                 170

Ala Asn Met Ser Val Tyr Leu Glu Gly Ala Ala Val Asn Gly Ser Val
        175                 180                 185

Val Gly Ala Pro Asn Lys Leu Ala Leu Ala Thr Thr Ser Thr Thr Ser
    190                 195                 200

Pro Ala Pro Thr Pro Ala Pro Ser Ala Pro Thr Pro Ser Val Ile Ala
205                 210                 215                 220

Thr Ser Asn Leu Asn Asn Tyr Thr Asn Ala Gln Trp Leu Asn Gly Met
```

```
                225                 230                 235

Tyr Arg Thr Ala Ala Gly Phe Ser Ile Gln Ala Ser Ser Ala Asn Val
            240                 245                 250

Ala Ala Phe Lys Ala Gly Ala Leu Val Arg Leu Ala Asp Gly Gln Thr
        255                 260                 265

Arg Lys Val Leu Arg Ala Gln Leu Val Gly Ser Asn Met Ser Val Phe
    270                 275                 280

Leu Asp Gly Ala Val Ile Asn Gly Thr Thr Leu Gly Tyr Pro Lys Thr
285                 290                 295                 300

Ile Ser Val Val Ser Thr Ser Thr Gly Thr Pro Ser Ser Pro Ala Leu
                305                 310                 315

Thr Thr Pro Pro Val Glu Pro Ala Pro Ala Pro Val Pro Thr Ala Pro
            320                 325                 330

Asp Thr Thr Asn Gly Lys Pro Leu Leu Val Gly Val Asn Leu Ser Gly
        335                 340                 345

Ala Gly Phe Gly Pro Ser Val Val Pro Gly Lys His Gly Thr Asn Tyr
    350                 355                 360

Thr Tyr Pro Ala Glu Ser Tyr Tyr Lys Lys Tyr Ser Asp Leu Gly Met
365                 370                 375                 380

Pro Leu Val Arg Leu Pro Phe Leu Trp Glu Arg Ile Gln Pro Lys Leu
                385                 390                 395

Asn Ser Pro Leu Asn Ala Glu Glu Phe Ala Arg Leu Lys Gln Ser Leu
            400                 405                 410

Asp Phe Ala Gln Lys His Asn Val Lys Val Ile Leu Asp Leu His Asn
        415                 420                 425

Tyr Tyr Arg Tyr Tyr Gly Lys Leu Ile Gly Ser Lys Glu Val Pro Ile
    430                 435                 440

Ser Ser Phe Ala Ala Val Trp Lys Gln Ile Val Gln Gln Val Val Asn
445                 450                 455                 460

His Pro Ala Val Glu Gly Tyr Gly Leu Met Asn Glu Pro His Ser Thr
                465                 470                 475
```

```
Asn Gly Leu Trp Pro Gln Ala Ala Leu Ala Ala Ala Gln Ala Ile Arg
            480                 485                 490

Thr Val Asp Ser Lys Arg Trp Ile Tyr Val Ala Gly Asp Arg Trp Ser
        495                 500                 505

Ser Ala Phe His Trp Pro His Tyr Asn Thr Gln Leu Val Thr Asn Pro
    510                 515                 520

Trp Met Arg Asp Pro Lys Asn Asn Leu Val Tyr Glu Ala His Met Tyr
525                 530                 535                 540

Val Asp Lys Asp Phe Ser Gly Asn Tyr Phe Asp Lys Ala Glu Lys Phe
                545                 550                 555

Asp Pro Met Ile Gly Val Asn Arg Val Lys Pro Phe Val Asp Trp Leu
            560                 565                 570

Lys Gln His Lys Leu Arg Gly Tyr Ile Gly Glu His Gly Val Pro Asp
        575                 580                 585

Phe Ser Pro Ser Ala Ile Val Ala Thr Asp Asn Leu Leu Ala Tyr Leu
    590                 595                 600

Arg Gln Asn Cys Ile Pro Ser Thr Tyr Trp Ala Ala Gly Pro Trp Trp
605                 610                 615                 620

Gly Glu Tyr Ala Met Ser Leu Asp Val Ser Ser Gly Lys His Arg Pro
                625                 630                 635

Gln Leu Pro Val Leu Gln Lys His Ala Lys Thr Ala Asn Ser Cys Thr
            640                 645                 650

Ser Ile Gly Pro Leu
        655
```

<210> 9
<211> 2409
<212> DNA
<213> Artificial

<220>
<223> Codon optimized

<400> 9

gcagactatt atctgaaagc atcacaaggc gcatcaaatc attggtcatc acatctgaca 60

gattggacag caaatgcaga tggcacaggc gcaaatccga cagttattgg cctggcagat 120

acatttgata caaataatcg cacactgaga acaccggcag ttaatgcaac aacaacatat 180

```
cctggcggag ttctgagact gtcaggcgga gcaggcgtta ttggcatgaa aacaggcgga      240
acagcagttg caattgttcc gaaactggtt tcaacagcag gcacagttga tgcatggcat      300
acaggcacac agtattttag agcagatgat tgggaaaatc ttgcatcagg cacaggcttt      360
acagcactga aagcagtcgc aggcagaaca cttaaagttt cagttggcaa actgacaggc      420
tcaggcgaaa caagactgca tggcggaggc gcagttagac tggatgttac agatggcgaa      480
agatatctgg gcgttgttag agtttcatca ggcgcagcag attttgataa taacgttttt      540
gtttcaggac cgctggttat tgaaacaggc gctacagttg ttctggatca agcagtttca      600
tttgcaggcc ttacagttgc tggcacagaa tattcaccgg gaaattatac atttgcagca      660
cttcaagcag cacatccgac ggtttttaca agcggcacag caggcggatc aattacagtt      720
agagcaccga gaacatggta tctgacagtt aatcaaggcg gagtccaaaa ttggacagaa      780
acatatctga gcaattggaa ttcagcagca aatggatcag gcgttgcacc gacatcaatt      840
aatggctatg acttttatat cgatcaggtc agcaatcgcg aaattagaac accgtcaaca      900
gcatcaacat ttggaggcgg agcgctggca ctggcatctg gcgcaaaact gacactgaaa      960
tcatcacctg gcgttgtttc aacaattccg gcatttgtta atacaaacag cccgattatt     1020
gttaatggcg gtggctcatt tagacaatca ctggcacttg gcgactggga aattgcaagc     1080
ggcattacaa aactgtcagc aggcagcggc agatcactgg gctttgatat tgattatctt     1140
ggcggagctg gcggactggt tacacaaaat ggcggatcat actttctgtc actggatgat     1200
ggctcaggct atacgggcac actgaatcat gcgtcaggcg cactgagatt tgaatcagtt     1260
tttagcacag aaggcgcact tacaattggc tcatcagcaa cagttcatct tgatcaacaa     1320
gtctatgtca caagctttag cgttgcaggc gtcgcaaaag cagcaggcat tcatacatat     1380
gcatcactga atgcagcgca tccggcacaa tttacagctg gcgcagcacc gggactggtt     1440
gcagtttata caccggatac agcaggaccg gttagaatga atggcgtcaa tattagcgga     1500
ccggaatcaa atacagcaaa tcttccggga acatatggct ataactatgt ctatccgaca     1560
gaagcggact ttgattatta tgcatcaaaa ggcctgaacc tgattagaat tccgtttaga     1620
tgggaaagaa tgcagcatgg cctgaatgtt ccgctgaata cagcacaact gggctatatg     1680
gatacagcgg ttgcaagagc atcagcaaga ggcatgaaag ttattctgga catgcataac     1740
tatgcacgct gcaaagttgg aggcgttaca tacaaatttg gagatgcaca acttccggca     1800
agcgcatatg cagatgtttg gcgcagactt gcagaccact ataaaaacga accggcaatt     1860
tatggctttg acattatgaa tgaaccgaat ggcctgagcg gaggcgtttg gcctgcgtat     1920
gcacaagcag cagtcaatgc aattagagaa gttaatctga gcacatgggt tattgtcgaa     1980
ggcgaatttt gggcaaatgc atggggcttt gaaacgaaaa atccgtatct gcataatgtg     2040
agagatccgg ttggcagact gatgttttca gcacattcat attggtcaga tgcaggcacg     2100
```

```
gatgtctata aaacatatga tgaagaaggc gcttatccgg aaatgggcgt taataatgtt    2160
aaaccgttta tcgattggct gaaaaaacat gacgcaaaag gctttgttgg cgaatatggc    2220
gttccgaata atgatccgag atggctggtt gtcctggata attttctggc atatctggca    2280
gcagaaggcg tttcaggcac atattgggct ggcggagcat ggtattcagg ctcaccgatt    2340
agctgccatc cgtcaagcaa ctatacagtt gatagagcag ttatgagcgt cctggaagat    2400
catccgtaa                                                            2409
```

<210> 10
<211> 2452
<212> DNA
<213> Artificial

<220>
<223> Codon optimized

<400> 10

```
gcttttagtt catcgatagc atcagcacat catcatcacc atcatccgag agcagattgg      60
tatctggata aaaatcaagc aagatatgcg agctgggata cactggcaga ttggaaaccg     120
aatccggatg gctcaggctc aaatccgtca gcactgtcac cgtcagatac atatcatctg     180
aatggcttta tgctgagaac accggaaggc ggatcaacat atacatttac aggcggactg     240
ctgagcctgg caaataatgc agataatttt gcgctgaaaa caacaggctc aggcgtttca     300
attattccgg cactgagaac aacagcaggc ctggttcaaa atgttggcag cggcacacaa     360
aatctgcaag ttggccatta tcaaaatctg tcaggcacaa caagctatta tgcacaaaca     420
ggcagaggcc tgaatctggc aattacaaca ctggttggct caggacagtt tagattttat     480
ggcggaggca catattatct gtctctggca aattcaccga catatgatgg cgatatttat     540
gtccaaagcg gcacaattga ttttaacaat gatctggcga cagcaggcac actgacagtt     600
aatacaggcg caaaagttgc actggatcaa gcagttacgt ttacaggact gacaattgca     660
ggcacagcat atccggttgg caattattca tatgcagcac tgcaagcagc acatccggca     720
gtttttgttt ctggcacatc aggcggagca attaatgtta gagcaccgag aaattggtac     780
ttgtcaacac atcagccggt tggcgcatca tggaatacac ttgcgcattg gagagcaaac     840
ccggatggaa caggcgctac agcagattca attaatagct ttgacaacta tatcaaccag     900
gtcagcggca gaacactgcg cacaccggaa acaacagcga catttgctgg cggatcactg     960
gttctggcag atggcggaaa tctttcactg aaagcaccgg caggccattc atcaacaatt    1020
ccggcatttg caacatcagg cagcatttca attacaaacg gctttagctc aattacacaa    1080
ccgctggtta ttggcgattg gcatcttggc gctggcacag cacaagtttc agttccgtca    1140
acatcaacag ttcaactgac agtcgataaa ctgagcggag atggcacact gcaatttcaa    1200
```

```
aatggcggta aatatacgct gaacattaga ggcgcatcag cttttacagg cacattaaga      1260
catctgagcg gaacacttac agttgcatca caaattggca caggcggaac attagttgtt      1320
gaatcaacag gcgcagttaa actggatcat ccgggatttt ttacaggtgt tacagtggct      1380
ggcacaccgc tggcaccggg atatcataca tatgcggcac ttaaagcggc tcatcctgcg      1440
agatttccga caggctcaac aaatgcgttt cttgcagttt atcctccgga tacaacagga      1500
ccggcacata tgtttggcgt taatctggct ggcggagaat ttggaacacc gatgcctggc      1560
gtttatggca cagattatat ctatccgagc gcagcagcat ttgattatta tcatggcaaa      1620
ggccttaaac tgattcgcct gccgtttaaa tgggaaagac tgcaacatac acttaatgca      1680
ccgctgaatg cagcagaact ggcaagaatt gatacagttg ttggctatgc atcagcaaga      1740
ggcatgaaag ttgttctgga tatgcataac tatgcgcgta gaaaagaatc aggcacgaca      1800
tatctgatcg gcacaggccc tgttacaatg gatgcatttg gagatgtttg gagaagaatc      1860
gcggatcatt ataaaggcaa tccggcaatt tatggctacg gcattatgaa tgaaccgtat      1920
agcacaaata caacgtggcc tcaaatggcg caaacagcag ttaatgcaat tagaacagtt      1980
gatctgacaa cgcatgttat tgttgcaggc gacggctggt caaatgcaac aggctggcgc      2040
tcaaaaaatc cgaatctgga tacacaagat ccggtcggca gactgattta tgaagcacat      2100
tgctattttg acagcaacct ttcaggcacg tatacacaaa gctatgatgc agcaggcgca      2160
catccgatga ttggcgttga tagagttaga gaatttgtcg aatggcttca agaaacaggc      2220
aacaaaggct ttattggaga atatggcgtt ccgggaaatg atccgagatg gctggttgtt      2280
cttgataatt ttctggcata tctggatgca aatggcgtta gcggaacata ttgggcaggc      2340
ggaccgtggt ggggcaatta tccgctgtca tgcgaaccga catcaaatta cacagttgat      2400
aaaccgcaaa tgagcgtcct ggaaaactac aactaaacgc gttaatcaat aa              2452
```

<210> 11
<211> 2403
<212> DNA
<213> Artificial

<220>
<223> Codon optimized

<400> 11

```
gcagattatt atctgaaagt taatcaaccg catccgaatt catgggcatc accggttaca      60
gattgggcag caaatccgga tggcacaggc gcagcaccgg cagcaattgc ggcaccggat     120
acattttata caaataatag aacacttcgc acaccggctg ttggcgttaa tgcaacattt     180
cctggcggag ttctgggcct gaatggcgga gtcattggca ttaaaacagg accgtcagca     240
ttttcaattg caccgaaact ggtttcaaca gcaggcgcaa ttgaatcatg gggcacaccg     300
cagaatttta gagcagatga ttgggaatca aatgcaccgt ttccgacatt tacaggcctg     360
```

agaacagcat caaatcatac acttaaagtt agcgttggca aactgagcgg aacaggcgaa 420
attagagttc atggcggagg cacagttctg ctggatgtta cagatgcaga aaattatctg 480
ggcacactgt gcgttgcatc aggcgcactg aattttgata atgcagtttt ttcatcagga 540
ccgctggata tcaaaacagg cgcaacagtt gttctggatc aagcagtttc atttgcaggc 600
cttgcagttg gagcaacaga atatccgcct ggcaattata cactggcagc actgcaagca 660
gcacatcctg gcgtttttac aggcacagca gcaggatcaa ttacagttag agcaccgaga 720
acatggtatc tgacagtttc acaaggctca caaaattgga cagaagcatt tctgtcaaat 780
tggaattcag cagcaaatgg ctcaggcgtc gcaccgaatt atatcaatgg acatgatatc 840
tatctgaacc aggtcaataa tcgcgaactg agaacaccgt atacagcaag cacgtttaca 900
ggcggaacac tggcactgac atttggctca aaactggttg ttaaaacaag cccgaatctg 960
gttagcacaa ttccggcact ggttacatct ggaacaccgc aatttgcgaa tggcagcggc 1020
tcaagacaaa atctggcaat tggcgattgg gatattatct caggcacatc aagactggtt 1080
gcaggctcaa caagatcact gggctttgat attggctggc tgacaggcgc tggcaatctg 1140
caaacagaag gcggaggctc atttttctg agactgattg atggatcagg ctatacaggc 1200
gctattaacc ataattctgg cgctctgaga tttgaaagcg tttttagcac agctggcgca 1260
cttaatattg gcgcatcagc aacagttcat cttgataaac cggtctatgt ttcaggcctt 1320
agcgttgcag gcgttgcgaa accggcaggc attcatacat atgcatcact taatgcagcg 1380
catccggcac aatttaatgc aggcgctgct ccgggacttg ttgcagttta tacaccgaac 1440
acagcagctc cggttagaat gaatggcgtc aatctgtcag gaccggaatc agttggcgga 1500
gcaggtacac cttttccggg aacatatggc tttcaatgga tttatccgac agtcgcggat 1560
tatgattatt atgcagcaaa aggccttaac ctgattagaa ttccgtttag atgggaaaga 1620
atgcaaggca cactgaatgg accgctgatt gcagcggaac tggcaagaat ggataatgca 1680
attgcgctgg catcagcgag aggcatgaaa gttattctgg atatgcataa ctatgcacgc 1740
tatagaacac cgacagcatc atatgttttt ggagatgcgc aacttccggc atcagcattt 1800
gcagatgttt ggagaaaact ggcggatcac tataaaaacg aaccggcaat ttatggcttt 1860
gacattatga atgaaccgca ttcaatgccg acaccgacaa cgtggccgac atatgcacaa 1920
gcagcagttc atgcaattag agaagtcaat ctggatacat ggattatcgt tgaaggcgaa 1980
acatatgcga actcatggaa atttggcgaa aaaaatccgc atctgcataa tgttagagat 2040
ccggttggca gactgatgtt ttcagcacat tcatattggt gcaaaaatgg cgacgatcgc 2100
tatggcacgt atgatgcgga aaatggccat ccgcaaatgg gcgttgattc actgaaacat 2160
tttgttgatt ggctgcgcaa acataatgca catggctttg ttggcgaata tggcgttccg 2220

aataatgatc cgagatggct ggaagttctg gaaaatgcac tgatttatct ggcgaacgaa 2280

aacattagcg gcacatattg ggcaggcgga gcatggctgg caggctcaca tatttcatgc 2340

catccgtcat ctaactatac agttgatcgt ccggttatga gcgtcctgca aaattatccg 2400

taa 2403

<210> 12
<211> 1974
<212> DNA
<213> Artificial

<220>
<223> Codon optimized

<400> 12

tcatcagttg cagcagtttc agtttcagca aaaatcaatg cgtttacgaa tagcgattgg 60
ctgaatggca tttggagaac aggcgcaggc ttttcaattc cggcaacatc agcaaataga 120
gcagcatttg ttgcaggcgc atcagttaga ctggcagatg gccaagttag aaaaattagc 180
agagcacaaa ttgtcggcag caacatgtca atttttctgg aaggcgcaaa actggatggc 240
aataaagttg gcgcaccgca agttgttaca attggctcaa cagcagttac agcaccggat 300
acatcagcac cgattacaac accgcctaca gtcacagcac attcaacatc aattaacgcc 360
tttacaaata atgactggct taacggcgtt tggcgcaaat caccgggatt tagcattccg 420
gcatctgcag cgaataaagc ggcttttaaa gttggagcaa cagcaaaact tgcggatgga 480
caggttcgca aaattacaca agttcaagtt gttggcgcta acatgagcgt ttatcttgaa 540
ggcgcagcag tcaatggctc agttgttgga gcaccgaata aactggcact ggcaacaaca 600
agcacaacat caccggcacc gacaccggct ccgtcagctc cgacaccgtc agttattgca 660
acatcaaatc tgaacaacta tacaaatgcg cagtggctga acggaatgta tagaacagca 720
gcgggatttt ctattcaagc atcaagcgca aatgtcgcag catttaaagc aggcgcactg 780
gtcagacttg ctgatggcca gacaagaaaa gttctgagag cacaactggt tggctcaaat 840
atgtcagtct ttcttgatgg cgctgtcatt aatggcacaa cactgggcta tccgaaaaca 900
atttcagttg ttagcacatc aacaggcaca ccgtcatctc cggcactgac aacacctccg 960
gttgaaccgg ctcctgcacc ggttccgaca gcgcctgata caacaaatgg caaaccgctg 1020
ctggttggcg ttaatctgag cggagcaggc tttggaccga gcgttgttcc gggaaaacat 1080
ggcacaaatt atacatatcc ggcagaaagc tactacaaaa aatactcaga tctgggcatg 1140
ccgctggtta gactgccgtt tctgtgggaa agaattcaac cgaaactgaa ttcaccgctg 1200
aatgcagaag aatttgcaag actgaaacag agcctggatt ttgcgcagaa acataacgtt 1260
aaagtcatcc tggatctgca taactattat cgctattacg gcaaactgat tggcagcaaa 1320
gaagttccga tttcaagctt tgcggcagtc tggaaacaaa ttgttcaaca agttgtcaat 1380

```
catccggcag ttgaaggcta tggcctgatg aatgaaccgc atagcacaaa tggcctgtgg     1440

cctcaagcag cactggcagc agcacaagca attagaacag ttgatagcaa acgctggatt     1500

tatgtcgcag gcgatagatg gtcatcagca tttcattggc ctcattataa cacacagctg     1560

gttacaaatc cgtggatgag agatccgaaa aataacctgg tttatgaagc gcatatgtat     1620

gtcgacaaag attttagcgg caactacttt gacaaagcgg aaaaatttga tccgatgatt     1680

ggcgtcaatc gcgttaaacc gtttgttgat tggcttaaac agcataaact gcgtggctat     1740

attggcgaac atggcgttcc ggatttttca ccgtcagcaa ttgttgcgac agataatctg     1800

ctggcatatc tgagacaaaa ttgcattccg tcaacatatt gggcagcagg accgtggtgg     1860

ggagaatatg caatgtcact ggatgtttca agcggcaaac atagaccgca acttccggtt     1920

cttcaaaaac atgcaaaaac agcgaatagc tgcacatcaa ttggaccgct gtaa           1974
```

<210> 13
<211> 811
<212> PRT
<213> Artificial

<220>
<223> HISTAG' ed

<220>
<221> MISC_FEATURE
<222> (1)..(9)
<223> His tag

<400> 13

```
Met His His His His His His Pro Arg Ala Asp Tyr Tyr Leu Lys Ala
1               5                   10                  15

Ser Gln Gly Ala Ser Asn His Trp Ser Ser His Leu Thr Asp Trp Thr
            20                  25                  30

Ala Asn Ala Asp Gly Thr Gly Ala Asn Pro Thr Val Ile Gly Leu Ala
        35                  40                  45

Asp Thr Phe Asp Thr Asn Asn Arg Thr Leu Arg Thr Pro Ala Val Asn
    50                  55                  60

Ala Thr Thr Thr Tyr Pro Gly Gly Val Leu Arg Leu Ser Gly Gly Ala
65                  70                  75                  80

Gly Val Ile Gly Met Lys Thr Gly Gly Thr Ala Val Ala Ile Val Pro
                85                  90                  95

Lys Leu Val Ser Thr Ala Gly Thr Val Asp Ala Trp His Thr Gly Thr
```

```
            100                 105                 110

Gln Tyr Phe Arg Ala Asp Asp Trp Glu Asn Leu Ala Ser Gly Thr Gly
        115                 120                 125

Phe Thr Ala Leu Lys Ala Val Ala Gly Arg Thr Leu Lys Val Ser Val
    130                 135                 140

Gly Lys Leu Thr Gly Ser Gly Glu Thr Arg Leu His Gly Gly Gly Ala
145                 150                 155                 160

Val Arg Leu Asp Val Thr Asp Gly Glu Arg Tyr Leu Gly Val Val Arg
                165                 170                 175

Val Ser Ser Gly Ala Ala Asp Phe Asp Asn Asn Val Phe Val Ser Gly
            180                 185                 190

Pro Leu Val Ile Glu Thr Gly Ala Thr Val Val Leu Asp Gln Ala Val
        195                 200                 205

Ser Phe Ala Gly Leu Thr Val Ala Gly Thr Glu Tyr Ser Pro Gly Asn
    210                 215                 220

Tyr Thr Phe Ala Ala Leu Gln Ala Ala His Pro Thr Val Phe Thr Ser
225                 230                 235                 240

Gly Thr Ala Gly Gly Ser Ile Thr Val Arg Ala Pro Arg Thr Trp Tyr
                245                 250                 255

Leu Thr Val Asn Gln Gly Gly Val Gln Asn Trp Thr Glu Thr Tyr Leu
            260                 265                 270

Ser Asn Trp Asn Ser Ala Ala Asn Gly Ser Gly Val Ala Pro Thr Ser
        275                 280                 285

Ile Asn Gly Tyr Asp Phe Tyr Ile Asp Gln Val Ser Asn Arg Glu Ile
    290                 295                 300

Arg Thr Pro Ser Thr Ala Ser Thr Phe Gly Gly Gly Ala Leu Ala Leu
305                 310                 315                 320

Ala Ser Gly Ala Lys Leu Thr Leu Lys Ser Ser Pro Gly Val Val Ser
                325                 330                 335

Thr Ile Pro Ala Phe Val Asn Thr Asn Ser Pro Ile Ile Val Asn Gly
            340                 345                 350
```

```
Gly Gly Ser Phe Arg Gln Ser Leu Ala Leu Gly Asp Trp Glu Ile Ala
        355                 360                 365

Ser Gly Ile Thr Lys Leu Ser Ala Gly Ser Gly Arg Ser Leu Gly Phe
    370                 375                 380

Asp Ile Asp Tyr Leu Gly Gly Ala Gly Gly Leu Val Thr Gln Asn Gly
385                 390                 395                 400

Gly Ser Tyr Phe Leu Ser Leu Asp Asp Gly Ser Gly Tyr Thr Gly Thr
                405                 410                 415

Leu Asn His Ala Ser Gly Ala Leu Arg Phe Glu Ser Val Phe Ser Thr
            420                 425                 430

Glu Gly Ala Leu Thr Ile Gly Ser Ser Ala Thr Val His Leu Asp Gln
        435                 440                 445

Gln Val Tyr Val Thr Ser Phe Ser Val Ala Gly Val Ala Lys Ala Ala
    450                 455                 460

Gly Ile His Thr Tyr Ala Ser Leu Asn Ala Ala His Pro Ala Gln Phe
465                 470                 475                 480

Thr Ala Gly Ala Ala Pro Gly Leu Val Ala Val Tyr Thr Pro Asp Thr
                485                 490                 495

Ala Gly Pro Val Arg Met Asn Gly Val Asn Ile Ser Gly Pro Glu Ser
            500                 505                 510

Asn Thr Ala Asn Leu Pro Gly Thr Tyr Gly Tyr Asn Tyr Val Tyr Pro
        515                 520                 525

Thr Glu Ala Asp Phe Asp Tyr Tyr Ala Ser Lys Gly Leu Asn Leu Ile
    530                 535                 540

Arg Ile Pro Phe Arg Trp Glu Arg Met Gln His Gly Leu Asn Val Pro
545                 550                 555                 560

Leu Asn Thr Ala Gln Leu Gly Tyr Met Asp Thr Ala Val Ala Arg Ala
                565                 570                 575

Ser Ala Arg Gly Met Lys Val Ile Leu Asp Met His Asn Tyr Ala Arg
            580                 585                 590

Cys Lys Val Gly Gly Val Thr Tyr Lys Phe Gly Asp Ala Gln Leu Pro
        595                 600                 605
```

Ala Ser Ala Tyr Ala Asp Val Trp Arg Arg Leu Ala Asp His Tyr Lys
610 615 620

Asn Glu Pro Ala Ile Tyr Gly Phe Asp Ile Met Asn Glu Pro Asn Gly
625 630 635 640

Leu Ser Gly Gly Val Trp Pro Ala Tyr Ala Gln Ala Ala Val Asn Ala
645 650 655

Ile Arg Glu Val Asn Leu Ser Thr Trp Val Ile Val Glu Gly Glu Phe
660 665 670

Trp Ala Asn Ala Trp Gly Phe Glu Thr Lys Asn Pro Tyr Leu His Asn
675 680 685

Val Arg Asp Pro Val Gly Arg Leu Met Phe Ser Ala His Ser Tyr Trp
690 695 700

Ser Asp Ala Gly Thr Asp Val Tyr Lys Thr Tyr Asp Glu Glu Gly Ala
705 710 715 720

Tyr Pro Glu Met Gly Val Asn Asn Val Lys Pro Phe Ile Asp Trp Leu
725 730 735

Lys Lys His Asp Ala Lys Gly Phe Val Gly Glu Tyr Gly Val Pro Asn
740 745 750

Asn Asp Pro Arg Trp Leu Val Val Leu Asp Asn Phe Leu Ala Tyr Leu
755 760 765

Ala Ala Glu Gly Val Ser Gly Thr Tyr Trp Ala Gly Gly Ala Trp Tyr
770 775 780

Ser Gly Ser Pro Ile Ser Cys His Pro Ser Ser Asn Tyr Thr Val Asp
785 790 795 800

Arg Ala Val Met Ser Val Leu Glu Asp His Pro
805 810

<210> 14
<211> 803
<212> PRT
<213> Artificial

<220>
<223> His tag'ed

<220>
<221> MISC_FEATURE

<222> (1)..(9)
<223> His tag

<400> 14

Met His His His His His His Pro Arg Ala Asp Trp Tyr Leu Asp Lys
1 5 10 15

Asn Gln Ala Arg Tyr Ala Ser Trp Asp Thr Leu Ala Asp Trp Lys Pro
20 25 30

Asn Pro Asp Gly Ser Gly Ser Asn Pro Ser Ala Leu Ser Pro Ser Asp
35 40 45

Thr Tyr His Leu Asn Gly Phe Met Leu Arg Thr Pro Glu Gly Gly Ser
50 55 60

Thr Tyr Thr Phe Thr Gly Gly Leu Leu Ser Leu Ala Asn Asn Ala Asp
65 70 75 80

Asn Phe Ala Leu Lys Thr Thr Gly Ser Gly Val Ser Ile Ile Pro Ala
85 90 95

Leu Arg Thr Thr Ala Gly Leu Val Gln Asn Val Gly Ser Gly Thr Gln
100 105 110

Asn Leu Gln Val Gly His Tyr Gln Asn Leu Ser Gly Thr Thr Ser Tyr
115 120 125

Tyr Ala Gln Thr Gly Arg Gly Leu Asn Leu Ala Ile Thr Thr Leu Val
130 135 140

Gly Ser Gly Gln Phe Arg Phe Tyr Gly Gly Gly Thr Tyr Tyr Leu Ser
145 150 155 160

Leu Ala Asn Ser Pro Thr Tyr Asp Gly Asp Ile Tyr Val Gln Ser Gly
165 170 175

Thr Ile Asp Phe Asn Asn Asp Leu Ala Thr Ala Gly Thr Leu Thr Val
180 185 190

Asn Thr Gly Ala Lys Val Ala Leu Asp Gln Ala Val Thr Phe Thr Gly
195 200 205

Leu Thr Ile Ala Gly Thr Ala Tyr Pro Val Gly Asn Tyr Ser Tyr Ala
210 215 220

Ala Leu Gln Ala Ala His Pro Ala Val Phe Val Ser Gly Thr Ser Gly

225 230 235 240

Gly Ala Ile Asn Val Arg Ala Pro Arg Asn Trp Tyr Leu Ser Thr His
245 250 255

Gln Pro Val Gly Ala Ser Trp Asn Thr Leu Ala His Trp Arg Ala Asn
260 265 270

Pro Asp Gly Thr Gly Ala Thr Ala Asp Ser Ile Asn Ser Phe Asp Asn
275 280 285

Tyr Ile Asn Gln Val Ser Gly Arg Thr Leu Arg Thr Pro Glu Thr Thr
290 295 300

Ala Thr Phe Ala Gly Gly Ser Leu Val Leu Ala Asp Gly Gly Asn Leu
305 310 315 320

Ser Leu Lys Ala Pro Ala Gly His Ser Ser Thr Ile Pro Ala Phe Ala
325 330 335

Thr Ser Gly Ser Ile Ser Ile Thr Asn Gly Phe Ser Ser Ile Thr Gln
340 345 350

Pro Leu Val Ile Gly Asp Trp His Leu Gly Ala Gly Thr Ala Gln Val
355 360 365

Ser Val Pro Ser Thr Ser Thr Val Gln Leu Thr Val Asp Lys Leu Ser
370 375 380

Gly Asp Gly Thr Leu Gln Phe Gln Asn Gly Gly Lys Tyr Thr Leu Asn
385 390 395 400

Ile Arg Gly Ala Ser Ala Phe Thr Gly Thr Leu Arg His Leu Ser Gly
405 410 415

Thr Leu Thr Val Ala Ser Gln Ile Gly Thr Gly Gly Thr Leu Val Val
420 425 430

Glu Ser Thr Gly Ala Val Lys Leu Asp His Pro Gly Phe Phe Thr Gly
435 440 445

Val Thr Val Ala Gly Thr Pro Leu Ala Pro Gly Tyr His Thr Tyr Ala
450 455 460

Ala Leu Lys Ala Ala His Pro Ala Arg Phe Pro Thr Gly Ser Thr Asn
465 470 475 480

Ala Phe Leu Ala Val Tyr Pro Pro Asp Thr Thr Gly Pro Ala His Met
485 490 495

Phe Gly Val Asn Leu Ala Gly Gly Glu Phe Gly Thr Pro Met Pro Gly
500 505 510

Val Tyr Gly Thr Asp Tyr Ile Tyr Pro Ser Ala Ala Ala Phe Asp Tyr
515 520 525

Tyr His Gly Lys Gly Leu Lys Leu Ile Arg Leu Pro Phe Lys Trp Glu
530 535 540

Arg Leu Gln His Thr Leu Asn Ala Pro Leu Asn Ala Ala Glu Leu Ala
545 550 555 560

Arg Ile Asp Thr Val Val Gly Tyr Ala Ser Ala Arg Gly Met Lys Val
565 570 575

Val Leu Asp Met His Asn Tyr Ala Arg Arg Lys Glu Ser Gly Thr Thr
580 585 590

Tyr Leu Ile Gly Thr Gly Pro Val Thr Met Asp Ala Phe Gly Asp Val
595 600 605

Trp Arg Arg Ile Ala Asp His Tyr Lys Gly Asn Pro Ala Ile Tyr Gly
610 615 620

Tyr Gly Ile Met Asn Glu Pro Tyr Ser Thr Asn Thr Thr Trp Pro Gln
625 630 635 640

Met Ala Gln Thr Ala Val Asn Ala Ile Arg Thr Val Asp Leu Thr Thr
645 650 655

His Val Ile Val Ala Gly Asp Gly Trp Ser Asn Ala Thr Gly Trp Arg
660 665 670

Ser Lys Asn Pro Asn Leu Asp Thr Gln Asp Pro Val Gly Arg Leu Ile
675 680 685

Tyr Glu Ala His Cys Tyr Phe Asp Ser Asn Leu Ser Gly Thr Tyr Thr
690 695 700

Gln Ser Tyr Asp Ala Ala Gly Ala His Pro Met Ile Gly Val Asp Arg
705 710 715 720

Val Arg Glu Phe Val Glu Trp Leu Gln Glu Thr Gly Asn Lys Gly Phe
725 730 735

Ile Gly Glu Tyr Gly Val Pro Gly Asn Asp Pro Arg Trp Leu Val Val
740 745 750

Leu Asp Asn Phe Leu Ala Tyr Leu Asp Ala Asn Gly Val Ser Gly Thr
755 760 765

Tyr Trp Ala Gly Gly Pro Trp Trp Gly Asn Tyr Pro Leu Ser Cys Glu
770 775 780

Pro Thr Ser Asn Tyr Thr Val Asp Lys Pro Gln Met Ser Val Leu Glu
785 790 795 800

Asn Tyr Asn

<210> 15
<211> 809
<212> PRT
<213> Artificial

<220>
<223> HISTAG'ed

<220>
<221> MISC_FEATURE
<222> (1)..(9)
<223> His tag

<400> 15

Met His His His His His His Pro Arg Ala Asp Tyr Tyr Leu Lys Val
1 5 10 15

Asn Gln Pro His Pro Asn Ser Trp Ala Ser Pro Val Thr Asp Trp Ala
20 25 30

Ala Asn Pro Asp Gly Thr Gly Ala Ala Pro Ala Ala Ile Ala Ala Pro
35 40 45

Asp Thr Phe Tyr Thr Asn Asn Arg Thr Leu Arg Thr Pro Ala Val Gly
50 55 60

Val Asn Ala Thr Phe Pro Gly Gly Val Leu Gly Leu Asn Gly Gly Val
65 70 75 80

Ile Gly Ile Lys Thr Gly Pro Ser Ala Phe Ser Ile Ala Pro Lys Leu
85 90 95

Val Ser Thr Ala Gly Ala Ile Glu Ser Trp Gly Thr Pro Gln Asn Phe
100 105 110

```
Arg Ala Asp Asp Trp Glu Ser Asn Ala Pro Phe Pro Thr Phe Thr Gly
        115                 120                 125

Leu Arg Thr Ala Ser Asn His Thr Leu Lys Val Ser Val Gly Lys Leu
    130                 135                 140

Ser Gly Thr Gly Glu Ile Arg Val His Gly Gly Gly Thr Val Leu Leu
145                 150                 155                 160

Asp Val Thr Asp Ala Glu Asn Tyr Leu Gly Thr Leu Cys Val Ala Ser
                165                 170                 175

Gly Ala Leu Asn Phe Asp Asn Ala Val Phe Ser Ser Gly Pro Leu Asp
            180                 185                 190

Ile Lys Thr Gly Ala Thr Val Val Leu Asp Gln Ala Val Ser Phe Ala
        195                 200                 205

Gly Leu Ala Val Gly Ala Thr Glu Tyr Pro Pro Gly Asn Tyr Thr Leu
    210                 215                 220

Ala Ala Leu Gln Ala Ala His Pro Gly Val Phe Thr Gly Thr Ala Ala
225                 230                 235                 240

Gly Ser Ile Thr Val Arg Ala Pro Arg Thr Trp Tyr Leu Thr Val Ser
                245                 250                 255

Gln Gly Ser Gln Asn Trp Thr Glu Ala Phe Leu Ser Asn Trp Asn Ser
            260                 265                 270

Ala Ala Asn Gly Ser Gly Val Ala Pro Asn Tyr Ile Asn Gly His Asp
        275                 280                 285

Ile Tyr Leu Asn Gln Val Asn Asn Arg Glu Leu Arg Thr Pro Tyr Thr
    290                 295                 300

Ala Ser Thr Phe Thr Gly Gly Thr Leu Ala Leu Thr Phe Gly Ser Lys
305                 310                 315                 320

Leu Val Val Lys Thr Ser Pro Asn Leu Val Ser Thr Ile Pro Ala Leu
                325                 330                 335

Val Thr Ser Gly Thr Pro Gln Phe Ala Asn Gly Ser Gly Ser Arg Gln
            340                 345                 350

Asn Leu Ala Ile Gly Asp Trp Asp Ile Ile Ser Gly Thr Ser Arg Leu
```

355 360 365

Val Ala Gly Ser Thr Arg Ser Leu Gly Phe Asp Ile Gly Trp Leu Thr
370 375 380

Gly Ala Gly Asn Leu Gln Thr Glu Gly Gly Gly Ser Phe Phe Leu Arg
385 390 395 400

Leu Ile Asp Gly Ser Gly Tyr Thr Gly Ala Ile Asn His Asn Ser Gly
405 410 415

Ala Leu Arg Phe Glu Ser Val Phe Ser Thr Ala Gly Ala Leu Asn Ile
420 425 430

Gly Ala Ser Ala Thr Val His Leu Asp Lys Pro Val Tyr Val Ser Gly
435 440 445

Leu Ser Val Ala Gly Val Ala Lys Pro Ala Gly Ile His Thr Tyr Ala
450 455 460

Ser Leu Asn Ala Ala His Pro Ala Gln Phe Asn Ala Gly Ala Ala Pro
465 470 475 480

Gly Leu Val Ala Val Tyr Thr Pro Asn Thr Ala Ala Pro Val Arg Met
485 490 495

Asn Gly Val Asn Leu Ser Gly Pro Glu Ser Val Gly Gly Ala Gly Thr
500 505 510

Pro Phe Pro Gly Thr Tyr Gly Phe Gln Trp Ile Tyr Pro Thr Val Ala
515 520 525

Asp Tyr Asp Tyr Tyr Ala Ala Lys Gly Leu Asn Leu Ile Arg Ile Pro
530 535 540

Phe Arg Trp Glu Arg Met Gln Gly Thr Leu Asn Gly Pro Leu Ile Ala
545 550 555 560

Ala Glu Leu Ala Arg Met Asp Asn Ala Ile Ala Leu Ala Ser Ala Arg
565 570 575

Gly Met Lys Val Ile Leu Asp Met His Asn Tyr Ala Arg Tyr Arg Thr
580 585 590

Pro Thr Ala Ser Tyr Val Phe Gly Asp Ala Gln Leu Pro Ala Ser Ala
595 600 605

Phe Ala Asp Val Trp Arg Lys Leu Ala Asp His Tyr Lys Asn Glu Pro
610 615 620

Ala Ile Tyr Gly Phe Asp Ile Met Asn Glu Pro His Ser Met Pro Thr
625 630 635 640

Pro Thr Thr Trp Pro Thr Tyr Ala Gln Ala Ala Val His Ala Ile Arg
645 650 655

Glu Val Asn Leu Asp Thr Trp Ile Ile Val Glu Gly Glu Thr Tyr Ala
660 665 670

Asn Ser Trp Lys Phe Gly Glu Lys Asn Pro His Leu His Asn Val Arg
675 680 685

Asp Pro Val Gly Arg Leu Met Phe Ser Ala His Ser Tyr Trp Cys Lys
690 695 700

Asn Gly Asp Asp Arg Tyr Gly Thr Tyr Asp Ala Glu Asn Gly His Pro
705 710 715 720

Gln Met Gly Val Asp Ser Leu Lys His Phe Val Asp Trp Leu Arg Lys
725 730 735

His Asn Ala His Gly Phe Val Gly Glu Tyr Gly Val Pro Asn Asn Asp
740 745 750

Pro Arg Trp Leu Glu Val Leu Glu Asn Ala Leu Ile Tyr Leu Ala Asn
755 760 765

Glu Asn Ile Ser Gly Thr Tyr Trp Ala Gly Gly Ala Trp Leu Ala Gly
770 775 780

Ser His Ile Ser Cys His Pro Ser Ser Asn Tyr Thr Val Asp Arg Pro
785 790 795 800

Val Met Ser Val Leu Gln Asn Tyr Pro
805

<210> 16
<211> 802
<212> PRT
<213> Artificial

<220>
<223> HISTAG'ed

<220>
<221> MISC_FEATURE

<222> (1)..(8)

<220>
<221> MISC_FEATURE
<222> (1)..(8)
<223> Has tag

<400> 16

```
His His His His His His Pro Arg Ala Asp Trp Tyr Leu Asp Lys Asn
1               5                   10                  15

Gln Ala Arg Tyr Ala Ser Trp Asp Thr Leu Ala Asp Trp Lys Pro Asn
            20                  25                  30

Pro Asp Gly Ser Gly Ser Asn Pro Ser Ala Leu Ser Pro Ser Asp Thr
        35                  40                  45

Tyr His Leu Asn Gly Phe Met Leu Arg Thr Pro Glu Gly Gly Ser Thr
    50                  55                  60

Tyr Thr Phe Thr Gly Gly Leu Leu Ser Leu Ala Asn Asn Ala Asp Asn
65                  70                  75                  80

Phe Ala Leu Lys Thr Thr Gly Ser Gly Val Ser Ile Ile Pro Ala Leu
                85                  90                  95

Arg Thr Thr Ala Gly Leu Val Gln Asn Val Gly Ser Gly Thr Gln Asn
            100                 105                 110

Leu Gln Val Gly His Tyr Gln Asn Leu Ser Gly Thr Thr Ser Tyr Tyr
        115                 120                 125

Ala Gln Thr Gly Arg Gly Leu Asn Leu Ala Ile Thr Thr Leu Val Gly
    130                 135                 140

Ser Gly Gln Phe Arg Phe Tyr Gly Gly Gly Thr Tyr Tyr Leu Ser Leu
145                 150                 155                 160

Ala Asn Ser Pro Thr Tyr Asp Gly Asp Ile Tyr Val Gln Ser Gly Thr
                165                 170                 175

Ile Asp Phe Asn Asn Asp Leu Ala Thr Ala Gly Thr Leu Thr Val Asn
            180                 185                 190

Thr Gly Ala Lys Val Ala Leu Asp Gln Ala Val Thr Phe Thr Gly Leu
        195                 200                 205

Thr Ile Ala Gly Thr Ala Tyr Pro Val Gly Asn Tyr Ser Tyr Ala Ala
    210                 215                 220
```

Leu Gln Ala Ala His Pro Ala Val Phe Val Ser Gly Thr Ser Gly Gly
225 230 235 240

Ala Ile Asn Val Arg Ala Pro Arg Asn Trp Tyr Leu Ser Thr His Gln
245 250 255

Pro Val Gly Ala Ser Trp Asn Thr Leu Ala His Trp Arg Ala Asn Pro
260 265 270

Asp Gly Thr Gly Ala Thr Ala Asp Ser Ile Asn Ser Phe Asp Asn Tyr
275 280 285

Ile Asn Gln Val Ser Gly Arg Thr Leu Arg Thr Pro Glu Thr Thr Ala
290 295 300

Thr Phe Ala Gly Gly Ser Leu Val Leu Ala Asp Gly Gly Asn Leu Ser
305 310 315 320

Leu Lys Ala Pro Ala Gly His Ser Ser Thr Ile Pro Ala Phe Ala Thr
325 330 335

Ser Gly Ser Ile Ser Ile Thr Asn Gly Phe Ser Ser Ile Thr Gln Pro
340 345 350

Leu Val Ile Gly Asp Trp His Leu Gly Ala Gly Thr Ala Gln Val Ser
355 360 365

Val Pro Ser Thr Ser Thr Val Gln Leu Thr Val Asp Lys Leu Ser Gly
370 375 380

Asp Gly Thr Leu Gln Phe Gln Asn Gly Gly Lys Tyr Thr Leu Asn Ile
385 390 395 400

Arg Gly Ala Ser Ala Phe Thr Gly Thr Leu Arg His Leu Ser Gly Thr
405 410 415

Leu Thr Val Ala Ser Gln Ile Gly Thr Gly Gly Thr Leu Val Val Glu
420 425 430

Ser Thr Gly Ala Val Lys Leu Asp His Pro Gly Phe Phe Thr Gly Val
435 440 445

Thr Val Ala Gly Thr Pro Leu Ala Pro Gly Tyr His Thr Tyr Ala Ala
450 455 460

Leu Lys Ala Ala His Pro Ala Arg Phe Pro Thr Gly Ser Thr Asn Ala

465 470 475 480

Phe Leu Ala Val Tyr Pro Pro Asp Thr Thr Gly Pro Ala His Met Phe
485 490 495

Gly Val Asn Leu Ala Gly Gly Glu Phe Gly Thr Pro Met Pro Gly Val
500 505 510

Tyr Gly Thr Asp Tyr Ile Tyr Pro Ser Ala Ala Ala Phe Asp Tyr Tyr
515 520 525

His Gly Lys Gly Leu Lys Leu Ile Arg Leu Pro Phe Lys Trp Glu Arg
530 535 540

Leu Gln His Thr Leu Asn Ala Pro Leu Asn Ala Ala Glu Leu Ala Arg
545 550 555 560

Ile Asp Thr Val Val Gly Tyr Ala Ser Ala Arg Gly Met Lys Val Val
565 570 575

Leu Asp Met His Asn Tyr Ala Arg Arg Lys Glu Ser Gly Thr Thr Tyr
580 585 590

Leu Ile Gly Thr Gly Pro Val Thr Met Asp Ala Phe Gly Asp Val Trp
595 600 605

Arg Arg Ile Ala Asp His Tyr Lys Gly Asn Pro Ala Ile Tyr Gly Tyr
610 615 620

Gly Ile Met Asn Glu Pro Tyr Ser Thr Asn Thr Thr Trp Pro Gln Met
625 630 635 640

Ala Gln Thr Ala Val Asn Ala Ile Arg Thr Val Asp Leu Thr Thr His
645 650 655

Val Ile Val Ala Gly Asp Gly Trp Ser Asn Ala Thr Gly Trp Arg Ser
660 665 670

Lys Asn Pro Asn Leu Asp Thr Gln Asp Pro Val Gly Arg Leu Ile Tyr
675 680 685

Glu Ala His Cys Tyr Phe Asp Ser Asn Leu Ser Gly Thr Tyr Thr Gln
690 695 700

Ser Tyr Asp Ala Ala Gly Ala His Pro Met Ile Gly Val Asp Arg Val
705 710 715 720

Arg Glu Phe Val Glu Trp Leu Gln Glu Thr Gly Asn Lys Gly Phe Ile
725 730 735

Gly Glu Tyr Gly Val Pro Gly Asn Asp Pro Arg Trp Leu Val Val Leu
740 745 750

Asp Asn Phe Leu Ala Tyr Leu Asp Ala Asn Gly Val Ser Gly Thr Tyr
755 760 765

Trp Ala Gly Gly Pro Trp Trp Gly Asn Tyr Pro Leu Ser Cys Glu Pro
770 775 780

Thr Ser Asn Tyr Thr Val Asp Lys Pro Gln Met Ser Val Leu Glu Asn
785 790 795 800

Tyr Asn

<210> 17
<211> 808
<212> PRT
<213> Artificial

<220>
<223> HASTAG'ed

<220>
<221> MISC_FEATURE
<222> (1)..(8)
<223> Has tag

<400> 17

His His His His His His Pro Arg Ala Asp Tyr Tyr Leu Lys Val Asn
1 5 10 15

Gln Pro His Pro Asn Ser Trp Ala Ser Pro Val Thr Asp Trp Ala Ala
20 25 30

Asn Pro Asp Gly Thr Gly Ala Ala Pro Ala Ala Ile Ala Ala Pro Asp
35 40 45

Thr Phe Tyr Thr Asn Asn Arg Thr Leu Arg Thr Pro Ala Val Gly Val
50 55 60

Asn Ala Thr Phe Pro Gly Gly Val Leu Gly Leu Asn Gly Gly Val Ile
65 70 75 80

Gly Ile Lys Thr Gly Pro Ser Ala Phe Ser Ile Ala Pro Lys Leu Val
85 90 95

Ser Thr Ala Gly Ala Ile Glu Ser Trp Gly Thr Pro Gln Asn Phe Arg
100 105 110

Ala Asp Asp Trp Glu Ser Asn Ala Pro Phe Pro Thr Phe Thr Gly Leu
115 120 125

Arg Thr Ala Ser Asn His Thr Leu Lys Val Ser Val Gly Lys Leu Ser
130 135 140

Gly Thr Gly Glu Ile Arg Val His Gly Gly Gly Thr Val Leu Leu Asp
145 150 155 160

Val Thr Asp Ala Glu Asn Tyr Leu Gly Thr Leu Cys Val Ala Ser Gly
165 170 175

Ala Leu Asn Phe Asp Asn Ala Val Phe Ser Ser Gly Pro Leu Asp Ile
180 185 190

Lys Thr Gly Ala Thr Val Val Leu Asp Gln Ala Val Ser Phe Ala Gly
195 200 205

Leu Ala Val Gly Ala Thr Glu Tyr Pro Pro Gly Asn Tyr Thr Leu Ala
210 215 220

Ala Leu Gln Ala Ala His Pro Gly Val Phe Thr Gly Thr Ala Ala Gly
225 230 235 240

Ser Ile Thr Val Arg Ala Pro Arg Thr Trp Tyr Leu Thr Val Ser Gln
245 250 255

Gly Ser Gln Asn Trp Thr Glu Ala Phe Leu Ser Asn Trp Asn Ser Ala
260 265 270

Ala Asn Gly Ser Gly Val Ala Pro Asn Tyr Ile Asn Gly His Asp Ile
275 280 285

Tyr Leu Asn Gln Val Asn Asn Arg Glu Leu Arg Thr Pro Tyr Thr Ala
290 295 300

Ser Thr Phe Thr Gly Gly Thr Leu Ala Leu Thr Phe Gly Ser Lys Leu
305 310 315 320

Val Val Lys Thr Ser Pro Asn Leu Val Ser Thr Ile Pro Ala Leu Val
325 330 335

Thr Ser Gly Thr Pro Gln Phe Ala Asn Gly Ser Gly Ser Arg Gln Asn
340 345 350

```
Leu Ala Ile Gly Asp Trp Asp Ile Ile Ser Gly Thr Ser Arg Leu Val
        355                 360                 365

Ala Gly Ser Thr Arg Ser Leu Gly Phe Asp Ile Gly Trp Leu Thr Gly
    370                 375                 380

Ala Gly Asn Leu Gln Thr Glu Gly Gly Gly Ser Phe Phe Leu Arg Leu
385                 390                 395                 400

Ile Asp Gly Ser Gly Tyr Thr Gly Ala Ile Asn His Asn Ser Gly Ala
                405                 410                 415

Leu Arg Phe Glu Ser Val Phe Ser Thr Ala Gly Ala Leu Asn Ile Gly
            420                 425                 430

Ala Ser Ala Thr Val His Leu Asp Lys Pro Val Tyr Val Ser Gly Leu
        435                 440                 445

Ser Val Ala Gly Val Ala Lys Pro Ala Gly Ile His Thr Tyr Ala Ser
    450                 455                 460

Leu Asn Ala Ala His Pro Ala Gln Phe Asn Ala Gly Ala Ala Pro Gly
465                 470                 475                 480

Leu Val Ala Val Tyr Thr Pro Asn Thr Ala Ala Pro Val Arg Met Asn
                485                 490                 495

Gly Val Asn Leu Ser Gly Pro Glu Ser Val Gly Gly Ala Gly Thr Pro
            500                 505                 510

Phe Pro Gly Thr Tyr Gly Phe Gln Trp Ile Tyr Pro Thr Val Ala Asp
        515                 520                 525

Tyr Asp Tyr Tyr Ala Ala Lys Gly Leu Asn Leu Ile Arg Ile Pro Phe
    530                 535                 540

Arg Trp Glu Arg Met Gln Gly Thr Leu Asn Gly Pro Leu Ile Ala Ala
545                 550                 555                 560

Glu Leu Ala Arg Met Asp Asn Ala Ile Ala Leu Ala Ser Ala Arg Gly
                565                 570                 575

Met Lys Val Ile Leu Asp Met His Asn Tyr Ala Arg Tyr Arg Thr Pro
            580                 585                 590

Thr Ala Ser Tyr Val Phe Gly Asp Ala Gln Leu Pro Ala Ser Ala Phe
```

595 600 605

Ala Asp Val Trp Arg Lys Leu Ala Asp His Tyr Lys Asn Glu Pro Ala
610 615 620

Ile Tyr Gly Phe Asp Ile Met Asn Glu Pro His Ser Met Pro Thr Pro
625 630 635 640

Thr Thr Trp Pro Thr Tyr Ala Gln Ala Ala Val His Ala Ile Arg Glu
645 650 655

Val Asn Leu Asp Thr Trp Ile Ile Val Glu Gly Glu Thr Tyr Ala Asn
660 665 670

Ser Trp Lys Phe Gly Glu Lys Asn Pro His Leu His Asn Val Arg Asp
675 680 685

Pro Val Gly Arg Leu Met Phe Ser Ala His Ser Tyr Trp Cys Lys Asn
690 695 700

Gly Asp Asp Arg Tyr Gly Thr Tyr Asp Ala Glu Asn Gly His Pro Gln
705 710 715 720

Met Gly Val Asp Ser Leu Lys His Phe Val Asp Trp Leu Arg Lys His
725 730 735

Asn Ala His Gly Phe Val Gly Glu Tyr Gly Val Pro Asn Asn Asp Pro
740 745 750

Arg Trp Leu Glu Val Leu Glu Asn Ala Leu Ile Tyr Leu Ala Asn Glu
755 760 765

Asn Ile Ser Gly Thr Tyr Trp Ala Gly Gly Ala Trp Leu Ala Gly Ser
770 775 780

His Ile Ser Cys His Pro Ser Ser Asn Tyr Thr Val Asp Arg Pro Val
785 790 795 800

Met Ser Val Leu Gln Asn Tyr Pro
805

<210> 18
<211> 665
<212> PRT
<213> Artificial

<220>
<223> HASTAG'ed

<220>
<221> MISC_FEATURE
<222> (1)..(8)
<223> Has tag

<400> 18

```
His His His His His His Pro Arg Ser Ser Val Ala Ala Val Ser Val
1               5                   10                  15

Ser Ala Lys Ile Asn Ala Phe Thr Asn Ser Asp Trp Leu Asn Gly Ile
            20                  25                  30

Trp Arg Thr Gly Ala Gly Phe Ser Ile Pro Ala Thr Ser Ala Asn Arg
        35                  40                  45

Ala Ala Phe Val Ala Gly Ala Ser Val Arg Leu Ala Asp Gly Gln Val
    50                  55                  60

Arg Lys Ile Ser Arg Ala Gln Ile Val Gly Ser Asn Met Ser Ile Phe
65                  70                  75                  80

Leu Glu Gly Ala Lys Leu Asp Gly Asn Lys Val Gly Ala Pro Gln Val
                85                  90                  95

Val Thr Ile Gly Ser Thr Ala Val Thr Ala Pro Asp Thr Ser Ala Pro
            100                 105                 110

Ile Thr Thr Pro Pro Thr Val Thr Ala His Ser Thr Ser Ile Asn Ala
        115                 120                 125

Phe Thr Asn Asn Asp Trp Leu Asn Gly Val Trp Arg Lys Ser Pro Gly
    130                 135                 140

Phe Ser Ile Pro Ala Ser Ala Ala Asn Lys Ala Ala Phe Lys Val Gly
145                 150                 155                 160

Ala Thr Ala Lys Leu Ala Asp Gly Gln Val Arg Lys Ile Thr Gln Val
                165                 170                 175

Gln Val Val Gly Ala Asn Met Ser Val Tyr Leu Glu Gly Ala Ala Val
            180                 185                 190

Asn Gly Ser Val Val Gly Ala Pro Asn Lys Leu Ala Leu Ala Thr Thr
        195                 200                 205

Ser Thr Thr Ser Pro Ala Pro Thr Pro Ala Pro Ser Ala Pro Thr Pro
    210                 215                 220
```

```
Ser Val Ile Ala Thr Ser Asn Leu Asn Asn Tyr Thr Asn Ala Gln Trp
225                 230                 235                 240

Leu Asn Gly Met Tyr Arg Thr Ala Ala Gly Phe Ser Ile Gln Ala Ser
                245                 250                 255

Ser Ala Asn Val Ala Ala Phe Lys Ala Gly Ala Leu Val Arg Leu Ala
            260                 265                 270

Asp Gly Gln Thr Arg Lys Val Leu Arg Ala Gln Leu Val Gly Ser Asn
        275                 280                 285

Met Ser Val Phe Leu Asp Gly Ala Val Ile Asn Gly Thr Thr Leu Gly
    290                 295                 300

Tyr Pro Lys Thr Ile Ser Val Val Ser Thr Ser Thr Gly Thr Pro Ser
305                 310                 315                 320

Ser Pro Ala Leu Thr Thr Pro Pro Val Glu Pro Ala Pro Ala Pro Val
                325                 330                 335

Pro Thr Ala Pro Asp Thr Thr Asn Gly Lys Pro Leu Leu Val Gly Val
            340                 345                 350

Asn Leu Ser Gly Ala Gly Phe Gly Pro Ser Val Val Pro Gly Lys His
        355                 360                 365

Gly Thr Asn Tyr Thr Tyr Pro Ala Glu Ser Tyr Tyr Lys Lys Tyr Ser
    370                 375                 380

Asp Leu Gly Met Pro Leu Val Arg Leu Pro Phe Leu Trp Glu Arg Ile
385                 390                 395                 400

Gln Pro Lys Leu Asn Ser Pro Leu Asn Ala Glu Glu Phe Ala Arg Leu
                405                 410                 415

Lys Gln Ser Leu Asp Phe Ala Gln Lys His Asn Val Lys Val Ile Leu
            420                 425                 430

Asp Leu His Asn Tyr Tyr Arg Tyr Tyr Gly Lys Leu Ile Gly Ser Lys
        435                 440                 445

Glu Val Pro Ile Ser Ser Phe Ala Ala Val Trp Lys Gln Ile Val Gln
    450                 455                 460

Gln Val Val Asn His Pro Ala Val Glu Gly Tyr Gly Leu Met Asn Glu
465                 470                 475                 480
```

```
Pro His Ser Thr Asn Gly Leu Trp Pro Gln Ala Ala Leu Ala Ala Ala
                485                 490                 495

Gln Ala Ile Arg Thr Val Asp Ser Lys Arg Trp Ile Tyr Val Ala Gly
            500                 505                 510

Asp Arg Trp Ser Ser Ala Phe His Trp Pro His Tyr Asn Thr Gln Leu
        515                 520                 525

Val Thr Asn Pro Trp Met Arg Asp Pro Lys Asn Asn Leu Val Tyr Glu
    530                 535                 540

Ala His Met Tyr Val Asp Lys Asp Phe Ser Gly Asn Tyr Phe Asp Lys
545                 550                 555                 560

Ala Glu Lys Phe Asp Pro Met Ile Gly Val Asn Arg Val Lys Pro Phe
                565                 570                 575

Val Asp Trp Leu Lys Gln His Lys Leu Arg Gly Tyr Ile Gly Glu His
            580                 585                 590

Gly Val Pro Asp Phe Ser Pro Ser Ala Ile Val Ala Thr Asp Asn Leu
        595                 600                 605

Leu Ala Tyr Leu Arg Gln Asn Cys Ile Pro Ser Thr Tyr Trp Ala Ala
    610                 615                 620

Gly Pro Trp Trp Gly Glu Tyr Ala Met Ser Leu Asp Val Ser Ser Gly
625                 630                 635                 640

Lys His Arg Pro Gln Leu Pro Val Leu Gln Lys His Ala Lys Thr Ala
                645                 650                 655

Asn Ser Cys Thr Ser Ile Gly Pro Leu
            660                 665
```

<210> 19
<211> 8
<212> PRT
<213> Artificial

<220>
<223> Has tag

<400> 19

```
His His His His His His Pro Arg
1               5
```

<210> 20

<211> 27
<212> PRT
<213> Bacillus clausii

<400> 20

```
Met Lys Lys Pro Leu Gly Lys Ile Val Ala Ser Thr Ala Leu Leu Ile
1               5                   10                  15

Ser Val Ala Phe Ser Ser Ser Ile Ala Ser Ala
            20                  25
```

<210> 21
<211> 795
<212> PRT
<213> Paenibacillus sp

<220>
<221> mat_peptide
<222> (28)..(795)

<400> 21

```
Met Lys Lys Pro Leu Gly Lys Ile Val Ala Ser Thr Ala Leu Leu Ile
        -25                 -20                 -15

Ser Val Ala Phe Ser Ser Ser Ile Ala Ser Ala His His His His His
    -10                 -5                  -1  1               5

His Pro Arg Ala Glu Ala Ser Asp Met Phe Asp Glu Leu Arg Glu Lys
                10                  15                  20

Tyr Ala Thr Met Leu Thr Gly Gly Thr Ala Tyr Ser Leu Ser Asp Pro
            25                  30                  35

Asp Ile Ala Ala Arg Val Ala Ser Ile Thr Thr Asn Ala Gln Thr Leu
        40                  45                  50

Trp Thr Ser Met Lys Lys Asp Ala Asn Arg Val Arg Leu Trp Asp Asn
    55                  60                  65

Ala Pro Leu Gly Asn Asp Ser Ala Ser Ile Thr Thr Ser Tyr Arg Gln
70                  75                  80                  85

Leu Ala Ala Met Ala Leu Ala Tyr Arg Thr Tyr Gly Ser Ser Leu Met
                90                  95                  100

Gly Asp Pro Asp Leu Arg Asp Asp Ile Ile Asp Gly Leu Asp Trp Ile
            105                 110                 115
```

```
Asn Thr Phe Gln His Gly Phe Cys Glu Gly Cys Ser Met Tyr Gln Asn
        120                 125                 130

Trp Trp His Trp Gln Ile Gly Gly Pro Ile Ala Leu Asn Glu Val Ile
    135                 140                 145

Ala Leu Met Tyr Asp Glu Leu Thr Gln Thr Gln Ile Asp Ser Tyr Ile
150                 155                 160                 165

Ala Ala Ile Asn Tyr Ala Gln Pro Ser Val Asn Met Thr Gly Ala Asn
                170                 175                 180

Arg Leu Trp Glu Ser Gln Val Ile Ala Leu Ala Gly Ile Asn Gly Lys
            185                 190                 195

Asn Gly Asp Lys Ile Ala His Ala Arg Asp Gly Leu Ser Ala Leu Leu
        200                 205                 210

Thr Tyr Val Val Gln Gly Asp Gly Phe Tyr Glu Asp Gly Ser Phe Val
    215                 220                 225

Gln His Ser Tyr Tyr Ser Tyr Asn Gly Gly Tyr Gly Leu Asp Leu Leu
230                 235                 240                 245

Lys Gly Ile Ala Asp Leu Thr Tyr Leu Leu His Asp Ser Asn Trp Glu
                250                 255                 260

Val Val Asp Pro Asn Lys Gln Asn Ile Phe Asn Trp Val Tyr Asp Ser
            265                 270                 275

Phe Glu Pro Phe Ile Tyr Asn Gly Asn Leu Met Asp Met Val Arg Gly
        280                 285                 290

Arg Glu Ile Ser Arg His Ala Arg Gln Ser Asn Val Val Gly Val Glu
    295                 300                 305

Ala Val Ala Ala Ile Leu Arg Leu Ser His Val Ala Pro Pro Ala Asp
310                 315                 320                 325

Ala Ala Ala Phe Lys Ser Met Val Lys His Trp Leu Gln Glu Gly Gly
                330                 335                 340

Gly Ser Gln Phe Leu Gln Gln Ala Ser Ile Thr His Ile Leu Ser Ala
            345                 350                 355

Gln Asp Val Leu Asn Asp Ser Gly Ile Val Pro Arg Gly Glu Leu Glu
        360                 365                 370
```

```
Ala Tyr Arg Gln Phe Ala Gly Met Asp Arg Ala Leu Gln Leu Arg Gln
    375                 380                 385

Gly Tyr Gly Phe Gly Ile Ser Met Phe Ser Ser Arg Ile Gly Gly His
390                 395                 400                 405

Glu Ala Ile Asn Ala Glu Asn Asn Lys Gly Trp His Thr Gly Ala Gly
                410                 415                 420

Met Thr Tyr Leu Tyr Asn Asn Asp Leu Ser Gln Phe Asn Asp His Phe
            425                 430                 435

Trp Pro Thr Val Asn Ser Tyr Arg Leu Pro Gly Thr Thr Val Leu Arg
        440                 445                 450

Asp Thr Pro Gln Ala Ala Asn Thr Arg Gly Asp Arg Ser Trp Ala Gly
    455                 460                 465

Gly Thr Asp Met Leu Gly Leu Tyr Gly Ile Thr Gly Met Glu Tyr His
470                 475                 480                 485

Ala Ile Gly Lys Ser Leu Thr Ala Lys Lys Ser Trp Phe Met Phe Asp
                490                 495                 500

Asp Glu Ile Val Ala Leu Gly Ala Asp Ile Thr Ser Gly Asp Gly Val
            505                 510                 515

Ala Val Glu Thr Ile Val Glu Asn Arg Lys Leu Asn Gly Ala Gly Asp
        520                 525                 530

Asn Ser Leu Thr Val Asn Gly Thr Ala Lys Pro Ala Thr Leu Gly Trp
    535                 540                 545

Ser Glu Thr Met Gly Thr Thr Ser Tyr Ala His Leu Gly Gly Ser Val
550                 555                 560                 565

Ala Asp Ser Asp Ile Gly Tyr Tyr Phe Pro Asp Gly Gly Ala Thr Leu
                570                 575                 580

His Ala Leu Arg Glu Ala Arg Thr Gly Asn Trp Arg Gln Ile Asn Ser
            585                 590                 595

Ala Gln Gly Ser Pro Asn Ala Pro His Thr Arg Asn Tyr Leu Thr Met
        600                 605                 610

Trp Leu Glu His Gly Val Asn Pro Ser Asn Gly Ala Tyr Ser Tyr Val
```

```
    615                 620                 625

Leu Leu Pro Asn Lys Thr Ser Ala Ala Thr Ala Ser Tyr Ala Ala Ser
630                 635                 640                 645

Pro Asp Ile Thr Ile Ile Glu Asn Ser Ser Ser Ala Gln Ala Val Lys
                650                 655                 660

Glu Asn Gly Leu Asn Met Ile Gly Val Asn Phe Trp Asn Asn Glu Arg
            665                 670                 675

Lys Thr Ala Gly Gly Ile Thr Ser Asn Ala Lys Ala Ser Val Met Thr
        680                 685                 690

Arg Glu Thr Ala Ser Glu Leu Asn Val Ser Val Ser Asp Pro Thr Gln
    695                 700                 705

Ser Asn Val Gly Met Ile Tyr Ile Glu Ile Asp Lys Ser Ala Thr Gly
710                 715                 720                 725

Leu Ile Ala Lys Asp Asp Ala Val Thr Val Leu Gln Tyr Ser Pro Thr
                730                 735                 740

Ile Lys Phe Lys Val Asp Val Asn Lys Ala Arg Gly Lys Ser Phe Lys
            745                 750                 755

Ala Ala Phe Ser Leu Thr Gly Ala Gln Gln Pro
        760                 765
```

<210> 22
<211> 1073
<212> PRT
<213> Paenibacillus sp

<220>
<221> mat_peptide
<222> (28)..(1973)

<400> 22

```
Met Lys Lys Pro Leu Gly Lys Ile Val Ala Ser Thr Ala Leu Leu Ile
        -25                 -20                 -15

Ser Val Ala Phe Ser Ser Ser Ile Ala Ser Ala His His His His His
    -10                 -5                  -1  1               5

His Pro Arg Ala Asp Glu Phe Asp Thr Leu Arg Glu Lys Tyr Lys Ala
                10                  15                  20
```

```
Met Leu Asn Gly Gly Thr Thr Tyr Asn Leu Ser Asp Pro Asp Ile Ala
            25                  30                  35

Ala Arg Val Asn Ala Ile Thr Val Thr Ala Gln Gly Tyr Trp Asp Ser
        40                  45                  50

Met Leu Lys Asp Pro Asn Arg Asn Arg Leu Trp Asn Asp Ala Pro Phe
    55                  60                  65

Gly Ser Asp Ser Thr Ser Ile Thr Thr Thr Tyr Arg His Leu Tyr Asp
70                  75                  80                  85

Met Ala Leu Ala Tyr Thr Thr Tyr Gly Ser Ser Leu Gln Gly Asn Ala
                90                  95                  100

Ala Leu Lys Ala Asp Ile Ile Ser Gly Leu Asp Trp Met Asn Ala Asn
            105                 110                 115

Gln Phe Tyr Asn Gly Cys Ser Gln Tyr Gln Asn Trp Trp His Trp Gln
        120                 125                 130

Ile Gly Gly Pro Met Ala Leu Asn Asp Ile Val Ala Leu Met Tyr Thr
    135                 140                 145

Glu Leu Thr Ala Thr Gln Ile Ser Asn Tyr Met Ala Ala Ile Tyr Tyr
150                 155                 160                 165

Thr Gln Ala Ser Val Thr Met Thr Gly Ala Asn Arg Leu Trp Glu Ser
                170                 175                 180

Gln Val Ile Ala Ile Ser Gly Ile Leu Asn Lys Asp Ser Ala Arg Val
            185                 190                 195

Ala Ala Gly Arg Asp Gly Ile Ser Ala Leu Leu Pro Tyr Val Ala Lys
        200                 205                 210

Gly Asp Gly Phe Tyr Asn Asp Gly Ser Phe Val Gln His Thr Tyr Tyr
    215                 220                 225

Ala Tyr Asn Gly Gly Tyr Gly Ser Glu Leu Leu Ser Gly Ile Ala Asp
230                 235                 240                 245

Leu Ile Phe Ile Leu Asn Gly Ser Ser Trp Gln Val Thr Asp Pro Asn
                250                 255                 260

Lys Asn Asn Val Tyr Arg Trp Ile Tyr Asp Ser Tyr Glu Pro Phe Ile
            265                 270                 275
```

```
Tyr Lys Gly Asn Leu Met Asp Met Val Arg Gly Arg Glu Ile Ser Arg
        280                 285                 290

His Gly Leu Gln Asp Asp Lys Ala Ala Val Thr Val Met Ala Ser Ile
    295                 300                 305

Ile Arg Leu Ser Gln Thr Ala Ala Ser Ala Asp Ala Thr Ala Phe Lys
310                 315                 320                 325

Arg Met Val Lys Tyr Trp Leu Leu Leu Asp Thr Asp Lys Thr Phe Leu
                330                 335                 340

Lys Ala Val Ser Ile Asp Leu Ile Ile Ala Ala Asn Gln Leu Val Asn
            345                 350                 355

Asp Ser Thr Val Thr Ser Arg Gly Glu Leu Val Lys Tyr Lys Gln Phe
        360                 365                 370

Ser Gly Met Asp Arg Ala Val Gln Leu Arg Pro Gly Phe Gly Phe Gly
    375                 380                 385

Leu Ser Met Phe Ser Ser Arg Ile Gly Asn Tyr Glu Ser Ile Asn Ala
390                 395                 400                 405

Glu Asn Asn Lys Gly Trp His Thr Gly Asp Gly Met Thr Tyr Leu Tyr
                410                 415                 420

Asn Thr Asp Leu Ser Gln Phe Asn Asp His Phe Trp Ala Thr Val Asp
            425                 430                 435

Asn Tyr Arg Leu Pro Gly Thr Thr Val Leu Gln Asn Thr Thr Gln Thr
        440                 445                 450

Ala Asn Ser Arg Ser Asp Lys Ser Trp Ala Gly Gly Thr Asp Ile Leu
    455                 460                 465

Gly Gln Tyr Gly Val Ser Gly Met Glu Leu His Thr Val Gly Lys Ser
470                 475                 480                 485

Leu Thr Ala Lys Lys Ser Trp Phe Met Phe Asp Asp Glu Ile Val Ala
                490                 495                 500

Leu Gly Ser Gly Ile Ala Ser Thr Asp Gly Ile Ala Thr Glu Thr Ile
            505                 510                 515

Val Glu Asn Arg Lys Leu Asn Ser Ser Gly Asn Asn Ala Leu Ile Val
        520                 525                 530
```

```
Asn Gly Thr Ala Lys Pro Gly Ser Leu Gly Trp Ser Glu Thr Met Thr
    535                 540                 545

Gly Thr Asn Tyr Ile His Leu Ala Gly Ser Val Pro Gly Ser Asp Ile
550                 555                 560                 565

Gly Tyr Tyr Phe Pro Gly Gly Ala Ala Val Lys Gly Leu Arg Glu Ala
                570                 575                 580

Arg Ser Gly Ser Trp Ser Ser Leu Asn Ser Ser Ala Ser Trp Lys Asp
            585                 590                 595

Ser Thr Leu His Thr Arg Asn Phe Met Thr Leu Trp Phe Asp His Gly
        600                 605                 610

Met Asn Pro Thr Asn Gly Ser Tyr Ser Tyr Val Leu Leu Pro Asn Lys
    615                 620                 625

Thr Ser Ser Ala Val Ala Ser Tyr Ala Ala Thr Pro Gln Ile Ser Ile
630                 635                 640                 645

Leu Glu Asn Ser Ser Ser Ala Gln Ala Val Lys Glu Thr Gln Leu Asn
                650                 655                 660

Val Thr Gly Ile Asn Phe Trp Asn Asp Glu Pro Thr Thr Val Gly Leu
            665                 670                 675

Val Thr Ser Asn Arg Lys Ala Ser Val Met Thr Lys Glu Thr Ala Ser
        680                 685                 690

Asp Phe Glu Ile Ser Val Ser Asp Pro Thr Gln Ser Asn Val Gly Thr
    695                 700                 705

Ile Tyr Ile Asp Val Asn Lys Ser Ala Thr Gly Leu Ile Ser Lys Asp
710                 715                 720                 725

Asn Glu Ile Thr Val Ile Gln Tyr Tyr Pro Thr Met Lys Phe Lys Val
                730                 735                 740

Asn Val Asn Asn Ser Gly Gly Lys Ser Tyr Lys Val Lys Phe Ser Leu
            745                 750                 755

Thr Gly Thr Pro Gly Ser Asn Pro Ser Pro Ile Pro Ile Pro Asn Pro
        760                 765                 770

Tyr Glu Ala Glu Ala Leu Pro Ile Asn Ala Leu Thr Asp Thr Pro Val
```

```
    775                 780                 785

Val Tyr Asn Asp Ala Asn Ala Ser Gly Gly Lys Lys Leu Gly Phe Asn
790                 795                 800                 805

Asn Asn Ala Val Asp Asp Tyr Val Glu Phe Ser Leu Asp Val Thr Gln
                810                 815                 820

Pro Gly Thr Tyr Asp Val Lys Ser Arg Ile Met Lys Ser Thr Asn Ser
            825                 830                 835

Gly Ile Tyr Gln Leu Ser Ile Asn Gly Thr Asn Val Gly Ser Ala Gln
        840                 845                 850

Asp Met Phe Trp Thr Thr Ser Glu Leu Ser Lys Glu Phe Thr Met Gly
    855                 860                 865

Ser Tyr Ser Phe Ser Thr Pro Gly Ser Tyr Leu Phe Arg Leu Lys Thr
870                 875                 880                 885

Thr Gly Lys Asn Val Ser Ser Ser Gly Tyr Lys Leu Met Leu Asp Asn
                890                 895                 900

Phe Ser Leu Val Ser Thr Gly Ile Asp Thr Thr Val Ile Val Asp Asn
            905                 910                 915

Ala Asp Ala Ala Gly Val Thr Lys Val Gly Thr Trp Thr Gly Thr Asn
        920                 925                 930

Thr Gln Thr Asp Arg Tyr Gly Ala Asp Tyr Ile His Asp Gly Asn Thr
    935                 940                 945

Gly Lys Gly Thr Lys Ser Val Thr Phe Thr Pro Asn Val Pro Ile Ser
950                 955                 960                 965

Gly Thr Tyr Gln Val Tyr Met Met Trp Ala Ala His Thr Asn Arg Ala
                970                 975                 980

Thr Asn Val Pro Val Asp Val Thr His Ser Gly Gly Thr Ala Thr Leu
            985                 990                 995

Asn Val Asn  Gln Gln Gly Asn Gly  Gly Val Trp Asn Leu  Leu Gly
        1000                 1005                 1010

Thr Tyr Ser  Phe Asn Ala Gly Ser  Thr Gly Ala Ile Lys  Ile Arg
        1015                 1020                 1025
```

Thr Asp Ala Thr Asn Gly Tyr Val Val Ala Asp Ala Val Lys Leu
1030 1035 1040

Val Lys Val Pro
1045

<210> 23
<211> 1078
<212> PRT
<213> Paenibacillus sp

<220>
<221> mat_peptide
<222> (28)..(1078)

<400> 23

Met Lys Lys Pro Leu Gly Lys Ile Val Ala Ser Thr Ala Leu Leu Ile
-25 -20 -15

Ser Val Ala Phe Ser Ser Ser Ile Ala Ser Ala His His His His His
-10 -5 -1 1 5

His Pro Arg Ala Glu Ala Ser Asp Met Phe Asp Glu Leu Arg Glu Lys
10 15 20

Tyr Ala Thr Met Leu Thr Gly Gly Thr Ala Tyr Ser Leu Ser Asp Pro
25 30 35

Asp Ile Ala Ala Arg Val Ala Ser Ile Thr Thr Asn Ala Gln Thr Leu
40 45 50

Trp Thr Ser Met Lys Lys Asp Ala Asn Arg Val Arg Leu Trp Asp Asn
55 60 65

Ala Pro Leu Gly Asn Asp Ser Ala Ser Ile Thr Thr Ser Tyr Arg Gln
70 75 80 85

Leu Ala Ala Met Ala Leu Ala Tyr Arg Thr Tyr Gly Ser Ser Leu Met
90 95 100

Gly Asp Pro Asp Leu Arg Asp Asp Ile Ile Asp Gly Leu Asp Trp Ile
105 110 115

Asn Thr Phe Gln His Gly Phe Cys Glu Gly Cys Ser Met Tyr Gln Asn
120 125 130

Trp Trp His Trp Gln Ile Gly Gly Pro Ile Ala Leu Asn Glu Val Ile
135 140 145

```
Ala Leu Met Tyr Asp Glu Leu Thr Gln Thr Gln Ile Asp Ser Tyr Ile
150                 155                 160                 165

Ala Ala Ile Asn Tyr Ala Gln Pro Ser Val Asn Met Thr Gly Ala Asn
                170                 175                 180

Arg Leu Trp Glu Ser Gln Val Ile Ala Leu Ala Gly Ile Asn Gly Lys
            185                 190                 195

Asn Gly Asp Lys Ile Ala His Ala Arg Asp Gly Leu Ser Ala Leu Leu
        200                 205                 210

Thr Tyr Val Val Gln Gly Asp Gly Phe Tyr Glu Asp Gly Ser Phe Val
    215                 220                 225

Gln His Ser Tyr Tyr Ser Tyr Asn Gly Gly Tyr Gly Leu Asp Leu Leu
230                 235                 240                 245

Lys Gly Ile Ala Asp Leu Thr Tyr Leu Leu His Asp Ser Asn Trp Glu
                250                 255                 260

Val Val Asp Pro Asn Lys Gln Asn Ile Phe Asn Trp Val Tyr Asp Ser
            265                 270                 275

Phe Glu Pro Phe Ile Tyr Asn Gly Asn Leu Met Asp Met Val Arg Gly
        280                 285                 290

Arg Glu Ile Ser Arg His Ala Arg Gln Ser Asn Val Val Gly Val Glu
    295                 300                 305

Ala Val Ala Ala Ile Leu Arg Leu Ser His Val Ala Pro Pro Ala Asp
310                 315                 320                 325

Ala Ala Ala Phe Lys Ser Met Val Lys His Trp Leu Gln Glu Gly Gly
                330                 335                 340

Gly Ser Gln Phe Leu Gln Gln Ala Ser Ile Thr His Ile Leu Ser Ala
            345                 350                 355

Gln Asp Val Leu Asn Asp Ser Gly Ile Val Pro Arg Gly Glu Leu Glu
        360                 365                 370

Ala Tyr Arg Gln Phe Ala Gly Met Asp Arg Ala Leu Gln Leu Arg Gln
    375                 380                 385

Gly Tyr Gly Phe Gly Ile Ser Met Phe Ser Ser Arg Ile Gly Gly His
390                 395                 400                 405
```

```
Glu Ala Ile Asn Ala Glu Asn Asn Lys Gly Trp His Thr Gly Ala Gly
                410                 415                 420

Met Thr Tyr Leu Tyr Asn Asn Asp Leu Ser Gln Phe Asn Asp His Phe
            425                 430                 435

Trp Pro Thr Val Asn Ser Tyr Arg Leu Pro Gly Thr Thr Val Leu Arg
        440                 445                 450

Asp Thr Pro Gln Ala Ala Asn Thr Arg Gly Asp Arg Ser Trp Ala Gly
    455                 460                 465

Gly Thr Asp Met Leu Gly Leu Tyr Gly Ile Thr Gly Met Glu Tyr His
470                 475                 480                 485

Ala Ile Gly Lys Ser Leu Thr Ala Lys Lys Ser Trp Phe Met Phe Asp
                490                 495                 500

Asp Glu Ile Val Ala Leu Gly Ala Asp Ile Thr Ser Gly Asp Gly Val
            505                 510                 515

Ala Val Glu Thr Ile Val Glu Asn Arg Lys Leu Asn Gly Ala Gly Asp
        520                 525                 530

Asn Ser Leu Thr Val Asn Gly Thr Ala Lys Pro Ala Thr Leu Gly Trp
    535                 540                 545

Ser Glu Thr Met Gly Thr Thr Ser Tyr Ala His Leu Gly Gly Ser Val
550                 555                 560                 565

Ala Asp Ser Asp Ile Gly Tyr Tyr Phe Pro Asp Gly Gly Ala Thr Leu
                570                 575                 580

His Ala Leu Arg Glu Ala Arg Thr Gly Asn Trp Arg Gln Ile Asn Ser
            585                 590                 595

Ala Gln Gly Ser Pro Asn Ala Pro His Thr Arg Asn Tyr Leu Thr Met
        600                 605                 610

Trp Leu Glu His Gly Val Asn Pro Ser Asn Gly Ala Tyr Ser Tyr Val
    615                 620                 625

Leu Leu Pro Asn Lys Thr Ser Ala Ala Thr Ala Ser Tyr Ala Ala Ser
630                 635                 640                 645

Pro Asp Ile Thr Ile Ile Glu Asn Ser Ser Ser Ala Gln Ala Val Lys
```

650 655 660

Glu Asn Gly Leu Asn Met Ile Gly Val Asn Phe Trp Asn Asn Glu Arg
665 670 675

Lys Thr Ala Gly Gly Ile Thr Ser Asn Ala Lys Ala Ser Val Met Thr
680 685 690

Arg Glu Thr Ala Ser Glu Leu Asn Val Ser Val Ser Asp Pro Thr Gln
695 700 705

Ser Asn Val Gly Met Ile Tyr Ile Glu Ile Asp Lys Ser Ala Thr Gly
710 715 720 725

Leu Ile Ala Lys Asp Asp Ala Val Thr Val Leu Gln Tyr Ser Pro Thr
730 735 740

Ile Lys Phe Lys Val Asp Val Asn Lys Ala Arg Gly Lys Ser Phe Lys
745 750 755

Ala Ala Phe Ser Leu Thr Gly Ala Gln Gln Pro Asn Pro Ala Pro Ile
760 765 770

Pro Ile Pro Asn Pro Tyr Glu Ala Glu Leu Leu Pro Ile Ser Ala Thr
775 780 785

Thr Lys Thr Pro Thr Leu Ser Asn Asp Ser Asn Ala Ser Gly Gly Lys
790 795 800 805

Lys Leu Gly Leu Asn Ser Ser Val Val Gly Asp Tyr Thr Glu Phe Ser
810 815 820

Leu Asp Val Thr Gln Pro Gly Thr Tyr Asp Ile Ala Ala Lys Ile Met
825 830 835

Lys Val Ser Asn Asn Gly Ile Tyr Gln Phe Ser Ile Asn Gly Glu Pro
840 845 850

Val Gly Asp Pro Val Asp Met Tyr Trp Asn Thr Ser Glu Ser Thr Lys
855 860 865

Ser Phe Ser Pro Gly Ser Tyr Thr Phe Ser Glu Pro Gly Ser Tyr Leu
870 875 880 885

Leu Arg Val Thr Val Thr Gly Lys His Pro Ser Ser Ser Gly Tyr Lys
890 895 900

Leu Met Leu Asp His Phe Thr Leu Glu Glu Ile Pro Val Ser Leu Pro
905 910 915

Asn Pro Tyr Glu Ala Glu Thr Leu Pro Ile His His Arg Thr Gln Thr
920 925 930

Val Thr Ile Tyr Asn Asp Ser Asn Thr Ser Gly Gly Gln Arg Leu Gly
935 940 945

Leu Asn His Lys Val Val Gly Asp Tyr Thr Glu Phe Ile Leu Asp Val
950 955 960 965

Pro Gln Ala Gly Thr Tyr Asp Ile Thr Ala Arg Val Leu Lys Phe Ser
970 975 980

Asp Asn Gly Ile Tyr Gln Phe Ser Ile Asp Gly Asn Pro Val Gly Ala
985 990 995

Pro Ile Asp Thr Tyr Trp Asn Thr Ala Gly Tyr Ile Arg Asp Phe
1000 1005 1010

Thr Pro Gly Ser Tyr Thr Phe Ser Glu Pro Gly Ser Tyr Leu Leu
1015 1020 1025

Arg Leu Thr Ala Thr Gly Lys Asn Pro Ser Ala Ser Gly Leu Lys
1030 1035 1040

Ile Met Leu Asp Tyr Ile Trp Leu Asp
1045 1050

<210> 24
<211> 968
<212> PRT
<213> Paenibacillus sp

<220>
<221> mat_peptide
<222> (28)..(968)

<400> 24

Met Lys Lys Pro Leu Gly Lys Ile Val Ala Ser Thr Ala Leu Leu Ile
-25 -20 -15

Ser Val Ala Phe Ser Ser Ser Ile Ala Ser Ala His His His His His
-10 -5 -1 1 5

His Pro Arg Gly Gly Glu Ala Ser Gly Ser Ala Asp Asp Ala Ala Glu
10 15 20

```
Thr Ala Glu Ala Ala Glu Gly Glu Asn Ile Glu Asp Lys Met Val Ser
            25                  30                  35

Ala Tyr Asn Met Asp Ala Phe Asp Ile Met Arg Glu Val Arg Arg Thr
        40                  45                  50

Met Leu Thr Gly Gly Ala Ala Leu Asn Pro Ala Asp Pro Asp Ala Ala
    55                  60                  65

Ala Ala Val Ala Ala Leu Ala Ser Glu Ala Asn Gln Tyr Trp Gln Thr
70                  75                  80                  85

Met Asp Asp Ser Pro Gly Arg Thr Ser Leu Trp Ser Asp Asn Pro Gly
                90                  95                  100

Thr Gly Asn Ser Ile His Ile Arg Ile Thr Tyr Glu Arg Leu Lys Thr
            105                 110                 115

Met Ala Leu Ala Tyr Ala Ala Ala Gly Ser Pro Leu His Ser Asn Ala
        120                 125                 130

Ser Leu Glu Ala Asp Ile Val Asp Ala Leu Asp Tyr Met Tyr Ala Thr
    135                 140                 145

Arg Tyr His Glu Asn Val Thr Thr Thr Pro Ser Gly Thr Ser Asn Trp
150                 155                 160                 165

Trp Asp Trp Gln Ile Gly Ile Pro Met Gln Leu Asn Asp Thr Val Val
                170                 175                 180

Leu Met Tyr Asp Ser Leu Thr Pro Ala Gln Ile Ala Asn Tyr Met Asn
            185                 190                 195

Ala Val Glu Arg Phe Thr Pro Thr Val Asn Leu Thr Gly Ala Asn Arg
        200                 205                 210

Ser Trp Lys Ala Ile Val Val Ala Val Arg Gly Ile Leu Val Lys Asp
    215                 220                 225

Gly Ala Lys Ile Ala Ala Ala Arg Asp Gly Leu Ser Gln Ile Phe Asn
230                 235                 240                 245

Tyr Ala Val Ser Gly Asp Gly Phe Tyr Arg Asp Gly Ser Phe Ile Gln
                250                 255                 260

His Gly Asn Ile Pro Tyr Asn Gly Gly Tyr Gly Leu Asp Leu Leu Leu
            265                 270                 275
```

```
Ala Val Ser Asp Leu Met Thr Leu Leu His Gly Ser Ala Trp Gln Val
        280                 285                 290

Thr Asp Pro Asn Gln Ala Asn Val Trp Glu Trp Val Tyr Arg Ala Tyr
    295                 300                 305

Gln Pro Leu Ile Tyr Lys Gly Ala Met Met Asp Met Val Arg Gly Arg
310                 315                 320                 325

Glu Ile Ser Arg Val Tyr Arg Gln Asp His Ala Ala Gly His Ile Ala
                330                 335                 340

Met Gln Gly Ile Leu Arg Leu Ser Ala Val Ala Pro Pro Ala Gln Ala
            345                 350                 355

Glu Asp Phe Lys Arg Met Val Lys Gly Trp Met Val Val Asp Gly Phe
        360                 365                 370

Met Arg Phe Tyr Glu Gln Ala Pro Leu Gly Leu Ile Pro Leu Ala Lys
    375                 380                 385

Ala Val Glu Gly Asp Ala Ser Ile Ala Pro Ala Ser Glu Leu Ile Gln
390                 395                 400                 405

Tyr Arg Gln Tyr Ala Ala Met Asp Arg Ala Val Gln Leu Arg Pro Gly
                410                 415                 420

Tyr Gly Phe Gly Leu Ala Met Tyr Ser Ser Arg Ile Gly Ser Phe Glu
            425                 430                 435

Ala Ile Asn Ser Glu Asn Leu Arg Gly Trp Tyr Thr Ser Ala Gly Met
        440                 445                 450

Thr Ser Leu Tyr Asn Gly Asp Leu Gly His Tyr Ser Glu Asp Tyr Trp
    455                 460                 465

Pro Thr Val Asn Ala Tyr Arg Leu Pro Gly Thr Thr Val Leu Ser Gly
470                 475                 480                 485

Thr Ala Ala Ala Ser His Thr Ser Pro Asn Asn Trp Thr Gly Gly Thr
                490                 495                 500

Asp Met Gln Gly Leu Tyr Gly Val Ser Gly Met Asp Leu Lys Tyr Ala
            505                 510                 515

Ser Asn Ser Leu Ala Ala Arg Lys Ser Trp Phe Met Phe Asp Asp Glu
```

```
        520                 525                 530

Ile Val Ala Leu Gly Ala Gly Ile Ser Ser Ala Asp Gly Ile Pro Val
    535                 540                 545

Glu Thr Ile Ile Glu Asn Arg Arg Ile Gly Gly Ala Gly Asp Asn Ala
550                 555                 560                 565

Phe Leu Ala Asp Gly Ala Ala Met Pro Ala Glu Leu Gly Trp Ser Gly
                570                 575                 580

Thr Leu Glu Gly Val Arg Trp Ala His Leu Thr Gly Thr Ala Ala Gly
            585                 590                 595

Ala Asp Ile Gly Tyr Tyr Phe Pro Glu Pro Ala Ala Val His Ala Val
        600                 605                 610

Arg Glu Ala Arg Thr Gly Asn Trp Arg Gln Ile Asn Asn Arg Pro Val
    615                 620                 625

Thr Pro Ala Ala Ser Val Thr Arg Asn Tyr Leu Thr Phe Trp Phe Asp
630                 635                 640                 645

His Gly Ala Asn Pro Thr Asn Ala Asp Tyr Gln Tyr Val Leu Leu Pro
                650                 655                 660

Asn Lys Ser Gly Ala Gln Val Ala Gly Tyr Ala Ala Asn Pro Asp Val
            665                 670                 675

Glu Val Leu Ala Asn Ser Pro Glu Val Gln Ala Val Lys Glu Ser Ser
        680                 685                 690

Leu Gly Ile Ile Gly Ala Asn Phe Trp Ser Asp Gly Val Arg Thr Val
    695                 700                 705

Asp Leu Ile Thr Val Asn Lys Lys Ala Ser Val Met Thr Arg Glu Thr
710                 715                 720                 725

Pro Gly Ala Ile Leu Asp Leu Ser Val Ser Asp Pro Thr Gln Val Asn
                730                 735                 740

Ala Gly Thr Ile Glu Ile Glu Leu Asn Arg Ala Ala Ser Gly Phe Thr
            745                 750                 755

Ala Asp Pro Gly Val Thr Val Thr Arg Leu Ser Pro Thr Ile Lys Leu
        760                 765                 770
```

```
Thr Val Gln Val Ala Gly Ala Lys Gly Arg Ser Phe Lys Ala Ser Phe
    775                 780                 785

Glu Leu Gly Glu Ala Ser Gly Pro Gly Pro Asp Pro Gly Pro Gly Pro
790                 795                 800                 805

Ser Glu Ile Ile Val Asp Asn Gly Asp Ala Ala Gly Val Thr Lys Ile
                810                 815                 820

Gly Ser Trp Lys Thr Gly Thr Val Gln Thr Asp Arg Tyr Gly Pro Asp
            825                 830                 835

Tyr Leu His Asp Asp Asn Thr Gly Lys Gly Gly Lys Ser Val Arg Phe
        840                 845                 850

Thr Pro Asp Leu Pro Thr Ala Gly Thr Tyr Asp Val Tyr Met Met Trp
    855                 860                 865

Pro Gln His Phe Asn Arg Ala Thr Asn Ile Pro Val Thr Ile Ala His
870                 875                 880                 885

Ala Gly Gly Thr Ala Thr Val Thr Ile Asp Gln Thr Val Ser Gly Gly
                890                 895                 900

Val Trp Asn Tyr Leu Gly Ser Tyr Ser Phe Asp Thr Gly Ser Gly Gly
            905                 910                 915

Ser Val Thr Ile Ser Asn Ala Gly Thr Asn Gly Tyr Val Val Ala Asp
        920                 925                 930

Ala Val Lys Phe Glu Tyr Val Pro
    935                 940
```